# EUROPEAN PATENT SPECIFICATION

(11) **EP 2 714 924 B1**
(45) Date of publication and mention of the grant of the patent: **21.03.2018**
(21) Application number: 12790255.9
(22) Date of filing: 24.05.2012
(51) Int. Cl.: A61K 31/167, A61K 31/34, A61K 31/343, A61K 31/4178, A61K 31/4406, A61K 31/4468, A61K 31/4525, A61K 31/496, A61K 45/06, A61P 3/06, A61P 3/10, A61P 25/16, A61P 25/28, A61P 35/00, A61P 43/00

(54) **HDAC Inhibitors for use in the treatment of Gaucher's disease, von Hippel-Lindau disease, phaeochromocytoma and paragangliomas.**
HDAC-Hemmer zur Behandlung von Gaucher'scher Krankheit, von Hippel-Lindau'scher Krankheit, Phäochromocytom und Paragangliomen.
Inhibiteurs de l'HDAC destinées au traitement de la maladie de Gaucher, la maladie von Hippel-Lindau, du phéochromocytome et des paragangliomes.

(30) Priority: 24.05.2011 US 201161489469 P; 14.10.2011 US 201161547458 P; 18.11.2011 US 201161561747 P
(43) Date of publication of application: 09.04.2014
(73) Proprietor: Lixte Biotechnology, Inc., East Setauket, NY 11733 (US); The U.S.A. as represented by the Secretary, Department of Health and Human Services, Bethesda, MD 20892-7660 (US)
(72) Inventor: KOVACH, John, S., East Setauket, NY 11733 (US); ZHUANG, Zhengping, Bethesda, MD 20814 (US); LU, Jie, Rockville, MD 20852 (US); YANG, Chunzhang, Bethesda, MD 20814 (US); LONSER, Russell, Bethesda, Maryland 20817 (US)
(74) Representative: ABG Patentes, S.L.
(86) International application number: PCT/US2012/039405
(87) International publication number: WO 2012/162535

(56) References cited:
- WO-A2-2007/014327
- US-A1- 2004 204 339
- US-A1- 2010 029 640
- US-A1- 2010 029 683
- US-A1- 2010 183 577
- US-A1- 2011 071 109
- US-A1- 2011 092 447
- US-A1- 2012 184 624
- LU ET AL.: 'Histone deacetylase inhibitors prevent the degradation and restore the activity of glucocerebrosidase in Gaucher disease' PROCEEDINGS OF THE NATIONAL ACADEMY OF SCIENCES vol. 108, no. 52, 27 December 2011, pages 21200 - 21205, XP055139132

## Description

### BACKGROUND OF THE INVENTION

Various genetic disorders are caused by mutations in DNA that produce proteins with abnormal amino acid sequences, which cause the proteins to misfold. Such misfolding, often caused by genetic mutations, does not allow the protein to assume its correct functional shape or conformation. In disorders that result in loss-of-function, the misfolded proteins are unable to function properly and targeted for early destruction. This scenario causes a disruption of proteostasis. In gain-of-function diseases, the misfolded proteins are not destroyed. Instead, the misfolded proteins are broken down by the cell and reassembled into aggregates which cause cellular damage. Gain-of-function diseases are often associated with aging but are also caused by genetic mutations.

### Gaucher's Disease

Gaucher's Disease (GD) is the most prevalent lysosomal storage disorder of humans (Beutler and Grabowski (2001)). Individuals afflicted with Gaucher's disease have a rare genetic abnormality in glycosphingolipid metabolism due to deficiency of lysosomal acid beta-glucocerebrosidase (Brady et al. (1965); Brady et al. (1966)). Gaucher's Disease is a storage disorder, which produces a multisystem disease. Typical manifestations of GD include hepatosplenomegaly, cytopenias, bone disease, and in some patients, CNS involvement. The birth frequency of Gaucher's disease is ∼1:50,000 live births in the general population, but ∼1:400-1:865 in Ashkenazi Jews (de Fost et al., 2003).

Beta-glucocerebrosidase catalyses degradation of glucocerebroside, an intermediate in the degradation of complex glycosphingolipids, to produce glucose and ceramide (de Fost et al., 2003). Mutations in the gene coding for beta-glucocerebrosidase, *GBA,* cause the substrate, glucocerebroside, to build in concentration within the lysosomal stores. The abnormal storage of glucocerebroside ultimately engenders the pathologies associated with Gaucher's Disease.

The primary approaches to treating Gaucher's Disease are based on substrate reduction, gene therapy and enzyme replacement therapy (de Fost et al., 2003). Treatment by substrate reduction therapy (SRT) is not permitted to be used on all patients suffering from Gaucher's Disease. SRT is generally reserved for patients that may not be treated by enzyme replacement therapy and are suffering from Gaucher's Type 1. Additionally, enzyme replacement therapy (ERT) provides for the lifelong preparations of the protein that is lacking. ERT is highly expensive and ineffective for treatment of disease in the brain because the proteins do not gain entry into the brain.

New treatments for Gaucher's disease are needed. The current therapies, SRT and ERT, can achieve only partial success in treating patients diagnosed with Gaucher's disease.

### Von Hippel-Lindau Disease

Von Hippel-Lindau disease (VHL) is a heritable multisystem cancer syndrome that is caused by a germline mutation of the VHL gene. Missense mutations of the VHL gene is the most frequent genetic changes in VHL but the precise mechanism of tumorigenesis mechanism caused by mutation remains unclear.

The VHL gene was demonstrated to be a short lifespan protein with a half life that is as short as 100 min (McClellan et al., (2005)). Several mutations in VHL genes were demonstrated to be affecting the conformation and folding process, as well as the formation of early degradation complex immediately after translation (McClellan et al., (2005); Feldman et al., (2003)). Chaeroninne, cochaperonines like Hsp70, Hsp90 and Tric/CCT were demonstrated to be essential in determining the fate of mutated VHL protein (McClellan et al., (2005)). The potential proteostasis alterations due to the mutation might benefit the degradation of VHL protein. Loss-of-function mutations in VHL gene are known to be associated with the pathogenesis of von Hippel-Lindau syndrome. The absence of functional VHL protein led to marked increase in HIF activity in non-hypoxic conditions due to the impairment of VHL-dependent degradation of HIF-1a and HIF-2a. This increased HIF activity is oncogenic since it activates multiple signaling pathways related to cell proliferation, survival, motility, angiogenesis and metabolism. So far numerous of mutations were identified in VHL gene that spread the entire genomic sequence. However, the detailed mechanism that causes the loss of functional VHL protein is still not clear. Mutations at catalytic center of VHL may result in the disruption of the E3 ligase activity owning to a correlative pathogenesis, however, other potential mechanisms might be involved in the pathogenesis of other type of mutations.

At present, treatment for patients with VHL disease is based on early detection of potentially threatening lesions and interventional ablation of vascular tumors subject to hemorrhage and surgical removal of tumors.

### Pheochromocytomas and Paragangliomas

Pheochromocytomas (PHEOs) are rare but life-threatening catecholamine-producing neuroendocrine tumors that arise from chromaffin cells in the adrenal medulla; tumors arising from the peripheral sympathetic or parasympathetic nervous system are called paragangliomas (PGLs) (DeLellis et al., (2004)). Symptoms and signs of these tumors are a direct result of either the mass effects or hypersecretion of catecholamines (e.g., sustained or paroxysmal elevations in blood pressure, tachyarrhythmia, headaches, profuse sweating, pallor, and anxiety) (Lenders et al. (2005)). Additionally, approximately 10% to 30% of PHEOs/PGLs give rise to metastases, for which there are currently limited and suboptimal chemotherapeutic options (Huang et al. (2008)).

PHEOs/PGLs mainly occur as sporadic tumors, which could be associated with well-known inherited neuroendocrine disorders, including multiple endocrine neoplasia type 2 (MEN2), von Hippel-Lindau disease (VHL), and neurofibromatosis type 1 (NF1) (Neumann et al. (2002); Kaelin et al. (2007); Gutmann et al. (1997); Eng et al. (1999)). Recent studies have suggested that changes in a housekeeping gene, succinate dehydrogenase (SDH), are the major contributors to the pathogenesis of these mainly aggressive and metastatic tumors (Neumann et al. (2002); Eng et al. (1999); Baysal et al. (2000); Amar et al. (2005).

SDH is the mitochondrial protein complex II that is vital for mitochondrial electron transport as well as Krebs cycle function. It catalyzes the oxidation of succinate to fumarate and transfers electrons to ubiquinone through the coordination of its four subunits (SDHA, B, C, and D) (Gottlieb et al. (2005); Eng et al. (2003); Baysal et al. (2003)). Genetic defects in SDH coding sequences largely affect its physiologic functions and predispose carriers to the development of PHEOs/PGLs as well as renal cell carcinoma (Neumann et al. (2002); Hensen et al. (2011); Ricketts et al. (2008); Ricketts et al. (2010); Gill et al. (2011)). The pathogenesis of these tumors reflects the abnormal mitochondrial electron transport that results in a state of normoxic pseudohypoxia. Loss of SDH activity prevents the conversion of succinate to fumarate, which leads to mitochondrial and cytoplasmic accumulation of succinate. Previous studies have shown succinate to inhibit prolyl hydroxylases (PHD) responsible for the initial hydroxylation and subsequent degradation of HIF-a (Guzy et al. (2008); Selak et al. (2005); Astuti et al. (2011); Lee et al. (2005)). Stabilization of HIF-1α/2α leads to the overexpression of a large subset of genes factors that increase cellular survival and proliferation and contribute substantially to the tumorigenesis of PHEOs/PGLs (Eisenhofer et al. (2004); Favier et al. (2010); Dahia et al. (2005)). The disruption of mitochondrial electron transfer may also lead to the excess generation of reactive oxygen species (ROS) that have been shown to independently induce a state of pseudohypoxia by inhibiting PHD (Gerald et al. (2004); Gao et al. (2007); Slane et al. (2006); Tormos et al. (2010)). In addition, oxidative stress alters the expression of other tumor suppressor genes or oncogenes through mutagenesis and initiate tumorigenesis.

Germline mutations in the SDHB gene correspond to an autosomal dominant form of PHEO/PGL. Additionally, more than 50% of SDHB-associated PHEOs/PGLs carry missense mutations in the coding sequence of the gene (Gimenez-Roqueplo et al. (2003); Neumann et al. (2004)). These mutations commonly cause a single amino acid substitution in the protein product sufficient to lead to tumors after the loss of heterozygosity of the second wild type allele. However, the precise mechanism of how SDHB gene mutations lead to its functional loss is currently unknown.

Wiest et al. 2011 discloses histone deacetylase inhibitors as therapeutic agents for lysosomal storage disorders, but does not dislose the histone deacetylase inhibitors of the current invention for use in the treatment of a mammalian subject afflicted with a disease characterized by a loss of protein function caused by a genetic abnormality associated with the disease, wherein the disease is Gaucher's disease, von Hippel-Lindau disease, pheochromocytoma or paraganglioma.

Hentsch et al. 2011 discloses histone deacetylase inhibitors for the medical therapy of conditions which predispose a person for the development of a disease, but does not dislose the histone deacetylase inhibitors of the current invention for use in the treatment of a mammalian subject afflicted with a disease characterized by a loss of protein function caused by a genetic abnormality associated with the disease, wherein the disease is Gaucher's disease, von Hippel-Lindau disease, pheochromocytoma or paraganglioma.

DiMartino 2004 discloses compositions and methods for treating diseases associated with aberrant silencing of gene expression such as cancer by reestablishing the gene expression through inhibition of DNA hypomethylation and histone deacetylase, but does not dislose the histone deacetylase inhibitors of the current invention for use in the treatment of a mammalian subject afflicted with a disease characterized by a loss of protein function caused by a genetic abnormality associated with the disease, wherein the disease is Gaucher's disease, von Hippel-Lindau disease, pheochromocytoma or paraganglioma.

### SUMMARY OF THE INVENTION

The present invention provides a histone deacetylase inhibitor for use in the treatment of a mammalian subject afflicted with a disease characterized by a loss of protein function caused by a genetic abnormality associated with the disease as defined in claims 1 and 10.

### BRIEF DESCRIPTION OF THE FIGURES

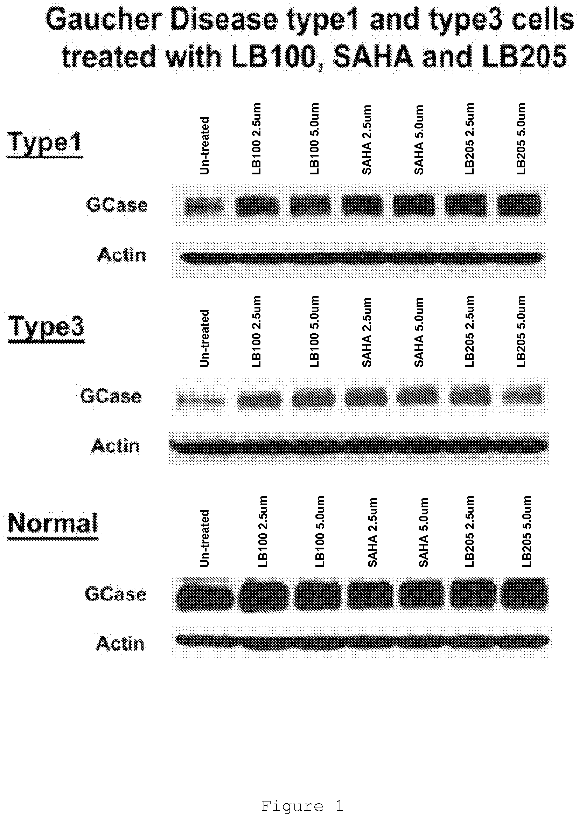

**Figure 1****. Gaucher's Disease type 1 and type 3 cell treated with LB100, SAHA and LB2 for 24 hours.** Gaucher's Disease type 1 and type 3 cells were treated with LB100, SAHA or LB205 for 24 hours and analyzed by western blot. When the cells were treated with LB100, SAHA or LB205, the amount of beta-glucocerebrosidase (GBA) increased.

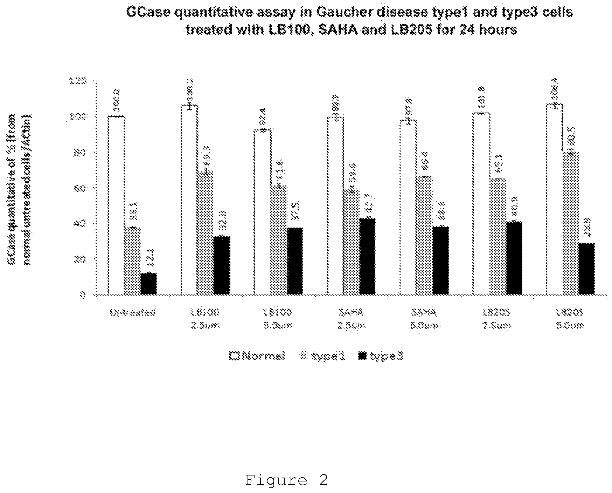

**Figure 2****. GBA quantitive of Gaucher's disease type 1 and type 3 treated with LB100, SAHA and LB2 for 24 hours.** The amount of GBA was quantified after Gaucher's Disease type 1 and type 3 cells were treated with LB100, SAHA or LB205 for 24 hours. Quantification of the treatments with LB100, SAHA or LB205 revealed that the amount of GBA was increased in all treatments, and the increase was not dose-dependent.

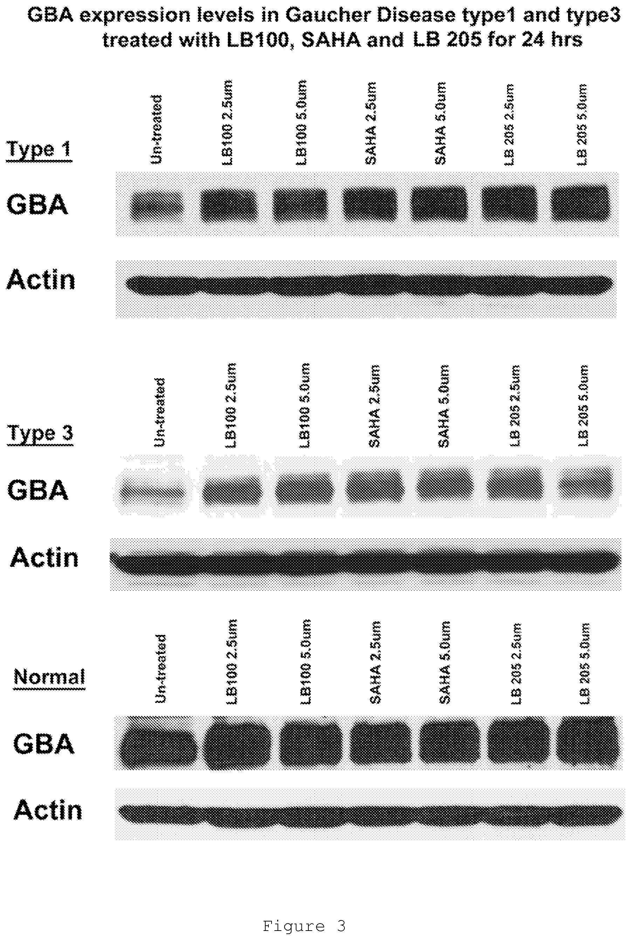

**Figure 3****. GBA expression levels in Gaucher Disease type 1 and type 3 treated with LB100, SAHA and LB 205 for 24 hours.** Gaucher Disease type 1 and type 3 cells were tested for expression of GBA after treatment with LB100, SAHA or LB 205. When the cells were treated with LB100, SAHA or LB205, the amount of beta-glucocerebrosidase (GBA) increased.

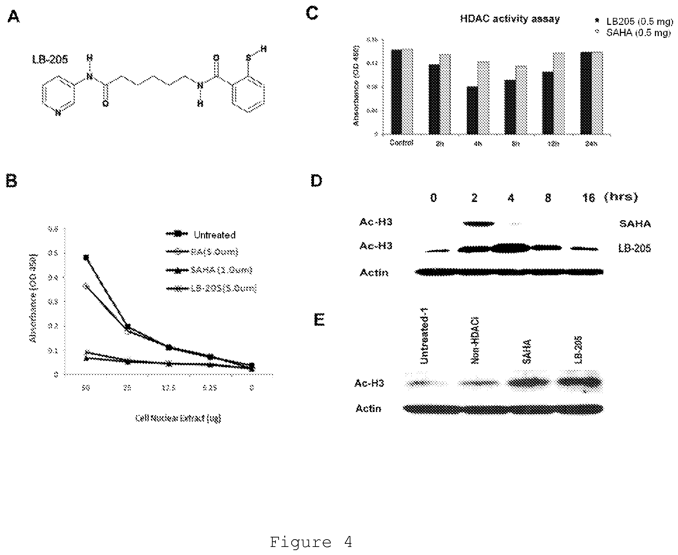

**Figure 4****. LB-205 inhibits HDAC activity *in vitro* and in *vivo.*** (A) Chemical structure of LB-205. LB-205 has a zinc binding moiety to inhibit zinc dependent class I and class II HDACs. (B) HDAC activity assay in DAOY cells, a medulloblastoma cell line, after treatment with LB-205 (C) HDAC activity in SCID mice with DAOY subcutaneous xenografts after single intraperitoneal injections of LB-205 and SAHA, each at 25 mg/Kg. (D) Western blot for acetylated histone 3 in DAOY xenografts confirm HDAC inhibition by LB-205 for at least 4 hours longer than that of SAHA *in vivo.* (E) Western blot for acetylated histone 3 demonstrate HDAC inhibition in type 1 GD fibroblasts treated with LB-205 (2.5 µM) and SAHA (2.5 µM).

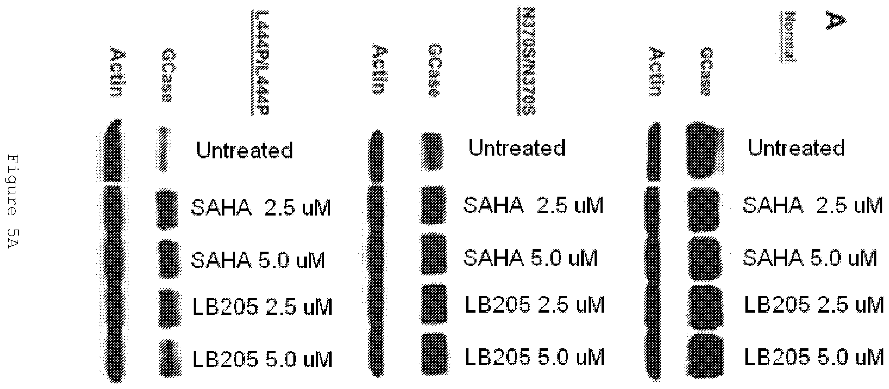

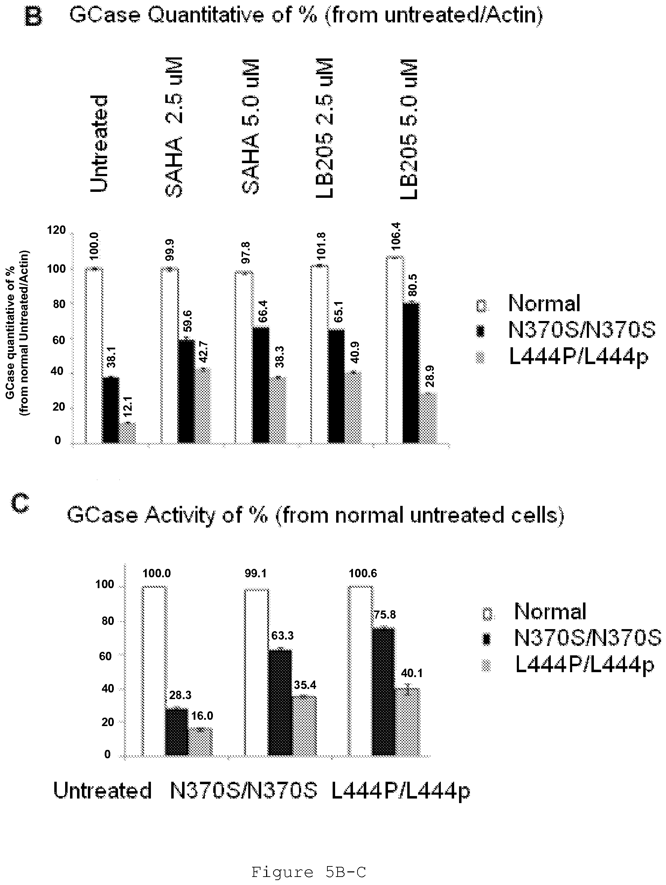

**Figure 5****. HDAC inhibitors increase functional GBA levels in patient-derived Gaucher fibroblasts.** (A) Western blot of analysis of GBA in normal and GD fibroblasts homozygous for N370S or L444P mutations after treatment with LB-205 and SAHA. (B) Quantification of GBA protein by densitometric analysis of Western blots. (C) GBA catalytic activity in normal fibroblasts, untreated GD fibroblasts, and GD fibroblasts treated with LB-205 (2.5 µM) and SAHA (2.5 µM) for 24 hours. GBA activity is quantified as a percentage of that of normal fibroblasts cells.

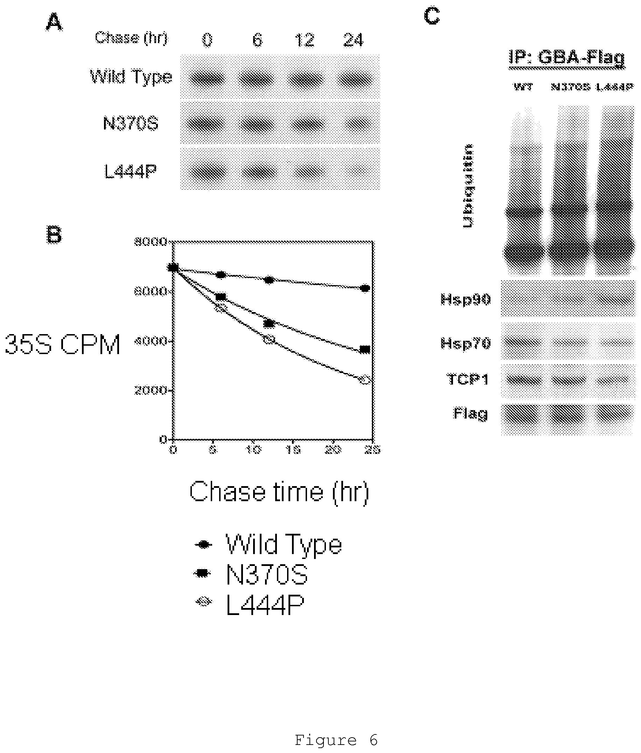

**Figure 6****. Mutated GBAs exhibit reduced half-life and abnormal binding to chaperonins.** (A) Metabolic [³⁵S] pulse chase assay shows quantitative loss of [³⁵S]-labeled GBA protein in HeLa cells with *ΔGBA*-N370S and *ΔGBA*-L444P mutant vectors after 24 hours. (B) Quantification of [³⁵S]-GBA protein through liquid scintillation confirmed the loss of stability of mutant GBA proteins. (C) Western blot of immunoprecipitated N370S and L444P GBA-flag proteins suggests increased degradation of mutated GBAs, as evidenced by reduced binding to chaperonins Hsp70 and TCP1, and increased ubiquitination and Hsp90 binding.

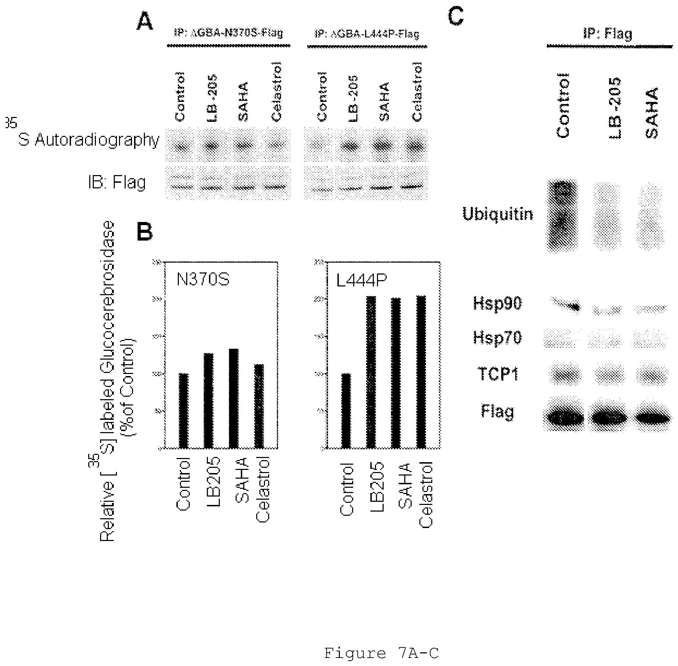

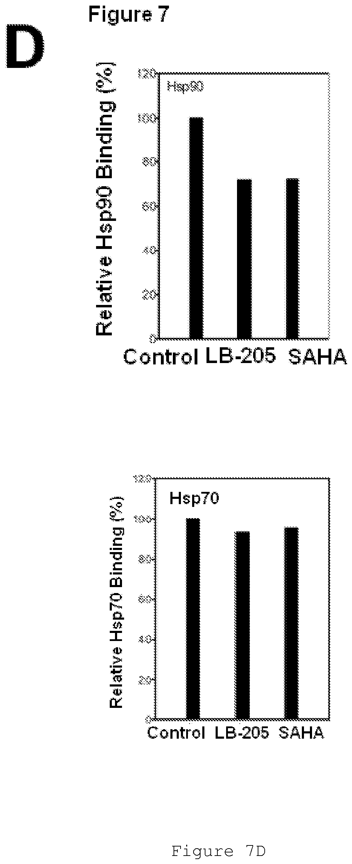

**Figure 7****. Proteostasis regulators prevent degradation of N370S and L444P GBA and promote proper binding to chaperonins.** (A) [³⁵S] pulse chase assay after treatment of HeLa cells with *ΔGBA*-N370S and *ΔGBA-*L444P mutant vectors with proteostasis regulators LB-205, SAHA, and Celastrol. All three proteostasis regulators increased GBA quantity in both N370S and L444P mutants. (B)Densitometric quantification confirmed quantitative increase in mutant GBA after treatment. (C) Western blot of co-immunoprecipitated GBA-flag proteins in L444P mutants treated with LB-205 and SAHA. HDAC inhibition reduced ubiquitin binding with no change in TCP1 and Hsp70 binding. (D) Quantification of relative TCP1 and Hsp70 binding.

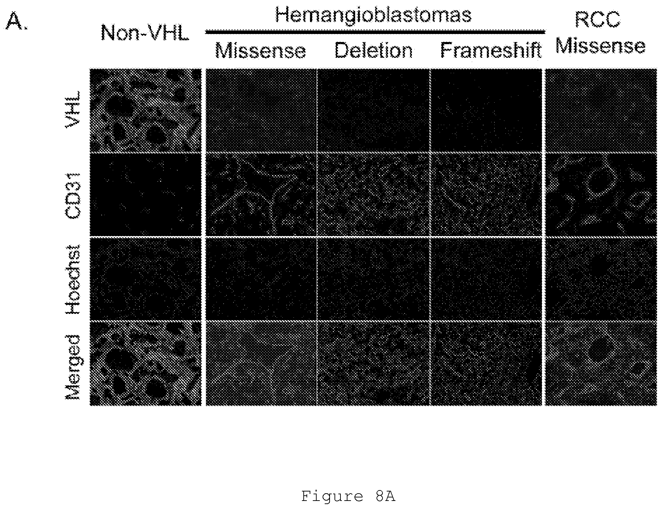

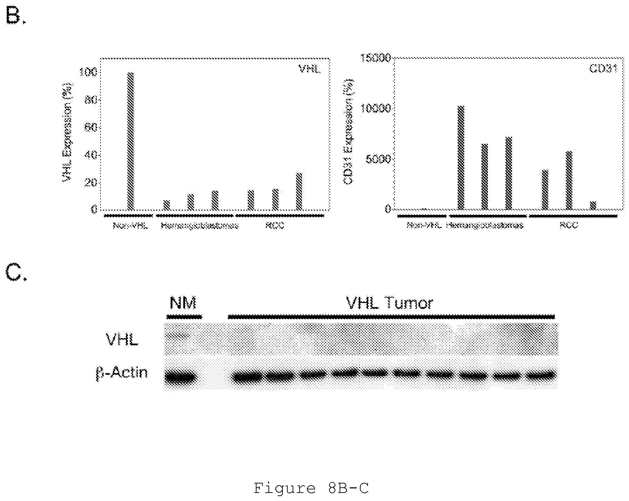

**Figure 8****. Quantification of VHL protein in HB and ccRCC.** (A) Immunofluorescence staining for VHL (greean) and CD31 (red) in VHL associated HB and ccRCC specimen. (B) Quantitative analysis of VHL and CD31 protein expression in HB and ccRCC with missense VHL mutations. (C) Western blot of VHL protein from various microdissected VHL associated HB samples.

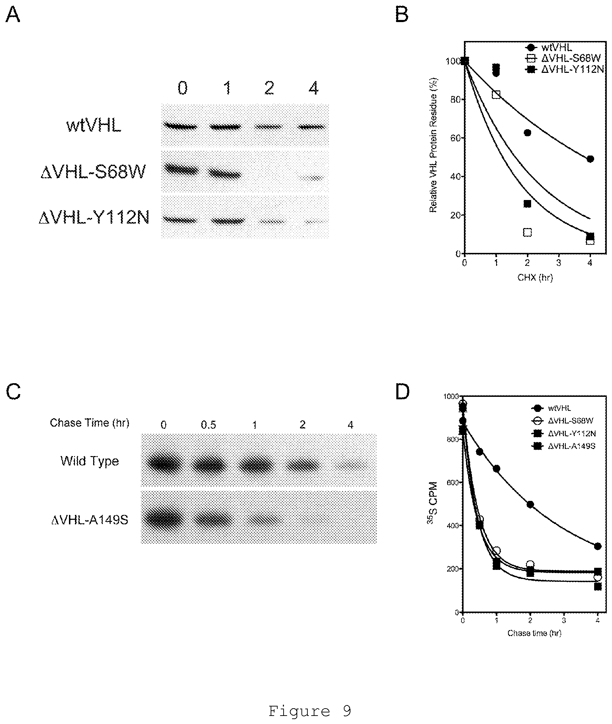

**Figure 9****. Half life kinetic analysis of reduction of mutant VHL proteins.** (A) Cycloheximide (CHX) treatment of wild type and VHL mutants at 0,1, 2 and 4 hr demonstrating increased turn over and degradation of mutant VHL compared to wild type control. (B) Calculation of protein degradation kinetic of wild type and VHL mutants. (C) Radioactive ³⁵S pulse chase assay for wild type and ΔVHL-A149S mutant at 0, 0.5, 1, 2 and 4 hr. (D) Liquid scintillation measurement of ³⁵S labeled VHL demonstrating fundamental losses of VHL protein stability in mutants compared to wild type control.

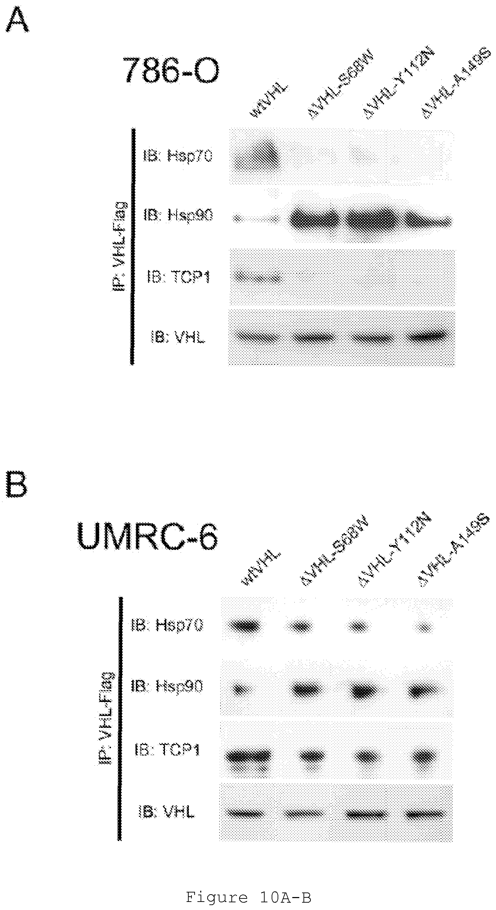

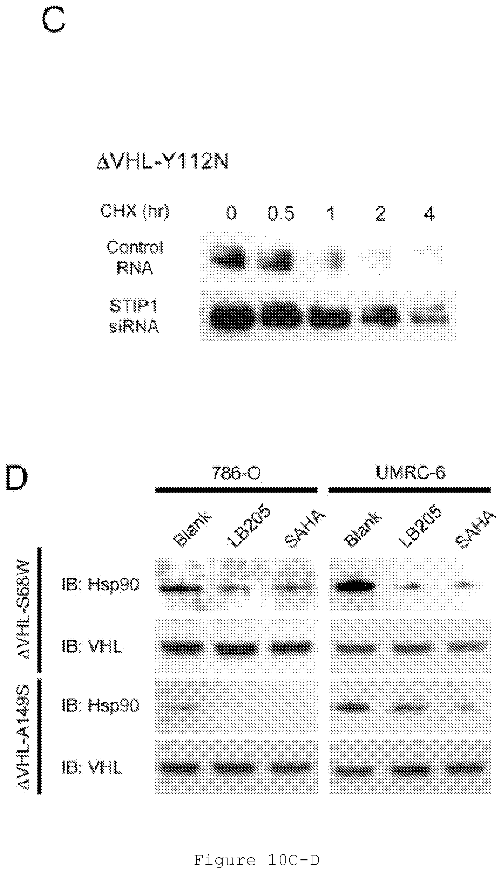

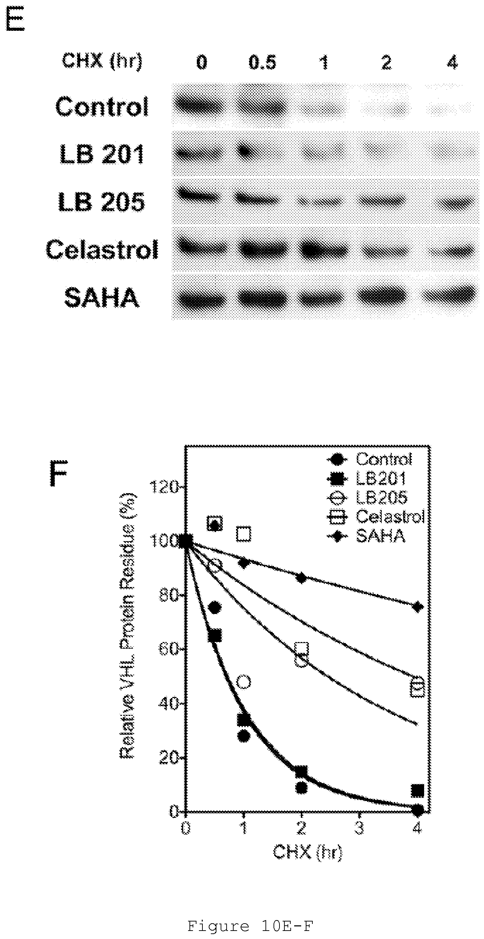

**Figure 10****. Increased survival VHL mutant protein after treatment with HDAC inhibitors.** (A and B) Immunoprecipitation assay for chaperonin binding to mutant VHL protein demonstrating loss of Hsp70 and TCP1 but increased Hsp90 binding. (C) Western blot for protein stability change of ΔVHL-Y112N with STIP1 RNA interference. (D) Immunoprecipitation assay for changes in chaperonin binding to mutant VHL protein after LB205 or SAHA treatment. (E) Western blot for ΔVHL-Y112N stability with proteostasis regulator treatments. (F) Quantification of protein half life.

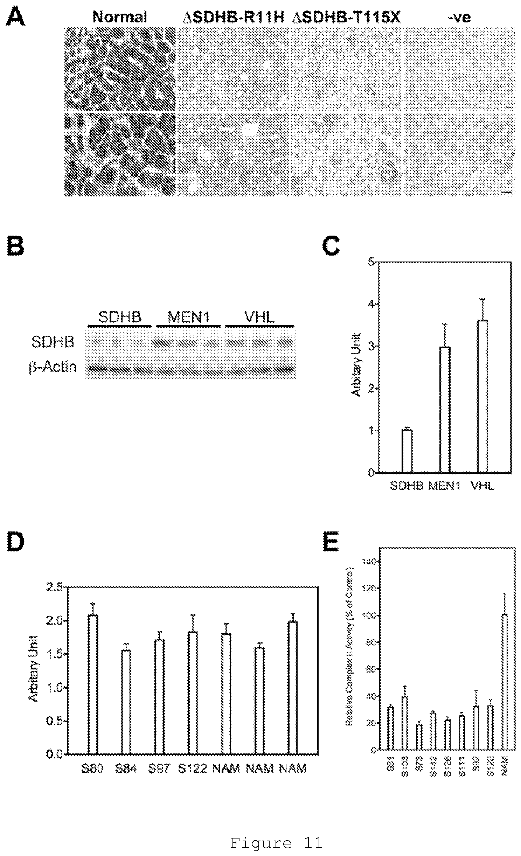

**Figure 11****. SDHB protein expression and enzymatic activity are altered in missense/nonsense mutation-associated tumor tissue.** (A) Immunohistochemical staining for SDHB in missense (ΔSDHB-R11H) and nonsense (ΔSDHB-T115X) associated tumor tissue. (B) Western blot of SDHB protein in microdissected PHEO/PGLs associated with mutations in SDHB, MEN1, or VHL gene. (C) Quantification of SDHB protein in microdissected tumor tissue. (D) Real time-PCR quantification of SDHB mRNA transcription in microdissected tumors. (E) SDHB enzyme activity of tumor samples with SDHB gene nonsense or missense mutations.

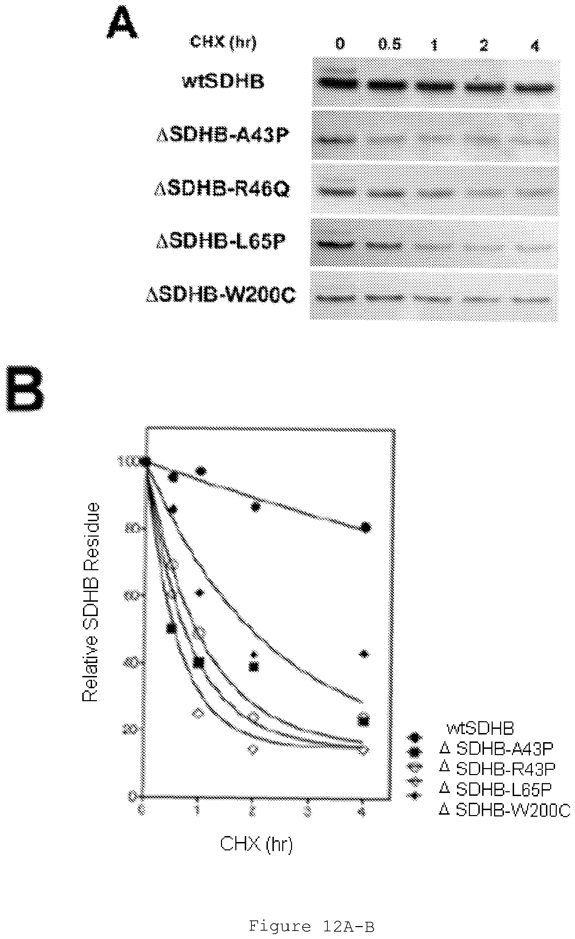

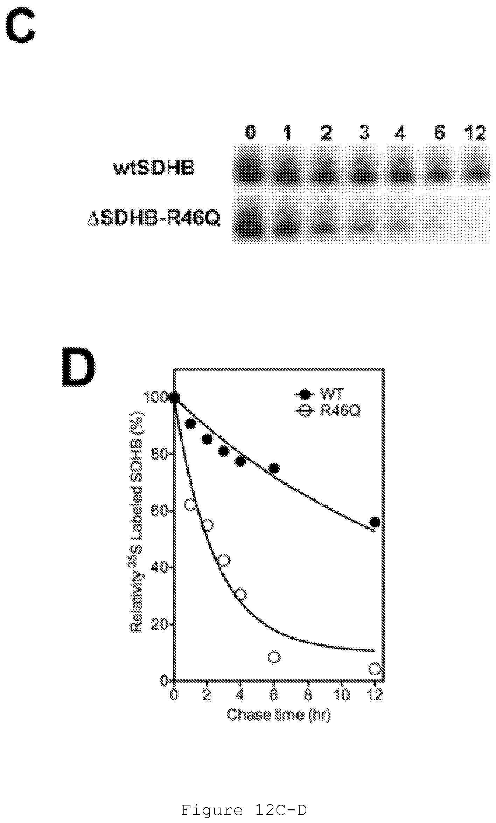

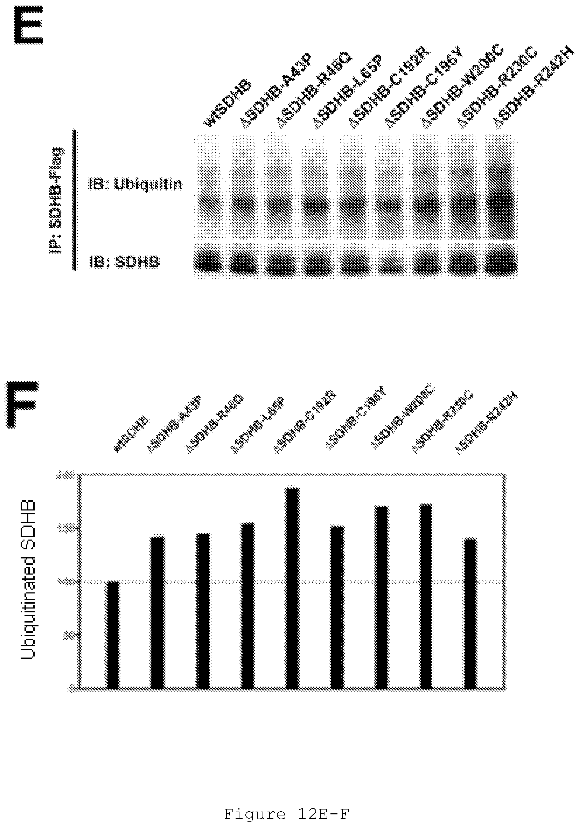

**Figure 12****. Missense mutantion in SDHB gene decrease protein stability.** (A) Western blot demonstrating protein stability loss of hotspot SDHB missense mutations through CHX treatment. (B) Hotspot missense mutations on SDHB protein decreases protein half-life. (C) [³⁵S]-methionine pulse chase assay measuring radiolabeled SDHB at 0-12 hrs in wild-type and R46Q mutant SDHB. (D) Liquid scintillation measurement of wild-type and R46Q mutant SDHB at 0-12 hrs after radiolabeling. (E) Immunoprecipitation assay of SDHB mutant protein ubiquitin binding. (F) Quantification of mutant SDHB ubiquitination relative to wild-type SDHB.

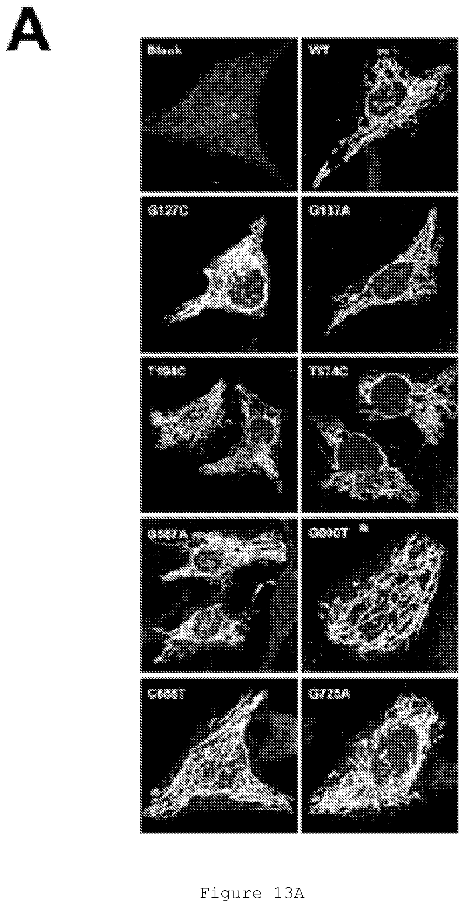

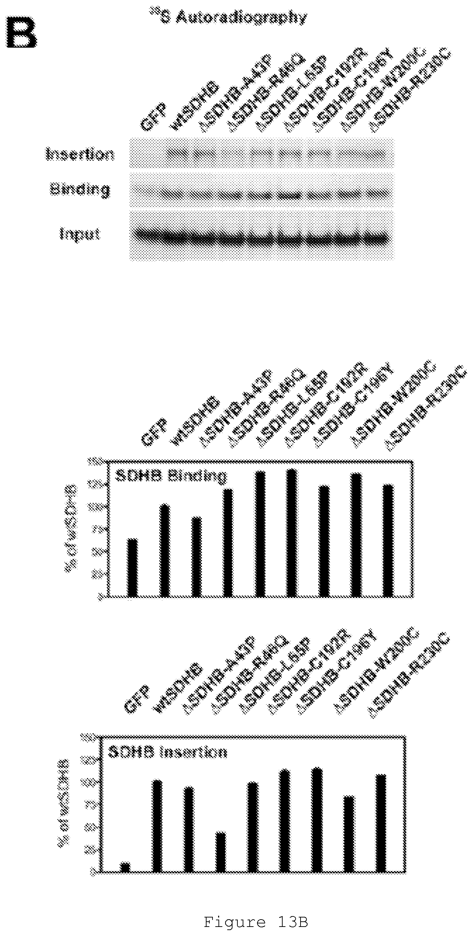

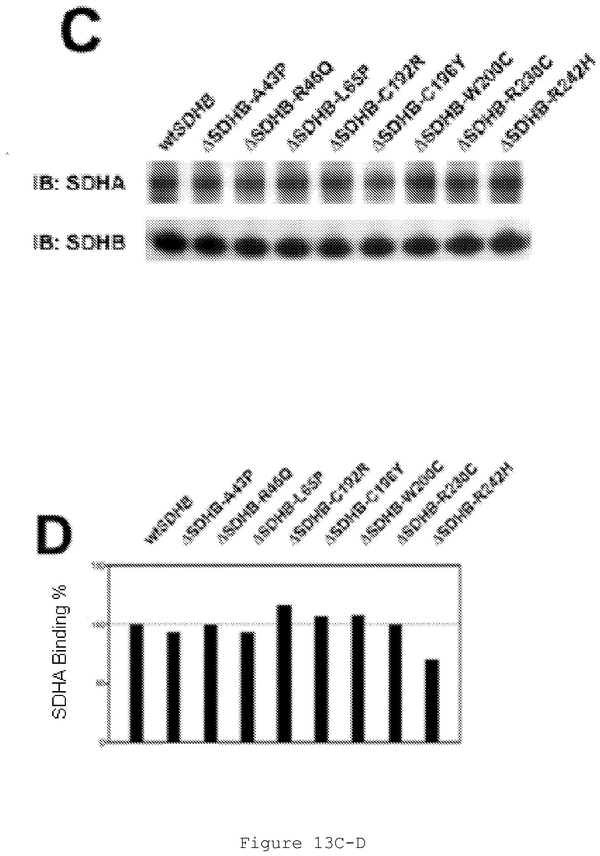

**Figure 13****. Normal mitochondria localization of missense SDHB mutant proteins.** (A) Immunofluorescence detection of SDHB mitochondria localization. Cells were labeled with anti-Flag (green), mitotracker (red) and Hoechst33342 (blue). (B) Autoradiography of [³⁵S]-methionine labeled SDHB binding and insertion into isolated mitochondria. Liquid scintillation quantification of binding and insertion of mutant SDHB proteins relative to wild-type SDHB. (C) Immunoprecipitation assay of SDHA binding to SDHB mutants. (D) Quantification of SDHA binding to mutant SDHB relative to wild-type SDHB.

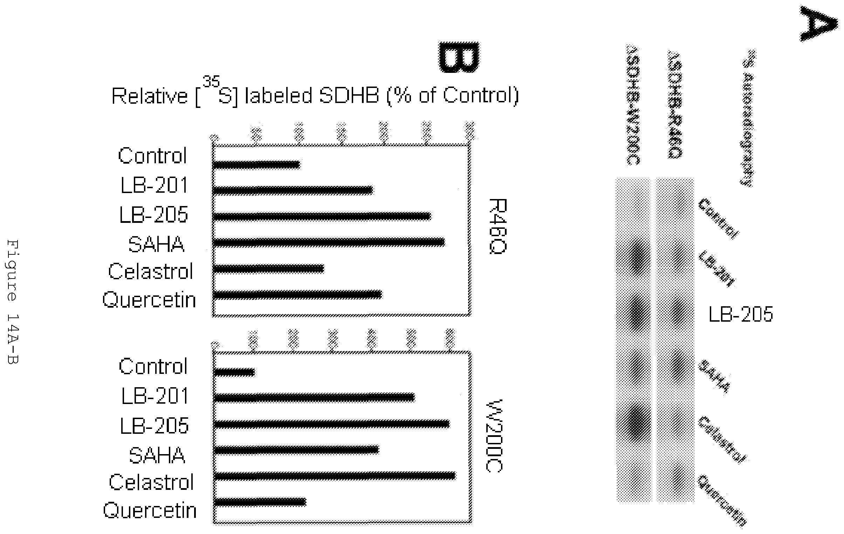

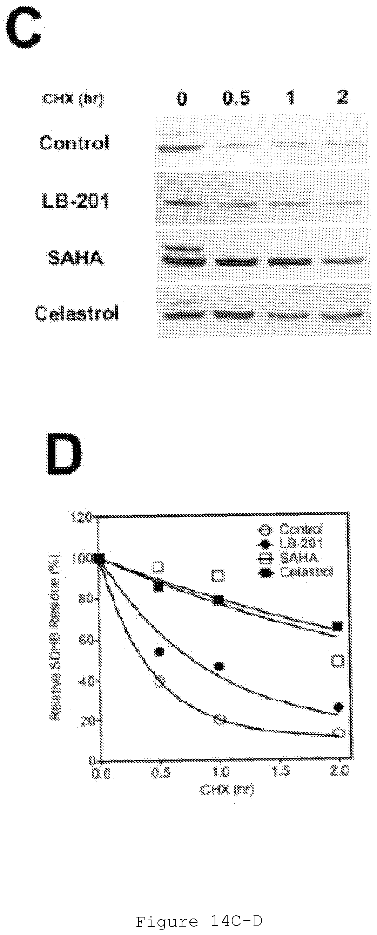

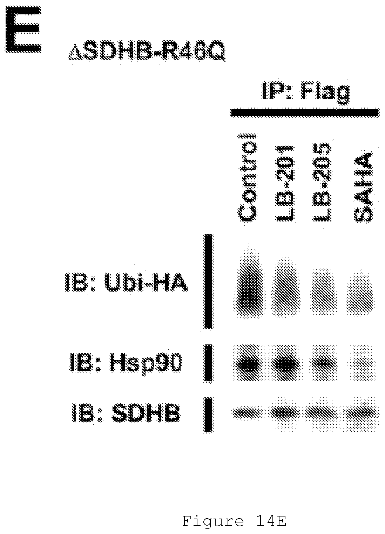

**Figure 14****. Effect of proteostasis modulators on SDHB protein stability.** (A) Autoradiography of [³⁵S] methionine labeled mutant SDHB at 4 hrs after administration of proteostasis modulators LB-201, LB-202, SAHA, celastrol, and quercetin. (B) Liquid scintillation quantification of [³⁵S]-methionine labeled SDHB at 4 hrs after administration of proteostasis modulators. (C) Western blot of ΔSDHB-L65P at 0-2 hrs after administration of CHX following treatment with LB-201, SAHA, and celestrol. (D) Relative quantity of ΔSDHB-L65P at 0-2 hrs after administration of CHX following treatment with LB-201, SAHA, and celestrol. (E) Western blot of ubiquitin and Hsp90 binding to immunoprecipitated ΔSDHB-R49Q following treatment with LB-201, LB-205, and SAHA.

### DETAILED DESCRIPTION OF THE INVENTION

The present invention provides a histone deacetylase inhibitor for use in the treatment of a mammalian subject afflicted with a disease characterized by a loss of protein function caused by a genetic abnormality associated with the disease as defined in claims 1 and 10.

In the present invention, the disease characterized by a loss of protein function is Gaucher's disease, von Hippel-Lindau disease, pheochromocytoma or paraganglioma.

In some embodiments, the disease characterized by a loss of protein function is Gaucher's disease.

The present invention discloses a histone deacetylase inhibitor for use in increasing the amount of a protein encoded by an abnormal gene in a human cell carrying the abnormal gene associated with a disease characterized by a loss of protein function.

In some embodiments, the protein is beta-glucocerebrosidase and the disease is Gaucher's Disease, the protein is von Hippel-Lindau tumor suppressor protein and the disease is von Hippel-Lindau disease, the protein is succinate dehydrogenase subunit B and the disease is pheochromocytoma or the protein is succinate dehydrogenase subunit B and the disease is paraganglioma.

In some embodiments, the histone deacetylase inhibitor is for use in increasing the amount of beta-glucocerebrosidase in a human cell carrying a genetic abnormality associated with Gaucher's Disease.

The present invention discloses a histone deacetylase inhibitor for use in increasing the half-life of a protein encoded by an abnormal gene in a human cell carrying the abnormal gene associated with a disease characterized by a loss of protein function.

In some embodiments, the protein is beta-glucocerebrosidase and the disease is Gaucher's Disease, the protein is von Hippel-Lindau tumor suppressor protein and the disease is von Hippel-Lindau disease, the protein is succinate dehydrogenase subunit B and the disease is pheochromocytoma or the protein is succinate dehydrogenase subunit B and the disease is paraganglioma.

In some embodiments, a histone deacetylase inhibitor is for use in increasing the half-life of beta-glucocerebrosidase in a human cell.

The present invention discloses a histone deacetylase inhibitor for use in decreasing the degradation of a protein encoded by an abnormal gene in a human cell the abnormal gene associated with a disease characterized by a loss of protein function.

In some embodiments, the protein is beta-glucocerebrosidase and the disease is Gaucher's Disease, the protein is von Hippel-Lindau tumor suppressor protein and the disease is von Hippel-Lindau disease, the protein is succinate dehydrogenase subunit B and the disease is pheochromocytoma or the protein is succinate dehydrogenase subunit B and the disease is paraganglioma.

In some embodiments, a histone deacetylase inhibitor is for use in decreasing the degradation of beta-glucocerebrosidase in a human cell.

Described herein are compounds for use effective for the treatment of a disease characterized by a loss of protein function.

Described herein are compounds for use effective for the treatment of Gaucher's Disease, von-Hippel Landaul Disease, Pheochromocytoma or Paraganglioma.

The present invention discloses a histone deactylase inhibitor for use in increasing the amount of beta-glucocerebrosidase in a human cell carrying a genetic abnormality associated with Gaucher's Disease relative to such a cell not contacted with the compound.

The present invention discloses a histone deactylase inhibitor for use in increasing the half-life of beta-glucocerebrosidase in a human cell relative to such a cell not contacted with the compound.

The present invention also discloses a histone deacetylase inhibitor for use in decreasing the degradation of beta-glucocerebrosidase in a human cell relative to such a cell not contacted with the compound.

In one embodiment, the protein phosphatase 2A inhibitor is used in combination together with the histone deacetylase inhibitor.

In one embodiment, the protein phosphatase 2A inhibitor has the structure 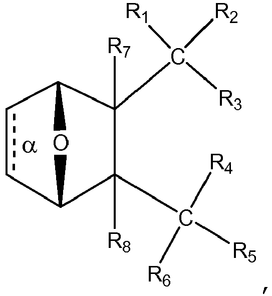 wherein
bond α is present or absent;
R₁ and R₂ is each independently H, O⁻ or OR₉,
   where R₉ is H, alkyl, alkenyl, alkynyl or aryl,
or R₁ and R₂ together are =O;
R₃ and R₄ are each different, and each is OH, O⁻, OR₉, SH, S⁻, SR₉, 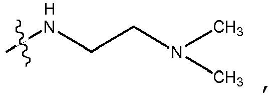 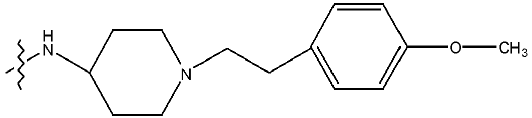 or 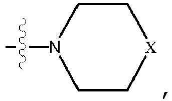 where X is O, S, NR₁₀, or N⁺R₁₀R₁₀,
   where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =O, 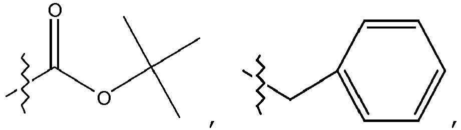
   -CH₂CN, -CH₂CO₂R₁₁, -CH₂COR₁₁, -NHR₁₁ or -NH⁺ (R₁₁)₂, where each R₁₁ is independently alkyl, alkenyl or alkynyl, each of which is substituted or unsubstituted, or H;
R₅ and R₆ is each independently H, OH, or R₅ and R₆ taken together are =O; and
R₇ and R₈ is each independently H, F, Cl, Br, SO₂Ph, CO₂CH₃, or SR₁₂,
   where R₁₂ is H, aryl or a substituted or unsubstituted alkyl, alkenyl or alkynyl,
or a salt, enantiomer or zwitterion of the compound.

In one embodiment, the protein phosphatase 2A inhibitor has the structure

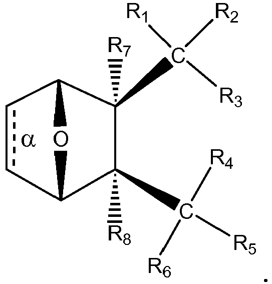

In one embodiment, the protein phosphatase 2A inhibitor has the structure

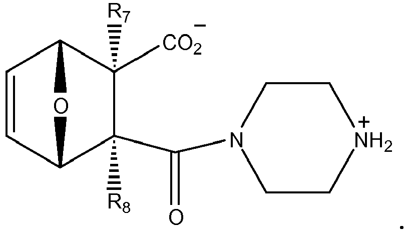

In one embodiment, the protein phosphatase 2A inhibitor has the structure

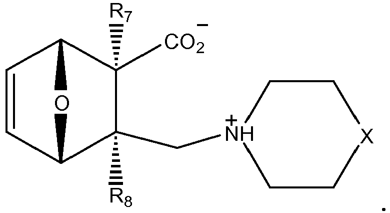

In one embodiment, bond α is present. In another embodiment, bond α is absent.

In one embodiment,
R₁ and R₂ together are =O;
R₃ is O⁻ or OR₉,
   where R₉ is H, methyl, ethyl or phenyl;
R₄ is 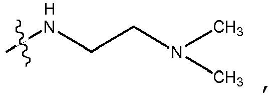 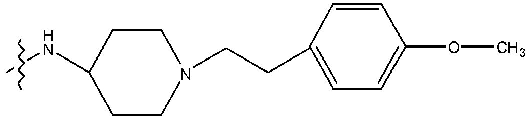 or 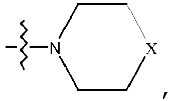 where X is O, S, NR₁₀, or N⁺R₁₀R₁₀,
   where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 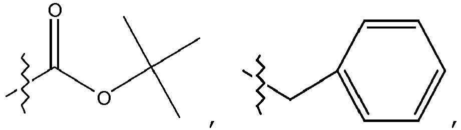
   -CH₂CN, -CH₂CO₂R₁₁, -CH₂COR₁₁, -NHR₁₁ or -NH⁺ (R₁₁)₂, where R₁₁ is alkyl, alkenyl or alkynyl, each of which is substituted or unsubstituted, or H;
R₅ and R₆ taken together are =O; and
R₇ and R₈ is each independently H, F, Cl, Br, SO₂Ph, CO₂CH₃, or SR₁₂,
   where R₁₂ is a substituted or unsubstituted alkyl, alkenyl or alkynyl.

In one embodiment, R₃ is O⁻. In another embodiment, R₄ is 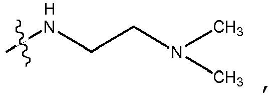 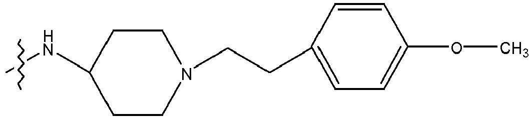 or 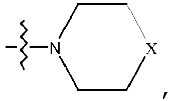 where X is O, NR₁₀, N⁺R₁₀R₁₀
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =O, 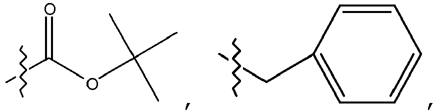
-CH₂CN, -CH₂CO₂R₁₁, -CH₂COR₁₁, -NHR₁₁ or -NH⁺ (R₁₁)₂, where R₁₁ is H or alkyl.

In one embodiment, the protein phosphatase inhibitor 2A has the structure 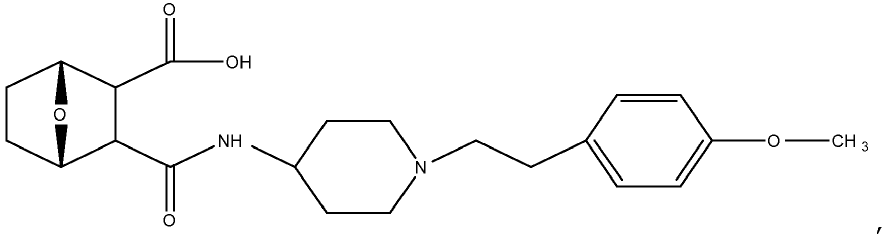 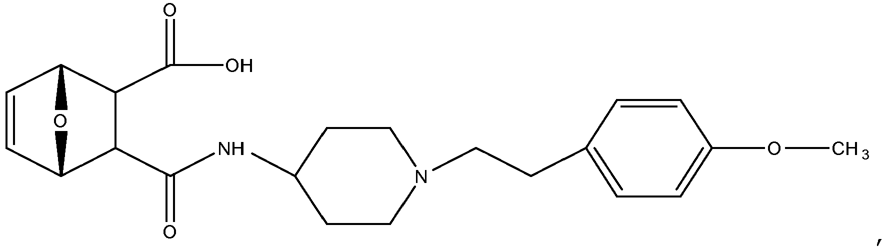 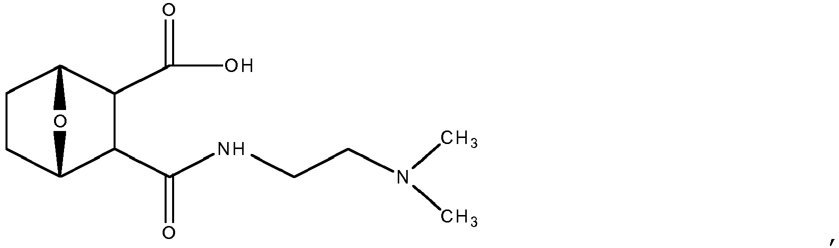 or

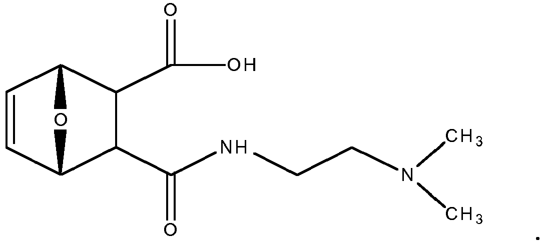

In one embodiment, R₄ is 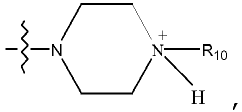 where R₁₀ is R₁₀ H, alkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =O, 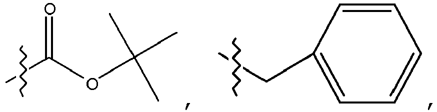 -CH₂CN, -CH₂CO₂R₁₁, -CH₂COR₁₁, -NHR₁₁ or -NH⁺(R₁₁)₂,
where R₁₁ is H or alkyl.

In one embodiment, R₄ is

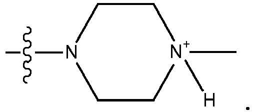

In one embodiment, R₄ is 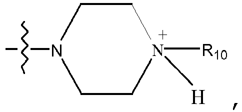 where R₁₀ is

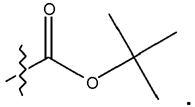

In one embodiment, R₄ is 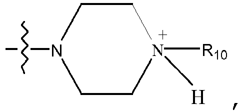 where R₁₀ is

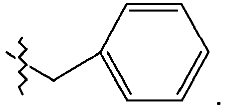

In one embodiment, R₄ is

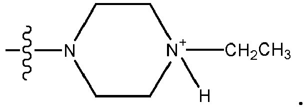

In one embodiment, R₄ is

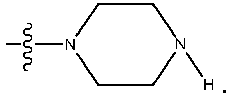

In one embodiment, R₅ and R₆ together are =O. In another embodiment, R₇ and R₈ are each H.

In one embodiment, 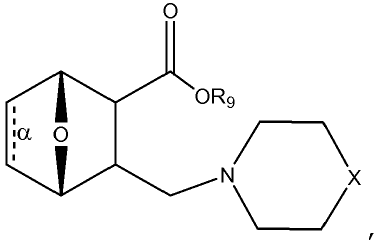 wherein bond α is present or absent; R₉ is present or absent and when present is H, C₁-C₁₀ alkyl, C₂-C₁₀ alkenyl or phenyl; and X is O, S, NR₁₀ or N⁺R₁₀R₁₀,
where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 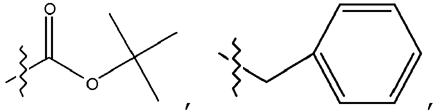 -CH₂CO₂R₁₁, -CH₂COR₁₁, -CH₂CN, or -CH₂CH₂R₁₆, where R₁₁ is H or alkyl, and where R₁₆ is any substitutent that is a precursor to an aziridinyl intermediate,
or a salt, zwitterion or enantiomer of the compound.

In one embodiment, the protein phosphatase 2A inhibitor has the structure 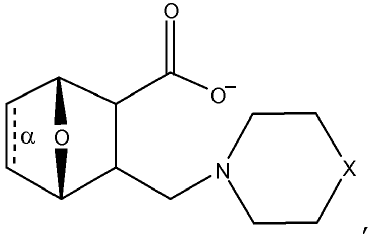 wherein,
bond α is present or absent;
X is O, S, NR₁₀ or N⁺R₁₀R₁₀,
   where each R₁₀ is independently H, alkyl, substituted C₂-C₁₂ alkyl, alkenyl, substituted C₄-C₁₂ alkenyl, alkynyl, substituted alkynyl, aryl, substituted aryl where the substituent is other than chloro, 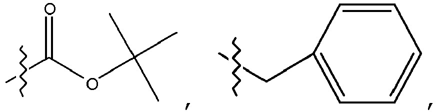 -CH₂CO₂R₁₁, -CH₂COR₁₁, -CH₂CN, or -CH₂CH₂R₁₆, where R₁₁ is H or alkyl, and where R₁₆ is any substitutent that is a aziridinyl intermediate,
or a salt, zwitterion or enantiomer of a compound.

In one embodiment, X is O or NH⁺R₁₀,
where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro,

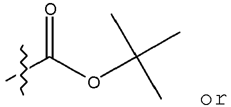

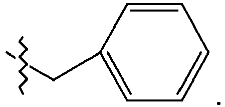

In one embodiment, X is -CH₂CH₂R₁₆, where R₁₆ is any substitutent that is a precursor to an aziridinyl intermediate.

In one embodiment, X is O. In another embodiment, X is NH⁺R₁₀,
where R₁₀ H, alkyl, substituted C₂-C₁₂ alkyl, alkenyl, substituted C₄-C₁₂ alkenyl, alkynyl, substituted alkynyl, aryl, substituted aryl where the substituent is other than chloro, 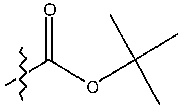 or

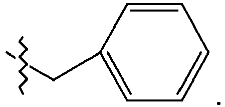

In one embodiment, R₁₀ is methyl. In another embodioment, R₁₀ is

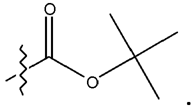

In one embodiment, R₁₀ is

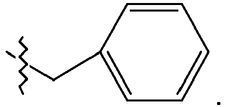

In one embodiment, R₁₀ is ethyl. In another embodiment, R₁₀ is absent.

In one embodiment, the protein phosphatase 2A inhibitor has the structure 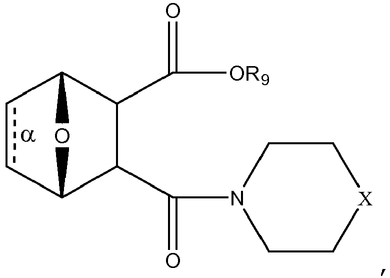 wherein
bond α is present or absent;
R₉ is present or absent and when present is H, alkyl, alkenyl, alkynyl or phenyl; and
X is O, NR₁₀, or N⁺R₁₀R₁₀,
   where each R₁₀ is indepdently H, alkyl, substituted C₂-C₁₂ alkyl, alkenyl, substituted C₄-C₁₂ alkenyl, alkynyl, substituted alkynyl, aryl, substituted aryl where the substituent is other than chloro, 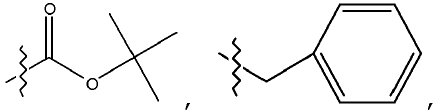 -CH₂CN, -CH₂CO₂R₁₂, or -CH₂COR₁₂, where R₁₂ is H or alkyl,
or a salt, zwitterion, or enantiomer of the compound.

In one embodiment, the protein phosphatase 2A inhibitor has the structure 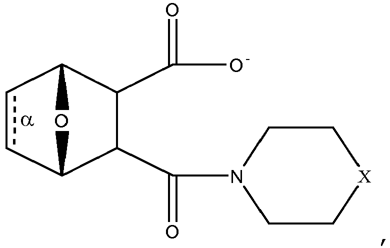 wherein
bond a is present or absent;
X is O or NH⁺R₁₀,
   where R₁₀ is H, alkyl, substituted C₂-C₁₂ alkyl, alkenyl, substituted C₄-C₁₂ alkenyl, alkynyl, substituted alkynyl, aryl, substituted aryl where the substituent is other than chloro, 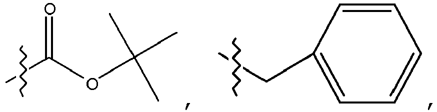 -CH₂CN, -CH₂CO₂R₁₂, or -CH₂COR₁₂, where R₁₂ is H or alkyl.

In one embodiment, bond α is present. In another embodiment, bond α is absent.

In one embodiment, the protein phosphatase 2A inhibitor has the structure 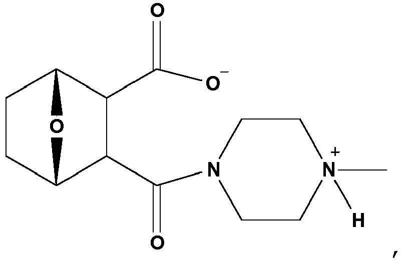 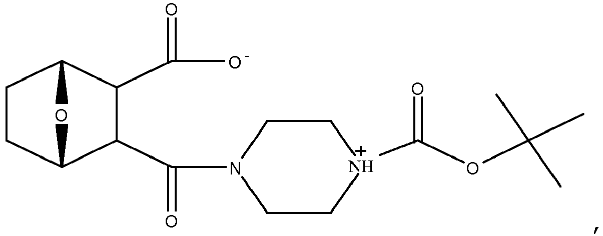 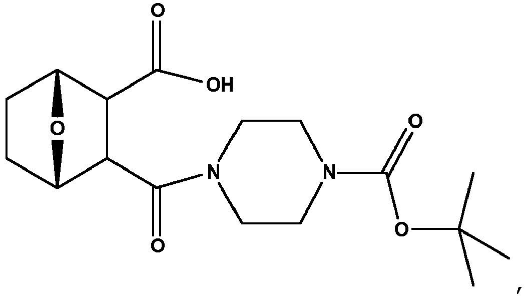 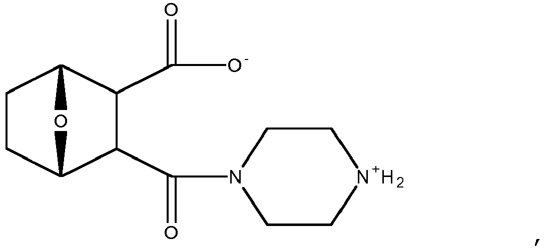 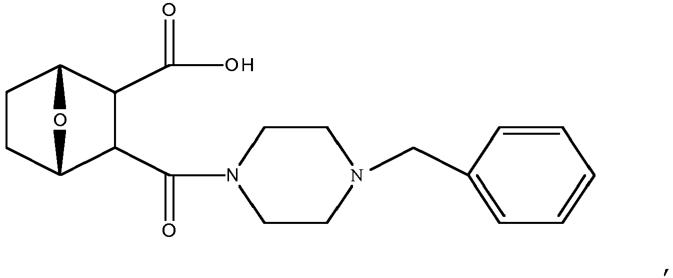 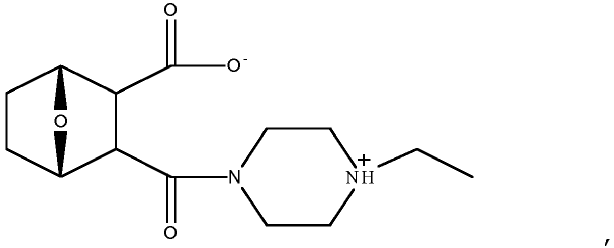 or

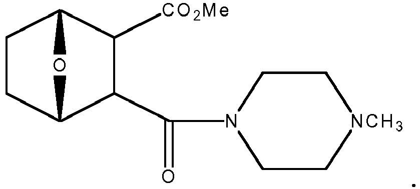

In one embodiment, the protein phosphatase 2A inhibitor has the structure 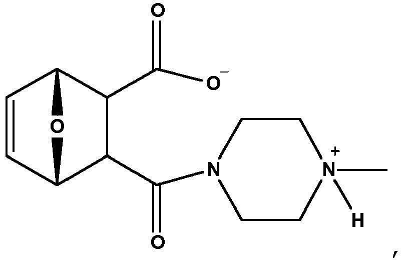 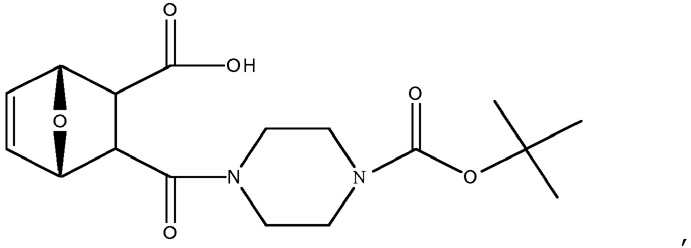 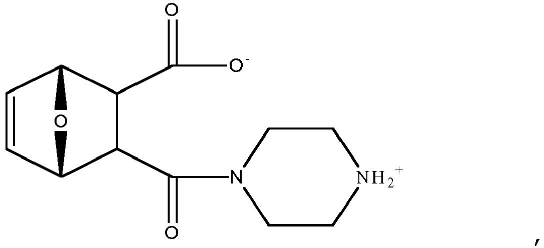 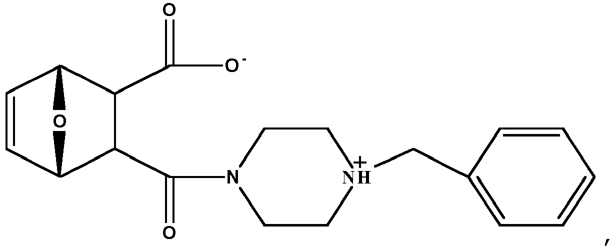 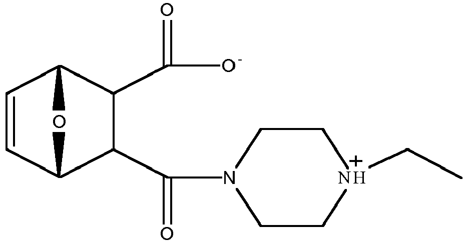 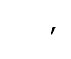 or

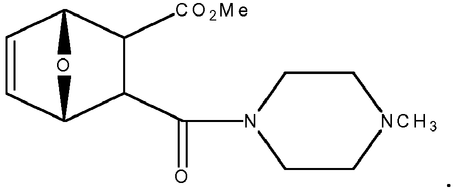

In one embodiment, the protein phosphatase 2A inhibitor has the structure 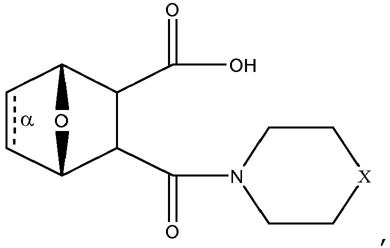 wherein
bond α is present or absent; X is NH⁺R₁₀,
where R₁₀ is present or absent and when present R₁₀ is is alkyl, substituted C2-C12 alkyl, alkenyl, substituted C4-C12 alkenyl, 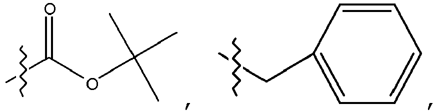 -CH₂CN, -CH₂CO₂R₁₂, or -CH₂COR₁₂, where R₁₂ is H or alkyl.

In one embodiment, the protein phosphatase 2A inhibitor has the structure

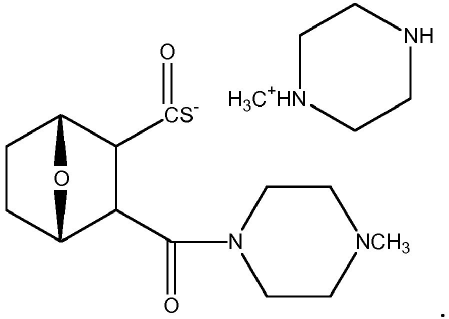

In one embodiment, the protein phosphatase 2A inhibitor has the structure 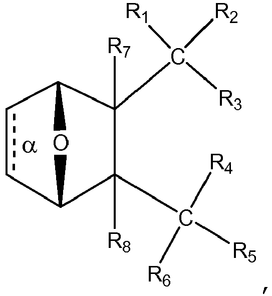 wherein
bond α is present or absent;
R₁ and R₂ is each independently H, O⁻ or OR₉,
   where R₉ is H, alkyl, substituted alkyl, alkenyl, alkynyl or aryl,
or R₁ and R₂ together are =O;
R₃ and R₄ are each different, and each is O(CH₂)₁₋₆R₉ or OR₁₀, or 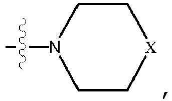
   where X is O, S, NR₁₁, or N⁺R₁₁R₁₁,
      where each R₁₁ is independently H, alkyl, hydroxyalkyl, substituted C₂-C₁₂ alkyl, alkenyl, substituted C₄-C₁₂ alkenyl, alkynyl, substituted alkynyl, aryl, substituted aryl where the substituent is other than chloro when R₁ and R₂ are =O, 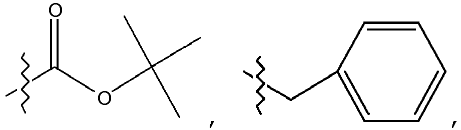 -CH₂CN, -CH₂CO₂R₁₂, -CH₂COR₁₂, -NHR₁₂ or -NH⁺(R₁₂)₂,
      where each R₁₂ is independently alkyl, alkenyl or alkynyl, each of which is substituted or unsubstituted, or H;
   where R₁₀ is substituted alkyl, substituted alkenyl, substituted alkynyl, or substituted aryl,
or R₃ and R₄ are each different and each is OH or 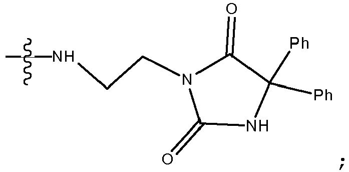
R₅ and R₆ is each independently H, OH, or R₅ and R₆ taken together are =O; and
R₇ and R₈ is each independently H, F, Cl, Br, SO₂Ph, CO₂CH₃, or SR₁₃,
   where R₁₃ is H, aryl or a substituted or unsubstituted alkyl, alkenyl or alkynyl,
or a salt, enantiomer or zwitterion of the compound.

In one embodiment, the protein phosphatase 2A inhibitor has the structure

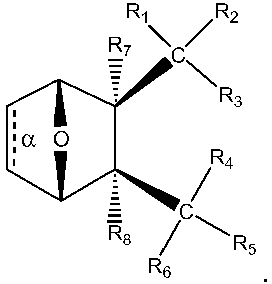

In one embodiment, bond α is present. In another embodiment, bond α is absent.

In one embodiment,
R₃ is OR₉ or O (CH₂)₁₋₆R₁₀,
   where R₉ is aryl or substituted ethyl;
   where R₁₀ is substituted phenyl, wherein the substituent is in the para position;
R₄ is 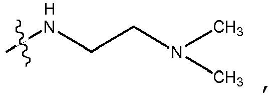 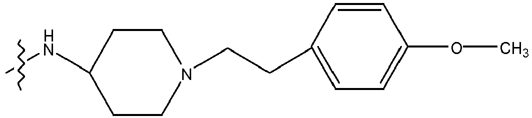 or 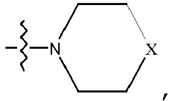 where X is O, S, NR₁₁, or N⁺R₁₁R₁₁,
   where each R₁₁ is independently H, alkyl, hydroxyalkyl, substituted C₂-C₁₂ alkyl, alkenyl, substituted C₄-C₁₂ alkenyl, alkynyl, substituted alkynyl, aryl, substituted aryl where the substituent is other than chloro, 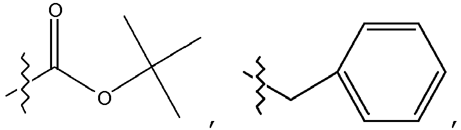 -CH₂CN, -CH₂CO₂R₁₂, -CH₂COR₁₂, -NHR₁₂ or -NH⁺ (R₁₂)₂, where R₁₂ is alkyl, alkenyl or alkynyl, each of which is substituted or unsubstituted, or H;
or where R3 is OH and R4 is

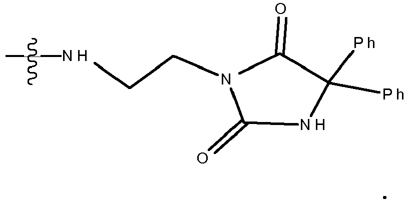

In one embodiment, R₄ is 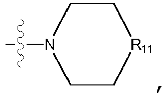 where R₁₁ is alkyl or hydroxylalkyl
or R₄ is 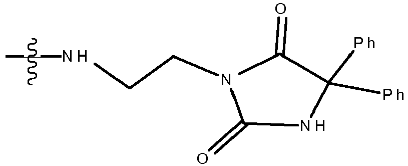 when R₃ is OH.

In one embodiment,
R₁ and R₂ together are =O;
R₃ is OR₉ or OR₁₀ or O (CH₂)₁₋₂R₉,
   where R₉ is aryl or substituted ethyl;
   where R₁₀ is substituted phenyl, wherein the substituent is in the para position;
or R₃ is OH and R₄ is 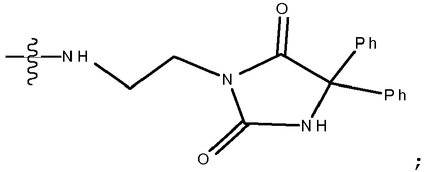
R₄ is 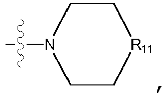 where R₁₁ is alkyl or hydroxyl alkyl;
R₅ and R₆ together are =O; and
R₇ and R₈ are each independently H.

In one eombodiment,
R₁ and R₂ together are =O;
R₃ is OH, O (CH₂)R₉, or OR₁₀,
   where R₉ is phenyl;
   where R₁₀ is CH₂CCl₃, 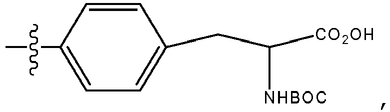
   or 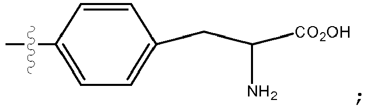
R₄ is 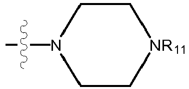 or 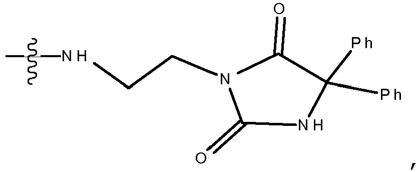 where R₁₁ is CH₃ or CH₃CH₂OH;
R₅ and R₆ together are =O; and
R₇ and R₈ are each independently H.

In one embodiment, R₃ is OR₁₀
where R₁₀ is (CH₂)₁₋₆(CHNHBOC)CO₂H, (CH₂)₁₋₆(CHNH₂)CO₂H, or (CH₂)₁-₆CCl₃.

In one embodiment, R₁₀ is CH₂ (CHNHBOC) CO₂H. In another embodiment, R₁₀ is CH₂(CHNH₂)CO₂H. In one embodiment, R₁₀ is CH₂CCl₃.

In one embodiment, R₃ is O (CH₂)₁₋₆R₉ where R₉ is phenyl. In another embodiment, R₃ is O(CH₂)R₉ where R9 is phenyl. In another embodiment, R₃ is OH and R₄ is

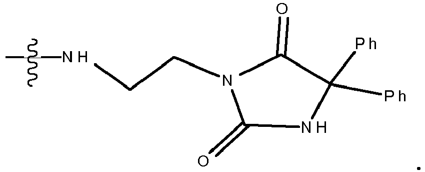

In one embodiment, R₄ is 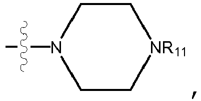 wherein R₁₁ is hydroxyalkyl.

In one embodiment, R₁₁ is -CH₂CH₂OH.

In one embodiment, R₄ is 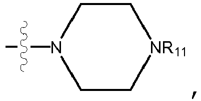 wherein R₁₁ is alkyl.

In one embodiment, R₁₁ is -CH3.

In one embodiment, R₄ is

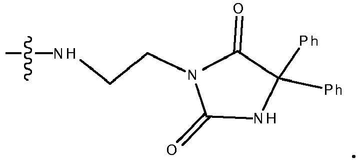

In one embodiment, the protein phosphatase 2A inhibitor has the structure 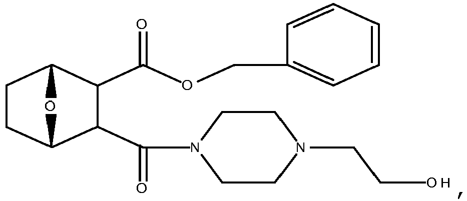 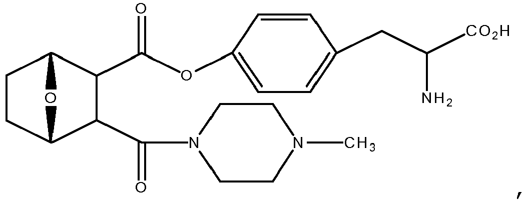 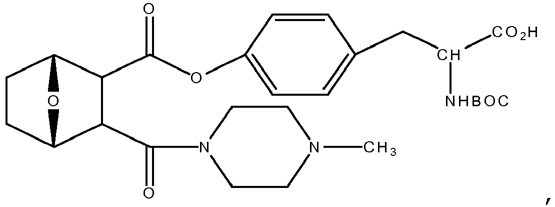 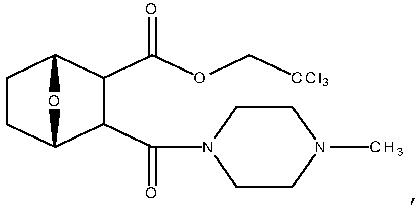 or

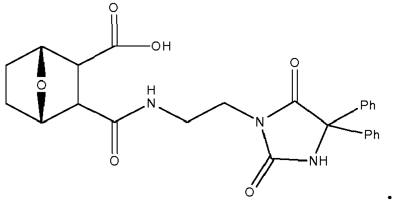

In one embodiment, the protein phosphatase 2A inhibitor has the structure 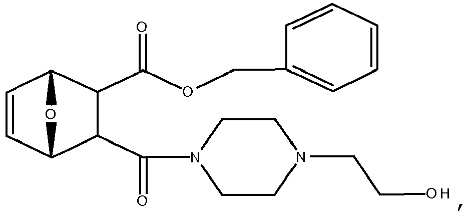 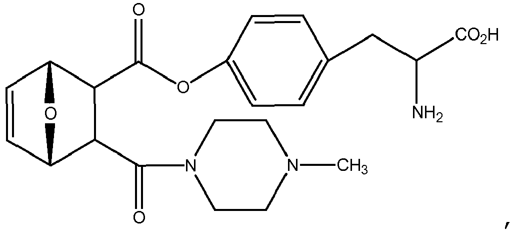 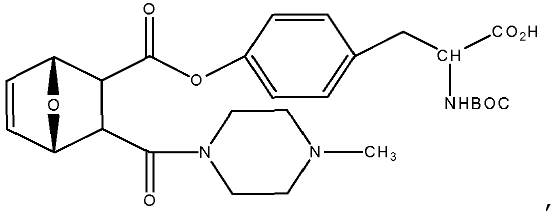 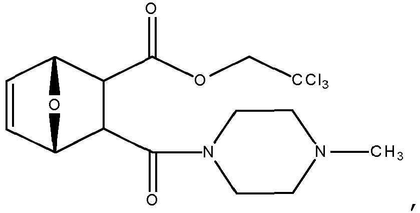 or

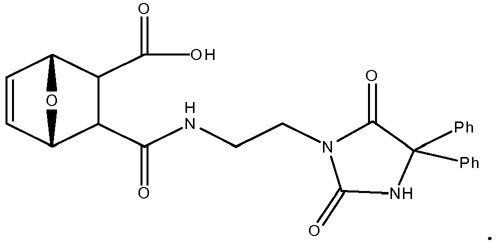

In one embodiment, the histone deacetylase inhibitor has the structure wherein 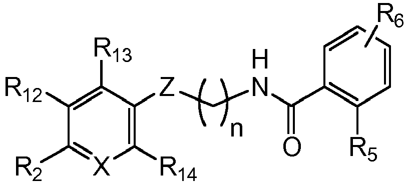
n is 1-10;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
Z is 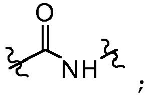
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, SO₂R₁₅, NO₂, trifluoromethyl, methoxy, or CO-R₁₅,
wherein R₁₅ is alkyl, alkenyl, alkynyl, C₃-C₈ cycloalkyl, or aryl, or
a salt of the compound.

In one embodiment, the histone deacetylase inhibitor has the structure 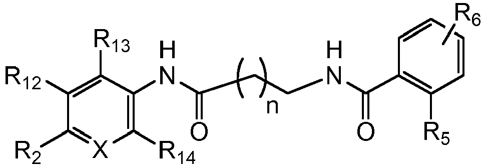 wherein
n is 1-9;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, SO₂R₁₅, NO₂, trifluoromethyl, methoxy, or CO-R₁₅, wherein R₁₅ is alkyl, alkenyl, alkynyl, C₃-C₈ cycloalkyl, or aryl.

In one embodiment, the histone deacetylase inhibitor has the structure 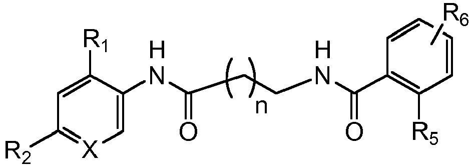 wherein
n is 1-8;
X is CH or N;
R₁ is H or OH;
R₂ is H or NR₃R₄, wherein R₃ and R₄ are each independently C₁-C₆ alkyl or C₃-C₈ cycloalkyl;
R₅ is OH or SH; and
R₆ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl.

In one embodiment, the histone deacetylase inhibitor has the structure 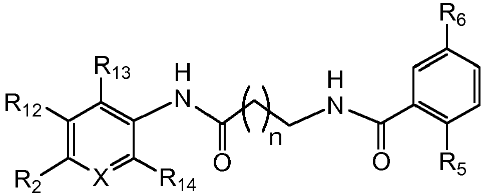 wherein
n is 1-9;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, trifluoromethyl, methoxy, or CO-R₁₅, wherein R₁₅ is alkyl, alkenyl, alkynyl, C₃-C₈ cycloalkyl, or aryl.

In one embodiment, the histone deacetylase inhibitor has the structure 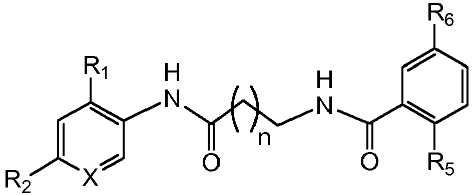 wherein
n is 1-8;
X is CH or N;
R₁ is H or OH;
R₂ is H or NR₃R₄, wherein R₃ and R₄ are each independently C₁-C₆ alkyl or C₃-C₈ cycloalkyl;
R₅ is OH or SH; and
R₆ is H, OH, SH, F, Cl, trifluoromethyl, methoxy, or CO-R₇, wherein R₇ is alkyl, alkenyl, alkynyl, or C₃-C₈ cycloalkyl, or aryl.

In one embodiment, the histone deacetylase inhibitor has the structure 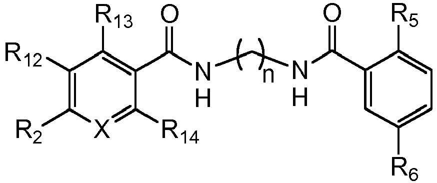 wherein
n is 1-8;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently C₁-C₆ alkyl or C₃-C₈ cycloalkyl; and
R₅ is OH or SH; and
R₆, R₁₂, R₁₃, and R₁₄ are each independently is H, OH, SH, F, Cl, SO₂R₁₅, NO₂, trifluoromethyl, methoxy, or CO-R₁₅, wherein R₁₅ is alkyl, alkenyl, alkynyl, C₃-C₈ cycloalkyl, or aryl, or
a salt of the compound.

In one embodiment, the histone deacetylase inhibitor has the structure 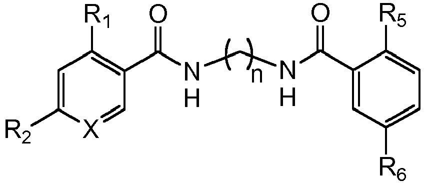 wherein
n is 1-8;
X is CH or N;
R₁ is H, OH or SH;
R₂ is H or NR₃R₄, wherein R₃ and R₄ are each independently C₁-C₆ alkyl or C₃-C₈ cycloalkyl; and
R₅ is OH or SH; and
R₆ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl, or
a salt of the compound.

In one embodiment, R₅ or R₆ is SH, and the aromatic ring bearing the SH group is a benzenoid, aza, or polyaza-aromatic five- or six-membered ring.

In one embodiment, R₁ and R₂ are H, X is CH, R₅ is SH, R₆ is H, and n is 4. In another embodiment, R₁ is OH, R₂ is H, X is CH, R₅ is OH, R₆ is H, and n is 6. In another embodiment, R₁ is SH, R₂ is H, X is CH, R₅ is SH, R₆ is H, and n is 6. In one embodiment, R₁ and R₂ are H, X is N, R₅ is SH, R₆ is H, and n is 4. In one embodiment, R₁ is H, R₂ is NR₃R₄, wherein R₃ and R₄ are each C₁ alkyl, X is CH, R₅ is SH, R₆ is H, and n is 4. In one embodiment, R₁ and R₂ are H, X is N, R₅ is SH, R₆ is Cl, and n is 4. In another embodiment, R₁ and R₂ are H, X is N, R₅ is SH, R₆ is H, and n is 5. In one embodiment, R₁ is H, R₂ is NR₃R₄, wherein R₃ and R₄ are each H, X is CH, R₅ is SH, R₆ is H, and n is 4. In one embodiment, R₁ and R₂ are H, X is CH, R₅ is SH, R₆ is Cl, and n is 4. In one embodiment, R₁ and R₂ are H, X is CH, R₅ is SH, R₆ is methoxy, and n is 4. In one embodiment, R₁ and R₂ are H, X is CH, R₅ is SH, R₆ is H, and n is 5. In one embodiment, R₁ and R₂ are H, X is CH, R₅ is SH, R₆ is H, and n is 6. In one embodiment, R₁ and R₂ are H, X is CH, R₅ is SH, R₆ is H, and n is 9.

In one embodiment, the HDAC inhibitor has the structure 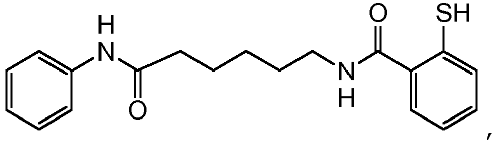 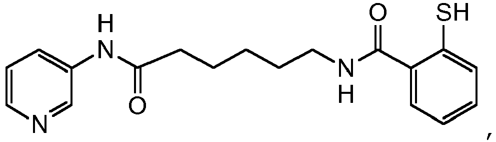 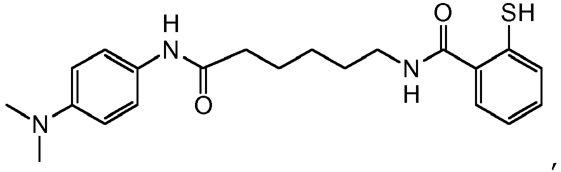 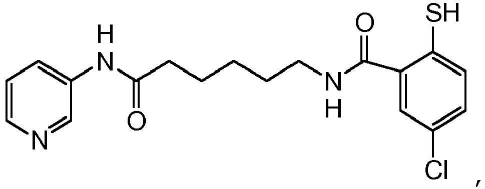 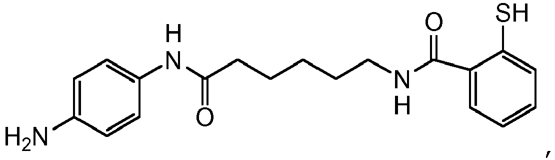 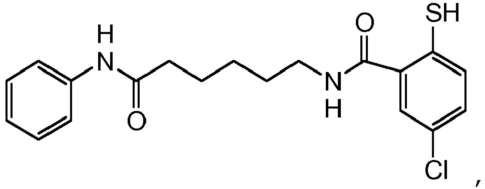 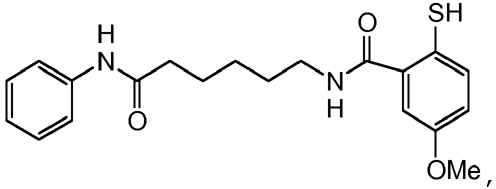 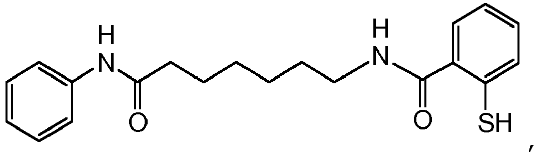 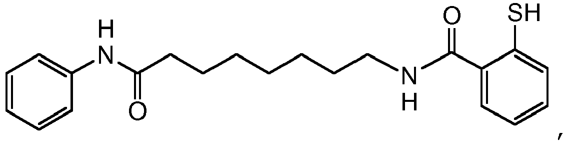 or

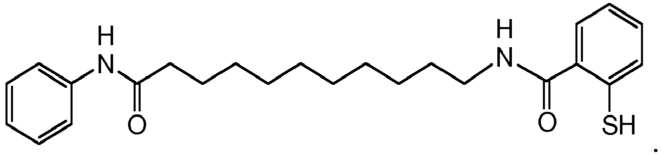

In one embodiment, the histone deacetylase inhibitor has the structure 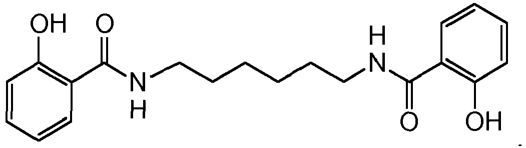 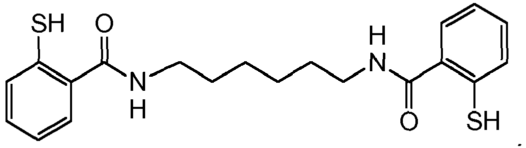 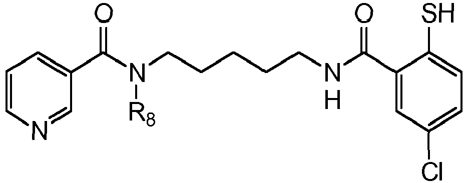 wherein R₈ = alkyl, or aryl, or

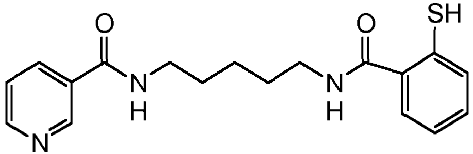

For the foregoing embodiments, each embodiment disclosed herein is contemplated as being applicable to each of the other disclosed embodiments.

### Definitions

As used herein, and unless otherwise stated, each of the following terms shall have the definition set forth below.

As used herein, "disease characterized by a loss of protein function" is any disease wherein loss of protein function is a factor in the cause and/or progression of the disease.

As used herein, a "loss of protein function disease" or a "loss of function disease" is a "disease characterized by a loss of protein function" as defined above. Disclosed herein are loss of function diseases in which the treatment stabilizes a mutant protein and increases function. Examples of a disease characterized by a loss of protein function include Gaucher' s disease, von Hippel-Lindau disease, cystic fibrosis, Phenylketonuria, Fabry disease, Tay-Sachs disease, Pompe disease, Neimann-Pick disease (Type A, B and C), Marfan syndrome, Hemophilia A & B, retinitis pigmentosa, Neurofibromatosis Type 2, pheochromocytoma, paraganglioma, Multiple Endocirne Neoplasia Type 1, Familial Hypercholesterolemia, Hurler's disease, Hunter syndrome, Sanfilippo syndrome, Morquio syndrome, Maroteaux-Lamy syndrome, Sly syndrome, Sandhoff's disease, Fucosidosis, alpha-mannosidosis, beta-mannosidosis, aspartylglucosaminuria, Sialidosis, Inclusion-cell (I-cell) disease, Pseudo-Hurler polydystrophy, Krabbe's disease, Metachromatic leukodystrophy, multiple sulfatase deficiency, Wolmen's disease, Cholesteryl ester storage disease, Late onset GAA deficiency, Danon's disease, Neutropenia, X-linked hyper IgM syndrome, X-linked agammaglobulinemia, X-linked lymphoproliferative disease, Severe Combined Immunodeficiency, Noonan syndrome, juvenile myelomonocytic leukemia, Basal cell carcinoma, STAT1 deficiency, Alzheimer's disease, Parkinson's disease, Huntington's disease, TTR Amyloid Polyneuropathy, Ataxia Telangiectasia, Creutzfeldt-Jakob disease, Type II diabetes and Hereditary transthyretin (TTR) amyloidosis.

In the invention the diseases are Gaucher's disease, von Hippel-Lindau disease, pheochromocytoma, and paraganglioma.

As used herein, "Gaucher's disease" is a genetic disease in which a fatty substance (lipid) accumulates in cells and certain organs. Gaucher's disease includes Type 1, Type 2, and Type 3, and intermediates and subgroups thereof. Gaucher's disease is the most common of the lysosomal storage diseases. It is caused by a hereditary deficiency of the enzyme glucocerebrosidase, which acts on glucocerebroside (glucosylceramide). When the enzyme is defective, glucocerebroside accumulates in the white blood cells, spleen, liver, kidneys, lungs, brain and bone marrow. Some forms of Gaucher's disease may be treated with enzyme replacement therapy.

As used herein, a "symptom" associated with Gaucher's Disease includes any clinical or laboratory manifestation associated with Gaucher's Disease.

As used herein, "treatment of the diseases" or "treating", e.g. of Gaucher's Disease, encompasses inducing inhibition, regression, or stasis of the disorder. In an embodiment, treatment of diseases having tremors, rigidity or instability as symptoms thereof comprise reduction in the occurrence of the tremors, rigidity or instability. Examples include primary motor symptoms (resting tremor, bradykinesia, rigidity, postural instability), secondary motor symptoms (stooped posture/tendency to lean forward, dystonia, fatigue, impaired fine motor dexterity and motor coordination, impaired gross motor coordination, poverty of movement, akathisia, speech problems, loss of facial expression, micrographia, difficulty swallowing, sexual dysfunction, cramping, drooling) and nonmotor symptoms (pain, dementia/confusion, sleep disturbances, constipation, skin problems, depression, fear/anxiety, memory difficulties and slowed thinking, urinary problems, fatigue and aching, loss of energy, compulsive behavior).

As used herein, "inhibition" of disease progression or disease complication in a subject means preventing or reducing the disease progression and/or disease complication in the subject.

As used herein, "beta-glucocerebrosidase," also called acid beta-glucosidase, D-glucosyl-N-acylsphingosine glucohydrolase, or glucocerebrosidase, is an enzyme with glucosylceramidase activity that is needed to cleave, by hydrolysis, the beta-glucosidic linkage of the chemical glucocerebroside, an intermediate in glycolipid metabolism (GenBank Accession No. J03059). This is an example of a lysosomal glycosidase that can be used in this invention. This term includes recombinantly produced enzyme. This term is abbreviated as "GBA."

As used herein, "human GBA gene" is a gene encoding beta-glucocerebrosidase. The GBA gene is on chromosome 1q21 and involves 11 exons (GenBank Accession No. J03059). A homologous pseudogene for GBA is downstream of the GBA gene (GenBank Accession No. M16328).

As used herein, "human GBA protein" is the wild-type human GBA protein, unless specified otherwise. The GBA protein is 497 amino acids and is in GenBank Accession No. J03059. This term includes recombinantly produced human GBA protein.

As used herein, human GBA proteins associated with Gaucher's Disease include N370S, L444P, K198T, D409H, R496H, V394L, 84GG, and R329C.

As used herein, "von Hippel-Lindau disease" is a hereditary cancer syndrome predisposing to a variety of malignant and benign tumors of the eye, brain, spinal cord, kidney, pancreas, and adrenal glandsin. The genetic disease results in hemangioblastomas that are found in the cerebellum, spinal cord, kidney and retina. It is frequently caused by a mutation in the von Hippel-Lindau tumor suppressor gene (VHL gene).

As used herein, "von Hippel-Lindau tumor suppressor gene" or "VHL gene" is a gene that encodes the VHL protein. As used herein, "von Hippel-Lindau tumor suppressor protein" or "VHL protein" is protein encoded by the VHL gene. Mutations in the VHL gene can cause significant changes to, including misfolding of, the VHL protein and such changes are related to the pathogenesis of von Hippel-Lindau disease.

As used herein, "pheochromocytoma" is a catecholamine-producing neuroendocrine tumor. It is frequently caused by a mutation of the SDHB gene.

As used herein, "paraganglioma" is a neuroendocrine neoplasm that may develop at various body sites including the head, neck, thorax and abdomen. It is frequently caused by a mutation of the SDHB gene.

As used herein, "succinate dehydrogenase subunit B" or "SDHB" is a subunit of succinate dehydrogenase, which is a mitochondrial protein complex that is vital for mitochondrial electron transport and Krebs cycle function. The "succinate dehydrogenase subunit B gene" or "SDHB gene" is a gene that encodes the SDHB protein. As used herein, "succinate dehydrogenase subunit B protein" or "SDHB protein" is protein encoded by the SDHB gene. Mutations in the SDHB gene can cause significant changes to, including misfolding of, the SDHB protein and such changes are related to the pathogenesis of pheochromocytoma or paraganglioma.

As used herein, "alkyl" includes both branched and straight-chain saturated aliphatic hydrocarbon groups having the specified number of carbon atoms. Thus, C₁-Cₙ as in "C₁-Cₙ alkyl" is defined to include groups having 1, 2, ...., n-1 or n carbons in a linear or branched arrangement, and specifically includes methyl, ethyl, propyl, butyl, pentyl, hexyl, and so on. An embodiment can be C₁-C₁₂ alkyl. "Alkoxy" represents an alkyl group as described above attached through an oxygen bridge.

The term "alkenyl" refers to a non-aromatic hydrocarbon radical, straight or branched, containing at least 1 carbon to carbon double bond, and up to the maximum possible number of non-aromatic carbon-carbon double bonds may be present. Thus, C₂-Cₙ alkenyl is defined to include groups having 2, ...., n-1 or n carbons. For example, "C₂-C₆ alkenyl" means an alkenyl radical having 2, 3, 4, 5, or 6 carbon atoms, and at least 1 carbon-carbon double bond, and up to, for example, 3 carbon-carbon double bonds in the case of a C₆ alkenyl, respectively. Alkenyl groups include ethenyl, propenyl, butenyl and cyclohexenyl. As described above with respect to alkyl, the straight, branched or cyclic portion of the alkenyl group may contain double bonds and may be substituted if a substituted alkenyl group is indicated. An embodiment can be C₂-C₁₂ alkenyl.

The term "alkynyl" refers to a hydrocarbon radical straight or branched, containing at least 1 carbon to carbon triple bond, and up to the maximum possible number of non-aromatic carbon-carbon triple bonds may be present. Thus, C₂-Cₙ alkynyl is defined to include groups having 1, 2, ...., n-1 or n carbons. For example, "C₂-C₆ alkynyl" means an alkynyl radical having 2 or 3 carbon atoms, and 1 carbon-carbon triple bond, or having 4 or 5 carbon atoms, and up to 2 carbon-carbon triple bonds, or having 6 carbon atoms, and up to 3 carbon-carbon triple bonds. Alkynyl groups include ethynyl, propynyl and butynyl. As described above with respect to alkyl, the straight or branched portion of the alkynyl group may contain triple bonds and may be substituted if a substituted alkynyl group is indicated. An embodiment can be a C₂-Cₙalkynyl.

As used herein, "aryl" means any stable monocyclic or bicyclic carbon ring of up to 10 atoms in each ring, wherein at least one ring is aromatic. Examples of such aryl elements include phenyl, naphthyl, tetrahydro-naphthyl, indanyl, biphenyl, phenanthryl, anthryl or acenaphthyl. In cases where the aryl substituent is bicyclic and one ring is non-aromatic, it is understood that attachment is via the aromatic ring. The substituted aryls included in this invention include substitution at any suitable position with amines, substituted amines, alkylamines, hydroxys and alkylhydroxys, wherein the "alkyl" portion of the alkylamines and alkylhydroxys is a C₂-Cₙ alkyl as defined hereinabove. The substituted amines may be substituted with alkyl, alkenyl, alkynl, or aryl groups as hereinabove defined.

The alkyl, alkenyl, alkynyl, and aryl substituents may be unsubstituted or unsubstituted, unless specifically defined otherwise. For example, a (C₁-C₆) alkyl may be substituted with one or more substituents selected from OH, oxo, halogen, alkoxy, dialkylamino, or heterocyclyl, such as morpholinyl, piperidinyl, and so on.

In the compounds of the present invention, alkyl, alkenyl, and alkynyl groups can be further substituted by replacing one or more hydrogen atoms by non-hydrogen groups described herein to the extent possible. These include halo, hydroxy, mercapto, amino, carboxy, cyano and carbamoyl.

The term "substituted" as used herein means that a given structure has a substituent which can be an alkyl, alkenyl, or aryl group as defined above. The term shall be deemed to include multiple degrees of substitution by a named substitutent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results.

As used herein, a "compound" is a small molecule that does not include proteins, peptides or amino acids.

As used herein, an "isolated" compound is a compound isolated from a crude reaction mixture or from a natural source following an affirmative act of isolation. The act of isolation necessarily involves separating the compound from the other components of the mixture or natural source, with some impurities, unknown side products and residual amounts of the other components permitted to remain. Purification is an example of an affirmative act of isolation.

As used herein, "administering" an agent may be performed using any of the various methods or delivery systems well known to those skilled in the art. The administering can be performed, for example, orally, parenterally, intraperitoneally, intravenously, intraarterially, transdermally, sublingually, intramuscularly, rectally, transbuccally, intranasally, liposomally, via inhalation, vaginally, intraoccularly, via local delivery, subcutaneously, intraadiposally, intraarticularly, intrathecally, into a cerebral ventricle, intraventicularly, intratumorally, into cerebral parenchyma or intraparenchchymally.

The following delivery systems, which employ a number of routinely used pharmaceutical carriers, may be used but are only representative of the many possible systems envisioned for administering compositions in accordance with the invention.

Injectable drug delivery systems include solutions, suspensions, gels, microspheres and polymeric injectables, and can comprise excipients such as solubility-altering agents (e.g., ethanol, propylene glycol and sucrose) and polymers (e.g., polycaprylactones and PLGA's).

Other injectable drug delivery systems include solutions, suspensions, gels. Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Implantable systems include rods and discs, and can contain excipients such as PLGA and polycaprylactone.

Oral delivery systems include tablets and capsules. These can contain excipients such as binders (e.g., hydroxypropylmethylcellulose, polyvinyl pyrilodone, other cellulosic materials and starch), diluents (e.g., lactose and other sugars, starch, dicalcium phosphate and cellulosic materials), disintegrating agents (e.g., starch polymers and cellulosic materials) and lubricating agents (e.g., stearates and talc).

Transmucosal delivery systems include patches, tablets, suppositories, pessaries, gels and creams, and can contain excipients such as solubilizers and enhancers (e.g., propylene glycol, bile salts and amino acids), and other vehicles (e.g., polyethylene glycol, fatty acid esters and derivatives, and hydrophilic polymers such as hydroxypropylmethylcellulose and hyaluronic acid).

Dermal delivery systems include, for example, aqueous and nonaqueous gels, creams, multiple emulsions, microemulsions, liposomes, ointments, aqueous and nonaqueous solutions, lotions, aerosols, hydrocarbon bases and powders, and can contain excipients such as solubilizers, permeation enhancers (e.g., fatty acids, fatty acid esters, fatty alcohols and amino acids), and hydrophilic polymers (e.g., polycarbophil and polyvinylpyrolidone). In one embodiment, the pharmaceutically acceptable carrier is a liposome or a transdermal enhancer.

Solutions, suspensions and powders for reconstitutable delivery systems include vehicles such as suspending agents (e.g., gums, xanthans, cellulosics and sugars), humectants (e.g., sorbitol), solubilizers (e.g., ethanol, water, PEG and propylene glycol), surfactants (e.g., sodium lauryl sulfate, Spans, Tweens, and cetyl pyridine), preservatives and antioxidants (e.g., parabens, vitamins E and C, and ascorbic acid), anti-caking agents, coating agents, and chelating agents (e.g., EDTA).

As used herein, "pharmaceutically acceptable carrier" refers to a carrier or excipient that is suitable for use with humans and/or animals without undue adverse side effects (such as toxicity, irritation, and allergic response) commensurate with a reasonable benefit/risk ratio. It can be a pharmaceutically acceptable solvent, suspending agent or vehicle, for delivering the instant compounds to the subject.

The compounds for use according to the present invention may be in a salt form. As used herein, a "salt" is a salt of the instant compounds which has been modified by making acid or base salts of the compounds. In the case of compounds used to treat an infection or disease, the salt is pharmaceutically acceptable. Examples of pharmaceutically acceptable salts include mineral or organic acid salts of basic residues such as amines; alkali or organic salts of acidic residues such as phenols. The salts can be made using an organic or inorganic acid. Such acid salts are chlorides, bromides, sulfates, nitrates, phosphates, sulfonates, formates, tartrates, maleates, malates, citrates, benzoates, salicylates, and ascorbates. Phenolate salts are the alkaline earth metal salts, sodium, potassium or lithium. The term "pharmaceutically acceptable salt" in this respect, refers to the relatively non-toxic, inorganic and organic acid or base addition salts of compounds of the present invention. These salts can be prepared in situ during the final isolation and purification of the compounds of the invention, or by separately reacting a purified compound of the invention in its free base or free acid form with a suitable organic or inorganic acid or base, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts. (See, e.g., Berge et al. (1977) "Pharmaceutical Salts", J. Pharm. Sci. 66: 1-19).

As used herein, an "amount" or "dose" of an agent measured in milligrams refers to the milligrams of agent present in a drug product, regardless of the form of the drug product.

As used herein, the term "therapeutically effective amount" or "effective amount" refers to the quantity of a component that is sufficient to yield a desired therapeutic response without undue adverse side effects (such as toxicity, irritation, or allergic response) commensurate with a reasonable benefit/risk ratio when used in the manner of this invention. The specific effective amount will vary with such factors as the particular condition being treated, the physical condition of the patient, the type of mammal being treated, the duration of the treatment, the nature of concurrent therapy (if any), and the specific formulations employed and the structure of the compounds or its derivatives.

Where a range is given in the specification it is understood that the range includes all integers and 0.1 units within that range, and any sub-range thereof. For example, a range of 77 to 90% is a disclosure of 77, 78, 79, 80, and 81%.

As used herein, "about" with regard to a stated number encompasses a range of +one percent to -one percent of the stated value. By way of example, about 100 mg/kg therefore includes 99, 99.1, 99.2, 99.3, 99.4, 99.5, 99.6, 99.7, 99.8, 99.9, 100, 100.1, 100.2, 100.3, 100.4, 100.5, 100.6, 100.7, 100.8, 100.9 and 101 mg/kg. Accordingly, about 100 mg/kg includes, in an embodiment, 100 mg/kg. It is understood that where a parameter range is provided, all integers within that range, and tenths thereof, are also provided by the invention. For example, "0.2-5 mg/kg/day" is a disclosure of 0.2 mg/kg/day, 0.3 mg/kg/day, 0.4 mg/kg/day, 0.5 mg/kg/day, 0.6 mg/kg/day up to 5.0 mg/kg/day.

This invention will be better understood by reference to the Experimental Details which follow, but those skilled in the art will readily appreciate that the specific experiments detailed are only illustrative of the invention as described more fully in the claims which follow thereafter.

### Experimental Details

### Example 1. Gaucher's Disease

### Methods

### Cell Culture and HDACi Treatment.

Fibroblasts from GD patients were maintained as previously described (Lu et al., 2010). Cell lines were obtained from the Development and Metabolic Neurology Branch of the National Institute of Neurological Disorders and Stroke (NINDS). Type 1 GD fibroblast is homozygous for N370S mutation. Type II and III GD fibroblasts are homozygous for L444P mutation. Cells were cultured in Eagle's minimum essential medium (Invitrogen; Carlsbad, CA) supplemented with 10% FBS. A total of 2.5x 10⁴cells were seeded in 24 well plates, allowed to attach for at least 24 h, and then treated with SAHA and LB-205 at concentrations of 2.5 µM and 5.0 µM for 24 h. LB-205 was provided by Lixte Biotechnology Holdings ("LBHI"; East Setauket, New York) under a Cooperative Research and Development Agreement between the National Institute of Neurologic Disorders and Stroke (NINDS), National Institutes of Health (NIH), and LBHI.

### HDAC Activity Assays

Cultured DAOY cells, a medulloblastoma cell line, were plated in 175-cm³ flasks. When the cells were 80% confluent, the media was replaced with media containing retinoic acid (RA) (5.0 µm), Trichostatin A (1µm), SAHA (2.0 µm), LB-205 (5.0 µm), LB-205 (2.5µm), or PBS control. After 1 h, the cells were washed 3 times with a 0.9% normal saline solution. Cells were lysed in Tissue protein extraction reagent (T-PER) (Pierce Biotechnology; Rockford, IL), sonicated, and centrifuged. Nuclear cellular extracts were assayed from 0 to 50 µg per well using HDAC Assay Kit (Active Motif; Carlsbad, CA).

HDAC activity in DAOY subcutaneous xenografts were assayed in 6-8 week old SCID mice (Taconic) with the same conditions as above. Each mouse weighed approximately 20 g. Animals were housed under barrier conditions and maintained on a 12-h light/12-h dark cycle with adequate food and water supplies. After being observed for 7 days, mice were injected subcutaneously in both flanks with 5 × 10⁶ DAOY tumor cells suspended in PBS. After the xenografts reached 0.5 ± 0.1 cm (day zero), animals were randomized to 4 groups of 5 animals each. Animals received intraperitoneal injections of PBS vehicle, LB-205 at 0.5 µM, or 0.5µM of SAHA. Tumor tissues were harvested after 2, 4, 8, 12, and 24 h and washed three times with a 0.9% normal saline solution for HDAC Activity Assays. All animal experiments were approved for use and care of animals under the guidelines of NIH ACUC animal protocol.

### GBA Enzyme Activity Assay

Fluorometric GBA enzyme activity assay was conducted as previously described (Lee et al., 2005). Samples were loaded into a 96-well plate, and fluorescence was measured with a VICTOR3 multilabel counter (Perkin Elmer; Waltham, MA) at an excitation/emission setting of 355 nm/460 nm. GBA activity was designated that 1 nmol of 4-methylumbelliferone release per hour as one unit.

### Western Blotting

Cell pellets were lysed in T-PER Tissue Protein Extraction Reagent solution (T-PER) (Thermo; Waltham, MA), sonicated, and centrifuged. Protein was determined in the supernatant solution by using the Bio-Rad Protein Assay kit (Bio-Rad; Hercules, CA). Proteins were separated by SDS-PAGE on 4-15% acrylamide gels (Invitrogen; Carlesbad, CA) and transferred to nitrocellulose membranes (Invitrogen; Carlesbad, CA). Blocking buffer solution was used before immunoblotting with primary antibody. Expression of GBA was determined by Western blotting using monoclonal antibody at a dilution of 1:1,000. Acetylated histone 3 rabbit antibody (Cell Signaling Technology; Beverley, MA) was used to detect HDACi function. A goat antibody directed against actin (sc-1616; Santa Cruz Biotechnology; Santa Cruz, CA) at a dilution of 1:1,000 was used as a loading control. Detection of antibodies was performed with a horseradish peroxidase-conjugated species-specific secondary antibody and an enhanced chemiluminescence system. Densitometric analysis using image software (NIH ImageJ software; Bethesda, MD) was used to quantify the expression of GBA.

### Immunoprecipitation

Immunoprecipitation was performed using DynaBeads Protein G immunoprecipitation kit (Invitrogen; Carlesbad, CA). Cells were harvested and extracted for protein using IP lysis buffer (Thermo; Waltham, MA). Two hundred microgram of whole cell protein was precipitated in 4°C with monoclonal antibody against Flag tag (1:200, Origene; Rockville, MD). Precipitated protein was eluted and analyzed through Western blotting.

### DNA Cloning and Site-directed Mutagenesis

Human *GBA* gene was cloned into pCMV6-Entry vector (Origene; Rockville, MD). Mutagenesis was performed using a QuikChange Lightning Site-directed mutagenesis kit (Agilent; Santa Clara, CA). Mutagenesis reaction was conducted using primers with N370S and L444P mutation sites. For each mutant, the sequence of *GBA* genes was verified by sequencing the entire coding regions.

### Metabolic [³⁵S] Labeling and Pulse Chase Assay

Metabolic [³⁵S] pulse chase assay was performed as previously described with minor modifications (16). A total of 5 x 10⁵ HeLa cells were transfected with *GBA* vectors through FuGene 6 transfection reagent (Roche; Indianapolis, IN). Cells were used for pulse chase assay 24 hr after transfection. Cells were starved in methionine-depleted medium for 15 min followed by 15 min labeling in methionine-free Delbecco's modified Eagle medium (DMEM) supplemented with 10% FBS and 0.2 mCi/mL [³⁵S]-methionine (>1,000 Ci/mL specific activity, Perkin Elmer). The cells were then chased in DMEM with 10% FBS and 3 mg/mL methionine. Cells were extracted for protein within various durations after radioactive labeling. Cells were lysed in RIPA lysis buffer containing with Halt proteinase inhibitor cocktail (Thermo; Waltham, MA). GBA proteins were purified through immunoprecipitation using 200 ug total lysate with Flag antibody (Origene; Rockville, MD). Precipitated proteins were resolved on NuPAGE Novex 4-12% Bis-Tris Gel (Invitrogen; Carlesbad, CA) and measured by liquid scintillation counting. The gel was fixed and incubated in EN3HANCE (Perkin Elmer; Waltham, MA) for 45 min and dried for X-ray film exposure.

### Results

### LB-205 inhibits HDAC activity and exhibits a longer half-life than SAHA in vivo

LB-205 contains a metal-binding functional group to inhibit Zn²⁺ dependent class I and class II HDACs (Figure 4A). To compare pharmacodynamic and pharmacokinetic properties of LB-205 to SAHA, HDAC activity assay was performed for DAOY cell lines and DAOY xenografts tumors. LB-205 demonstrated dose-dependent HDAC inhibition and a longer half-life of effectiveness than SAHA (Figure 4B,C). To determine the biological half-life of LB-205, we measured HDAC activity in subcutaneous xenografts of DAOY from SCID mice treated with intraperitoneal injections of SAHA and LB-205 after 2, 4, 8, and 12h (Figure 4C). HDACi function of SAHA decreases after 4-8 hours, while that of LB-205 is sustained for up to 12 hours. Western blotting for Ac-H3 demonstrated a longer effectiveness of LB-205 in vitro (Figure 4D). LB-205 and SAHA also inhibited HDACs in GD type 1 fibroblasts. A non-HDACi small molecule was used as a non-targeted vehicle control (Figure 4E).

### HDAC inhibitors increase functional GBA quantity in GD patient-derived fibroblasts

To test whether HDAC inhibitors ameliorate the decreased cellular levels of GBA observed in fibroblasts harboring N370S and L444P mutations, GBA protein levels were observed in patient-derived type I and type II/III GD fibroblasts treated with SAHA and LB-205. Western blotting demonstrated increased GBA levels from 38.1% up to 59.6%-80.5% in Type I and from 16% up to 35%-40% in type I and type II/III fibroblasts treated with LB-205 and SAHA for 24 hours compared to untreated fibroblasts (Figure 4A, 4B). HDACi treatment also increased catalytic activity of both N370S and L444P GBA, suggesting that quantitative increases in GBA correlated with increases in cellular enzyme activity (Figure 4D).

### Stability loss of GBA mutant protein

The findings of Lu et al. (2010) indicate that loss of GBA activity in GD is caused by quantitative loss of GBA protein, and not by a change in its intrinsic enzymatic functions. They hypothesized that quantitative loss of GBA protein is due to a change in protein stability and rapid degradation after protein translation. To test this, the stability changes of GBA protein with pathogenic mutations in type I and type III GD were investigated. *GBA* hot spot mutations (Δ*GBA*-N370S, A*GBA*-L444P) were cloned into pCMV6-Entry vector and transfected into HeLa cells. Quantitative changes of GBA protein were monitored through a [³⁵S]-methionine mediated pulse chase assay.

Autoradiography showed successful protein synthesis in pathogenic mutations. There were no differences in radioactively labeled GBA protein between wild type and mutants, suggesting identical gene transcription/translation efficiency. Consistent with our hypothesis, a quantitative loss of [³⁵S] labeled GBA protein in Δ*GBA*-N370S and Δ*GBA*-L444P mutations was observed across time (Figure 6A). This finding is consistent with previous studies that demonstrated nascent GBA mutant protein is retained in the endoplasmic reticulum (ER) and efficiently degraded within 24 hr (Schmitz et al. (2005); Bergmann and Grabowski (1989)). [³⁵S]-GBA protein was quantified through liquid scintillation on X-ray film (Figure 6B). Protein quantity changes across time were regressed through an exponential decay regression. After 24 hr chase, 88% wild type GBA protein remained in cell, whereas N370S and L444P mutant protein were reduced to 39% and 15%, respectively.

### Abnormal chaperonin binding to GBA protein

The rapid degradation immediately after protein translation strongly suggested dysfunction in protein folding and post-translational modifications in GBA mutants. To further elucidate the degradation pathways of N370S and L444P mutants, chaperonin binding to GBA was assessed using co-immunoprecipitation. We found decreased binding of Hsp70 and TCP1 to GBA mutants, suggesting the mutant proteins were less likely to be recognized by chaperonins and may be unable to form mature GBA molecules. In contrast, GBA was found to have elevated Hsp90 binding and protein ubiquitination in both N370S and L444P mutants, suggesting that the mutant protein was trapped by the Hsp90 system and directed towards the ubiquitin-proteasome mediated degradation system (Figure 6C).

### Increased GBA stability after treatment with proteostasis regulators

Mutant GBA proteins largely retain their intrinsic enzyme activity and the deactivation of GBA in Gaucher disease is due to rapid degradation. Modulation of proteostasis regulators that affect pre-functional degradation may be of great importance to restore the number of functional GBA molecules. In the present study, HeLa cells with Δ*GBA*-N370S or Δ*GBA*-L444P mutant vector were treated with HDACi, and investigated protein residue through a pulse chase assay. Among these compounds, celastrol, SAHA, and LB-205 increased protein stability in the N370S mutant (Figures 7A and 7B). Radioactively labeled protein quantity increased by 33%, 12% and 27%, respectively. For the L444P mutant, [³⁵S]-GBA residue was found increased by 104%, 101% and 104% by the same treatments.

To understand the molecular changes of GBA degradation during HDACi treatment, protein ubiquitination and Hsp90, Hsp70, and TCP1 binding was observed through co-immunoprecipitation of GBA (Figure 7C, 7D). Ubiquitination of mutant GBA protein and binding to Hsp90 were elevated in untreated cells, which was consistent with the greatly shortened half-life of the mutant protein. However, treatment with LB-205 and SAHA decreased GBA ubiquitination without changing the level of TCP1 binding. This indicates that a decrease in protein degradation through ubiquitin-proteasome pathway mediates the increase in GBA after HDACi, rather than an up-regulation of the chaperones involved in the unfolded protein response.

### Example 2. Gaucher's Disease

### Methods

Human cells from patients diagnosed with Gaucher's disease type 3 were treated with LB100 (comparative example), SAHA (comparative example) and LB2 (LB205) for 24 hours, and GBA levels were analyzed.

The structure of LB100 is:

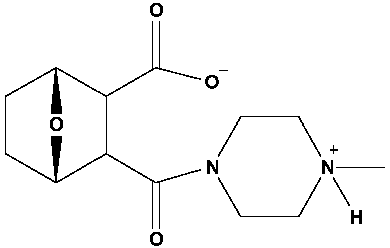

The structure of SAHA is:

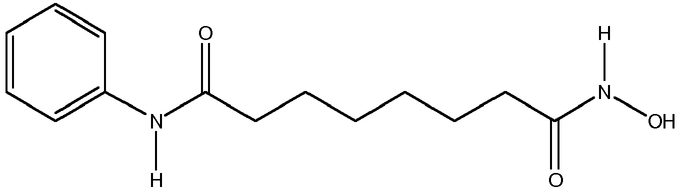

The structure of LB2 (LB205) is:

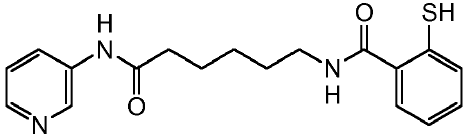

### Results

Human cells isolated from patients with Gaucher's disease type 3 were treated with LB100 (comparative example), SAHA (comparative example), or LB2 and GBA levels were quantified and compared. In all three treatments, GBA levels were significantly higher than in untreated cells. Treatment with a protein phosphatase 2A inhibitor, LB100, had similar results to treatment with histone deacetylase inhibitors, SAHA or LB2. The half-life of GBA was significantly increased as a result of treating Gaucher's disease type 3 cells with these compounds.

### Example 3. Gaucher's Disease

### Methods

Human cells from patients diagnosed with Gaucher's disease type 1 or Gaucher's disease type 3 were treated with LB100 (comparative example), SAHA (comparative example) and LB2 (LB205) for 24 hours, and GBA levels were analyzed.

The structure of LB100 is:

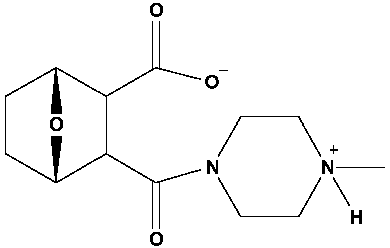

The structure of SAHA is:

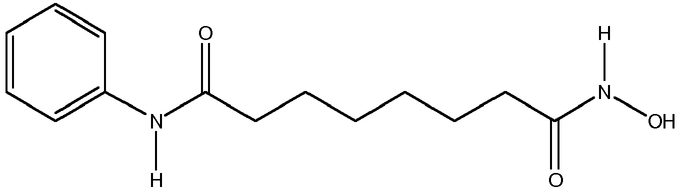

The structure of LB2 (LB205) is:

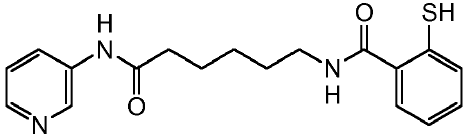

### Results

Human cells isolated from patients with Gaucher's disease type 1 or type 3 were treated with LB100 (comparative example), SAHA (comparative example), or LB2 and GBA levels were quantified and compared. In all three treatments, GBA levels were significantly higher than in untreated cells. Treatment with a protein phosphatase 2A inhibitor, LB100, had similar results to treatment with histone deacetylase inhibitors, SAHA or LB2. The half-life of GBA was significantly increased as a result of treating Gaucher's disease type 1 or Gaucher's disease type 3 cells with these compounds.

### Example 4. Gaucher's Disease

### Methods

Human cells from patients diagnosed with Gaucher's disease type 1 or Gaucher's disease type 3 were treated with a combination of LB100 (comparative example), SAHA (comparative example) and LB2 (LB205) for 24 hours, and GBA levels were analyzed.

The structure of LB100 is:

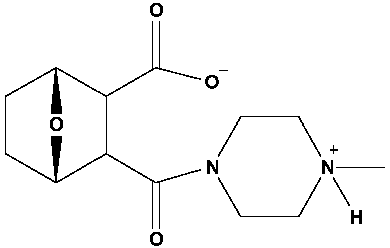

The structure of SAHA is:

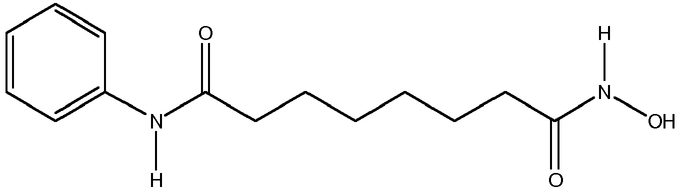

The structure of LB2 (LB205) is:

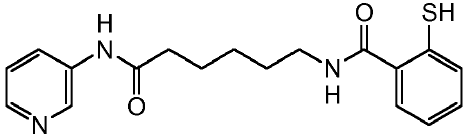

### Results

Human cells isolated from patients with Gaucher's disease type 1 or type 3 were treated with a combination of LB100 (comparative example), SAHA (comparative example), or LB2 and GBA levels were quantified and compared. In the treated cells, GBA levels were significantly higher than in untreated cells. Treatment combining a protein phosphatase 2A inhibitor, LB100, with a histone deacetylase inhibitor, SAHA or LB2, resulted in higher GBA levels than in untreated cells. The half-life of GBA was significantly increased as a result of treating Gaucher's disease type 1 or Gaucher's disease type 3 cells with these compounds.

### Example 5. Von-Hippel-Lindau Disease

### Methods

### VHL Tumor Samples and Tissue Dissection

All tissue was collected at the Surgical Neurology Branch at National Institute of Neurological Disorders and Stroke (NINDS). Tissue samples and clinical information was obtained as part of an Institute Review Board-approved study. Frozen tissue samples included 13 hemangioblastomas and 1 non-VHL tumor samples. Tissue dissection was performed as previously described.

### Western blot

Microdissected tissue and cell pellets were lysed in RIPA lysis buffer (Thermo), sonicated and centrifuged. The quantity of protein was determined in the supernatant solution using a Bio-Rad Protein Assay Kit. Proteins were separated by NuPAGE 4-12% Bis-Tris gel (Invitrogen) and transferred to PVDF membranes (Invitrogen). Membranes were blocked in 5% skim dried milk and immunoblotted with primary antibody. Antibodies used for this study: Flag (1:2000, Origene), HA (1:1000, Origene), Ubiquitin (1:1000, Cell Signaling), Hsp70 (1:1000, Sigma), Hsp90 (1:1000, Cell Signaling), TCP1 (1:1000, Sigma), β-Actin (1:2000, Sigma).

### Immunoprecipation

200 µg of whole-cell lysates were precipitated using DynaBeads Protein G immunopreciptation kit (Invitrogen) with monoclonal antibodies against Flag or HA (1:200, Origene). The beads were washed extensively and eluted with glycine. Precipitated protein was analyzed by western blot.

### Immunofluorescence Analysis

Frozen sections from tumor specimen were fixed in Histochoice and labeled overnight with primary antibodies against VHL (1:50, Cell Signaling Technology). Tumor vessels were labeled with anti-CD31 antibody (1:200, Millipore). Cell nuclei were counterstained with Hoechst 33342 (Invitrogen). The specimens were visualized using a Zeiss LSM 510 confocal microscope.

### Real-time PCR

Tumor samples were dissected and RNA extraction was performed using the PureLink RNA Mini kit (Invitrogen) . Total RNA was reverse-transcribed with the SuperScript III First-Strand Synthesis System for RT-PCR (Invitrogen) for cDNA. VHL, EPO, EDN1 and GLUT1 gene expression was determined by PCR using gene-specific primers (Invitrogen) .

### Cell Culture and Transfection

VHL deficient cell line 786-0 were maintained in Dulbecco's Modified Eagle Media (DMEM, Invitrogen) containing 10% FBS, 100 U/mL penicillin and 100 µg/mL streptomycin. Cells were transfected with VHL or HIF1/2 vectors by using Xtremegene 9 transfection reagent (Roche). For stable transfection, cells were incubated in growth medium supplemented with 0.5 mg/mL G-418 for 10 days followed by serial dilution. Monoclonal cells were collected and gene expression was tested through western blot.

### DNA Cloning and Site-Directed Mutagenesis

Mutagenesis was performed using QuikChange Lightning Site-Directed Mutagenesis Kit (Agilent). Missense mutations were selected based on frequent mutations in the VHL syndrome. Mutations in the VHL gene were generated in pCMV6-Entry vectors with the full-length wild type VHL gene (Origene). For each mutant, the sequence of mutant VHL genes was verified with DNA sequencing of the entire coding region.

### Metabolic ³⁵S Labeling and Pulse Chase Assay

A total of 7 × 105 786-0 cells were transfected with VHL vectors 12 hours before labeling. Radioisotopic protein labeling with radioisotopes was performed by methionine starvation for 15 minutes followed by growth in methionine-free DMEM supplemented with 0.2 mCi/mL [³⁵S]-methionine (>1,000 Ci/mmol specific activity, Perkin Elmer). The cells were then chased in DMEM with 10% FBS and 3 mg/mL methionine. Cells were lysed with RIPA lysis buffer containing a proteinase inhibitor cocktail (Thermo). VHL proteins were immunoprecipitated using 200 µg from whole cell lysates with monoclonal Flag antibody (Origene). Precipitated proteins were fractionated with NuPAGE Novex 4-12% Bis-Tris Gel (Invitrogen) and measured by liquid scintillation counting. The gel was fixed and incubated in EN3HANCE (Perkin Elmer) for 30 minutes and dried for subsequent X-ray film exposure.

### Results

### Quantitative loss of von Hippel-Lindau tumor suppressor protein in VHL derived tumors

To measure the levels of VHL protein expression in tumors, VHL expression in frozen sections from hemangioblastoma (HB, n=5) and clear cell renal cell carcinoma (ccRCC, n=3) was examined through immunofluorescence staining (Figure 8A). CD31 staining was applied to confirm the typical vascular structure in VHL derived tumors. One non-VHL tumor specimen was used as positive control. VHL protein expression was found to be absent in all VHL derived tumor specimen. VHL and CD31 protein expression were further exmined in VHL derived tumors based on immunostaining (Figure 8B). There was a 65.4-96.2% decrease in VHL protein expression in VHL associated tumors, whereas CD31 expression increased by 90.0%-890%. This is in accordance with previous findings that enhanced vasculargenesis occurs with the deactivation of VHL tumor suppressor. Consistent with immunofluorescence, western blot analysis in VHL-associated tumor specimens demonstrated similar absence of VHL protein expression, with normal VHL expression in corresponding normal tissue from VHL patient (Figure 8C).

### Expression of VHL mRNA in tumors

Germline mutations in the coding region of von Hippel-Lindau tumor suppressor could affect associated VHL protein expression through either abnormal mRNA and/or protein expression. Therefore, mRNA transcript levels were measured for VHL in microdissected HB (n=5) and ccRCC (n=3) specimens with VHL missense mutations. Two independent normal tissue specimens were used for comparison. Quantitative real-time PCR analysis revealed that VHL mRNA is expressed at a similar level in VHL-associated tumors compared with normal tissue. Hypoxia related gene expression was further tested to confirm the identity of tumor specimen tested. Up-regulation of EPO (increased by 131.3-643.4%), EDN1 (increased by 199.6-1126%) and GLUT1 (increased by 206.3-2255%) mRNA expression in all samples from VHL associated tumors were identified.

### Protein stability change of VHL tumor suppressor protein in tumor-derived mutants

Functional loss of von Hippel-Lindau tumor suppressor protein appeared to occur at the protein level. It was hypothesized that changes in protein stability underlies the net loss of function seen in VHL-associated tumors, which contribute to the pathogenesis of VHL derived tumors. To test this hypothesis, the stability of mutant and wild type VHL was investigated. Missense mutations are the most frequent genetic changes in VHL-associated tumors, which contributes to 30-38% of all VHL cases and ∼90% of VHL Type 2. In this study, hotspot missense mutations (S68W, Y112N and A149S) were selected from patients with VHL and these coding sequences were inserted into pCMV6-Entry vectors. After transfection of these vectors into VHL deficient cell line 786-0, we investigated protein stability through cycloheximide mediated pulse chase assay (Figure 9A). VHL mutants were successfully synthesized in full length in all cases. And there were no significant difference in protein expression at the beginning of the assay.

Cycloheximide treatment demonstrated significant loss of stability in tumor-derived mutant S68W and Y112N. Quantitative analysis on VHL protein residue shows that wild type VHL protein is rapidly turnover in physiologic condition and the half life of protein is 3.84 hr. In tumor-derived mutants, however, protein half lives were reduced to 1.21-1.87 hr (Figure 9B). The changes of VHL stability in mutants appear to be related to immediate degradation of VHL protein after synthesis as we shown in [³⁵S]-methionine mediated pulse chase assay (Figure 9C). Autoradiography showed that tumorigenic mutant ΔVHL-A149S is synthesized in full length at the beginning of the assay. Newly synthesized ΔVHL-A149S protein exhibit shortened half life as compared with wild type VHL. Protein half-life were calculated through liquid scintillation on [³⁵S] radioactivity and found a fundamental loss of protein stability in mutant VHL protein (Figure 9D).

### Mutant VHL protein maintains intrinsic functional capacity

Functional deactivation of von Hippel-Lindau tumor suppressor could results in the net loss of VHL protein product, or catastrophe in protein intrinsic function. To determine whether tumor derived VHL mutants exhibit impaired E3 ligase activity, HIFla degradation through re-introducing VHL protein was studied. Wild type and mutant VHL protein were expressed in 786-0 cells with HIFla stable expression (HA-HIF1α-786-O). HIF1α is stabilized in 786-0 cell due to lack of effective E3 ligase conjugates. HIF1α is efficiently degraded upon either wild type or mutant VHL transfection. VHL mutants exhibit similar E3 ligase activity. This is confirmed by immunoprecipitation assay showing HIF1α ubiquitination in associated with wild type or mutant VHL proteins. The Ex vivo peptide binding assay demonstrated that VHL mutant protein exhibit similar affinity to HIF derived peptide. Through measuring luciferase activity change driven by hypoxia regulated element promoter, intact VHL function in mutant was confirmed. Upon co-transfection VHL and HRE-luciferase plasmid into HA-HIF1α-786-O cells, HRE transcription activity was found reduced by 91.3% with wild type VHL, whereas tumorigenic VHL mutants transfection reduced HRE transcription activity by 81.4-53.6%. The changes in transcription activity due to VHL mutant reintroduction were confirmed by measuring hypoxia related gene expression through quantitative real-time PCR assay. The expression EDN1, EPO and GLUT1 mRNA was found reduced upon wild type or mutant VHL expression.

### Increased VHL expression after treatment with HDAC inhibitors

The co-translational degradation of VHL mutant in nascent peptide stage is largely dependent on the quality control system, which includes protein folding machinery and varies chaperon/chaperonin. In the present study the goal was to to elucidate the critical molecules that precede VHL mutant protein into degradation. Immunoprecipitation assays were performed to detect chaperonin binding to different mutant VHL proteins (Figure 10A and 10B). Robust Hsp70 and TCP1 binding to wild type VHL protein (wtVHL) was identified, but with less Hsp90 binding. Tumorigenic VHL mutant protein (ΔVHL-S68W, ΔVHL-Y112N and ΔVHL-A149S) exhibits increased binding of Hsp90 and less likely binding to TCP1. This is consistent with previous finding that VHL mutant protein exhibit abnormalities in chaperonin binding and probably contributes to their rapid degradation (REF). The involvement of Hsp70/90 seems to be critical for VHL mutant protein degradation, since siRNA silencing on Hsp70/90 organizing protein STIP1 elongated ΔVHL-Y112N protein half life (Figure 10C). This is similar to the finding that VHL mutant protein is not degraded in STP1, a homologous gene with STIP1, deficient yeast strains (REF). The essential role of chaperonine system in VHL mutant protein degradation indicates a potential therapeutic strategy that counteracts the chaperonine system. It was previously found that LB201 and LB205, two novel HDACi, protect missense glucocerebrosidase from co-translational degradation through affecting chaperonine binding (REF).

HDACi as a potential therapeutic strategy could reduce mutant VHL protein degradation and minimize the HIF1α related gene expression and tumorigenesis. Hsp90 affinity to VHL mutants decreased with LB205 or SAHA treatment (Figure 10D). Several HDAC inhibitors and proteostasis regulators were screened on their effect on VHL protein half life (Figure 10E). Consistent with the result above, VHL mutant protein is rapidly degraded in physiologic condition. HDAC inhibitors LB205 and SAHA (comparative example) stabilized the protein within the same assay. Celastrol, as a proteostasis modulator, also decreased the degradation of VHL mutant protein. Quantification of protein half lives reveals that with LB205 and SAHA (comparative example) treatment, the half life of VHL mutant protein increased into 2.46 hr and 10.22 hr (Figure 10F).

### Example 6. Von-Hippel-Lindau Disease

### Methods

Tumors cells from VHL derived tumors were treated with SAHA (comparative example), LB201 or LB2 (LB205), and VHL protein levels were analyzed.

The structure of SAHA is:

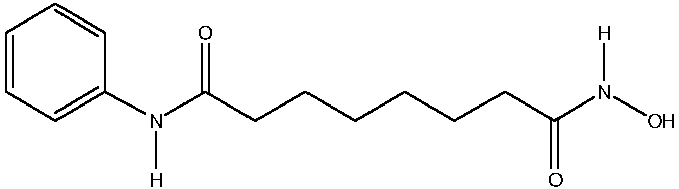

The structure of LB2 (LB205) is:

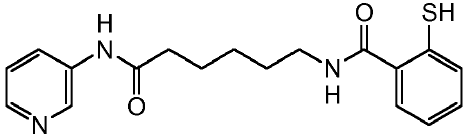

The Structure of LB201 is:

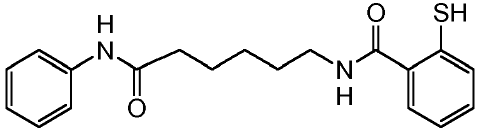

### Results

Tumors cells were treated with SAHA (comparative example), LB201 or LB205 and VHL levels are quantified and compared. In the treated cells, VHL levels were significantly higher than in untreated cells. Treatment with a histone deacetylase inhibitor, SAHA (comparative example), LB201 or LB205, resulted in higher VHL levels and decreased degradation of VHL than in untreated cells. The half-life of VHL was significantly increased as a result of treating the VHL protein with these compounds.

### Example 7. Von-Hippel-Lindau Disease

### Methods

Tumors cells from VHL derived tumors are treated with a combination of LB100, SAHA, LB201 or LB2 (LB205), and VHL protein levels are analyzed.

The structure of SAHA is:

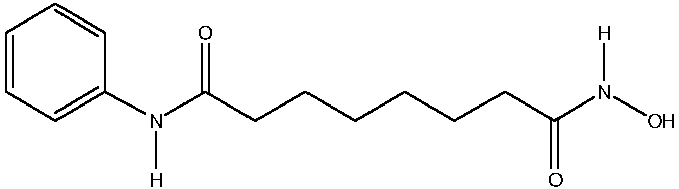

The structure of LB2 (LB205) is:

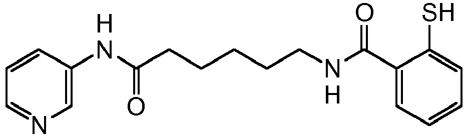

The Structure of LB201 is:

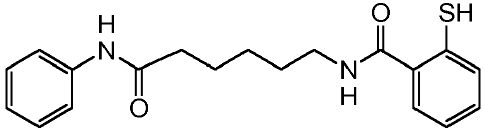

### Results

Tumors cells are treated with a combination of SAHA, LB201 or LB205. VHL levels are quantified and compared. In the treated cells, VHL levels are significantly higher than in untreated cells. Treatment with a combination of SAHA, LB201 or LB205, results in higher VHL levels and decreased degradation of VHL than in untreated cells. The half-life of VHL ik significantly increased as a result of treating the VHL protein with these compounds.

### Example 8. Pheochromocytoma and Paraganglioma

### Methods

### SDHB tumor samples and tissue dissection

Tumor tissue samples and normal human adrenal glands were obtained under studies approved by the appropriate Institutional Review Board, with informed consent obtained from all patients. Extraneous tissue was removed, dimensions of tumors were recorded, samples were dissected away from surrounding tissue, then divided into smaller 10 to 50 mg pieces, frozen on dry ice or Optimal Cutting Temperature (OCT, Sakura Finetek, Torrance, CA) blocks and stored at -80°C. Other samples of tissues were formalin-fixed for immunohistochemistry. Seven PHEOs/PGLs and 5 normal adrenal tissue specimens were used in the present study. Isolation of normal adrenal medulla was performed as described by our group previously (Fliedner et al. (2010)). Tissue microdissection was performed as previously described (Furuta et al. (2004); Zhang et al. (1995)).

### Reverse transcription and real-time PCR

RNA was extracted from frozen tissue samples of PHEOs/PGLs with homogenization in TRIzol reagent (Invitrogen, Carlsbad, CA) followed by RNeasy Maxi (Qiagen, Valencia, CA) according to the manufacturer's recommendations. One microgram of total RNA was reversibly transcribed to cDNA using random hexamers. Quantitative PCR (TaqMan PCR), using a 7000 Sequence Detector (Applied Biosystems, Foster City, CA), was used for quantification of mRNA for SDHB. The primers and TaqMan probe pre-made set were ordered through Applied Biosystems (Applied Biosystems, Foster City, CA). 18S ribosomal RNA was used as a housekeeping gene. Reaction tubes contained 25 ng cDNA product as template, 1x TaqMan Universal PCR Master Mix, 1x of pre-made sets of primers and TaqMan probes for SDHB gene or 18S ribosomal RNA, adjusted to a final volume of 50 µl with H20. PCR involved 45 cycles at the following temperature parameters: 15 seconds at 95°C, 1 min at 60°C. SDHB gene mRNA expression was evaluated through DDCT method.

### Western blot

Western blot analysis was performed as previously described with minor modifications (Zhuang et al, 2011). Microdissected tumor tissue and cell pellets were extracted for protein using RIPA lysis buffer supplemented with Halt proteinase inhibitor cocktail (Thermo Scientific, Rockford, IL). Protein was vortexed at 4°C for 20 min and centrifuged in 12,000 g for 10 min at 4°C. Supernatant was collected and protein quantity was identified through a Bio-Rad (Hercules, CA) Protein Assay kit. Equal amount of proteins were separated on NuPAGE 4-12% Bis-Tris gel (Invitrogen, Carlsbad, CA) and transferred to PVDF membranes (Invitrogen). Membranes were blocked in 5% skim dried milk in PBST and blotted with primary antibody. Protein expression was identified through chemiluminescence kit (Thermo Scientific). The following antibodies were used: SDHA (1:1,000, Cell Signaling Technology, Beverly, MA), SDHB (1:1,000, Sigma- Aldrich, St. Louis MO), Flag (1:2,000, Origene, Rockville, MD), Ubiquitin (1:1,000, Abcam, Cambridge, MA), HA (1:2,000, Origene), Hsp90 (1:1,000, Cell Signaling Technology) and β-Actin (1:1,000, Santa Cruz Biotechnology, Inc., Santa Cruz, CA) .

### Immunoprecipitation

Immunoprecipitation was performed as previously described (Lu et al., 2010). Protein was extracted from cell cultures using IP lysis buffer with Halt proteinase inhibitor cocktail (Thermo Scientific). Four hundred microgram of total protein was precipitated with Flag antibody (1:200, Origene) using a DynaBeads Protein G immunoprecipitation kit (Invitrogen). Proteins were precipitated overnight at 4°C and eluted for Western blot analysis.

### Immunofluorescence

Cells were preloaded with Mitotracker Red for 20 min before fixation. Cells were then washed three times in PBS and fixed in Histochoice for 15 min. SDHB mutants were labeled with anti-Flag antibody (1:200, Origene). Cell nuclei were counterstained with Hoechst 33342 (Invitrogen). The specimens were visualized using a Zeiss (Thornwood, NY) LSM 510 confocal microscope.

### Immunohistochemistry staining

Immunohistochemistry staining was performed using commercially available SDHB antibody (Sigma-Aldrich) on formalin-fixed paraffin embedded tissue mounted on positively charged slides. The primary antibody was used at a dilution of 1:500 after heat-induced antigen retrieval using 1 mM EDTA. Samples were then labeled and visualized using a DAB staining kit (Envision+Kit from DAKO, Carpinteria, CA).

### Cell culture and transfection

HeLa cells were maintained in DMEM containing 10% FBS (Invitrogen). Cells were transfected with SDHB vectors by FuGENE 6 transfection reagent (Roche, Indianapolis, IN). Medium was changed 4 hours after transfection and cells were maintained 48 hours before cycloheximide (20 µg/mL, Sigma- Aldrich) treatment.

### DNA cloning and site-directed mutagenesis

Ubiquitin-HA vector was described previously (Kamitani et al. (1997)). Human wild type SDHB gene was cloned into pCMV6-Entry vector (Origene). Hot spot missense mutations in SDHB related PHEOs/PGLs based on previous findings (Van Nederveen et al. (2006); Henderson et al. (2009); Pawlu et al. (2005)). Mutagenesis was performed based on wild type SDHB vector using a Quikchange Lightning Site-Directed Mutagenesis Kit (Agilent, Santa Clara, CA). For each mutant, the sequence of SDHB gene was verified through sequencing the entire coding region of the gene. Vectors were purified through PureLink HiPure Plasmid Maxiprep kit (Invitrogen) for transfection and cell free protein expression.

### Metabolic [³⁵S] pulse chase assay

Radioactive pulse chase assay was performed as previously described (Yang et al. (2011)). A total of 106 HeLa cells were transfected with SDHB vectors 12 hr before labeling. Cells were starved in methionine-free DMEM with 10% FBS for 5 min followed by 15 min incubation in the same medium supplemented with 0.2 mCi/mL [³⁵S]-methonine (>1,000 Ci/mmol specific activity, Perkin Elmer, Waltham, MA). Cells were then washed and chased in DMEM with 10% FBS supplemented with 3 mg/mL methionine. At the end of each time point, cells were extracted for protein using RIPA lysis buffer with Halt proteinase inhibitor cocktail. SDHB mutant proteins were precipitated from 200 µg total lysate with Flag antibody. Proteins were eluted and separated on 4-12% Bis-Tris gel or measured by liquid scintillation counting. Gel was fixed and incubated with EN3HANCE (Perkin Elmer) for 30 min and dried for autoradiography.

### In vitro synthesis of SDHB mutant proteins

SDHB mutant sequences were cloned into MCS region in pCMV6-Entry vector, which could be driven by T7 promoter in the upstream sequence. In vitro translations were performed using a TNT T7 Quick Coupled Transcription / Translation System (Promega, Madison, WI). For each mutant, 2 µg of plasmid DNA was incubated with 40 µL TNT Quick Master Mix and 20 µCi [³⁵S]-methionine (>1,000 Ci/mmol specific activity, Perkin Elmer) at 30°C for 2 hr. Synthesized SDHB proteins were loaded on 4-12% Bis-Tris gel and analyzed through autoradiography.

### In vitro mitochondria binding and insertion assay for SDHB mutant proteins

The mitochondria binding/insertion assay was performed based on the protocol developed in our lab as previously described (Zhuang et al. (1988); Zhuang et al. (1992)). Mitochondria were isolated from HeLa cells using Qproteome Mitochondria Isolation Kit (QIAGEN, Valencia, CA). Equal amount of radioactive labeled SDHB protein and isolated mitochondria were incubated with 4 mM ATP, 10 mM creatine phosphate, 1 U/mL creatine phosphokinase, 10 mM methionine, 10 mM MgCl2, 40 mM KCl, 0.4 mM dithiothreitol, 0.3 M sucrose and 25 mM HEPES buffered to a pH of 7.5 with KOH. The reaction mixture was kept in 37°C for 30 min and then placed on ice. Mitochondria was washed and collected to evaluate mitochondria binding. For measurement of mitochondria insertion, the reaction mixture was treated with 10 µg proteinase K for 15 min on ice. Treatment was terminated by addition of 2 mM PMSF. Mitochondria was washed and lysed in 50 µL RIPA lysis buffer with Halt proteinase inhibitor cocktail. SDHB residue was analyzed by 4-12% Bis-Tris gel and autoradiography.

### Results

### Quantitative loss of SDHB protein expression in PHEOs/PGLs

To assess the level of SDHB expression in these tumors, SDHB protein expression through immunohistochemistry staining (Figure 11A) were investigated. Reductions of SDHB protein expression in SDHB-associated tumor tissues were identified. A significant reduction of SDHB protein expression was identified for the tumor sample that contained a missense mutation (ΔSDHB-R11H) as well as for a tumor sample with a nonsense mutation (ΔSDHB-T115X) (Figure 11A). In contrast, robust SDHB protein expression was found in the cytoplasm of adjacent normal adrenal cells. Quantification of SDHB revealed a 90.0% and 72.5% reduction in SDHB protein in nonsense and missense mutations associated tumors, respectively. Much of the remaining SDHB protein, particularly in tumor samples associated with nonsense mutations, likely derived from normal tissue mingled with tumor cells. To confirm this protein quantity change, we next measured SDHB protein in microdissected tumor samples with SDHB, MEN2, and VHL mutations using Western blot analysis (Figure 11B). Consistent with immunohistochemistry analysis, substantially lower expression of SDHB protein was present with approximately only 30% in SDHB PHEO/PGL samples (n=3); as compared with normally expressed SDHB protein in VHL (p<0.05, n=3) or MEN2 (p<0.01, n=3) PHEOs (Figure 11C).

In order to determine the mechanism of the protein loss, SDHB mRNA transcription in dissected tumor specimen was measured through real-time PCR (Figure 11D). There was only a slight reduction in messenger RNA expression in missense mutation associated tumors as compared with normal adrenal medullary tissue (p>0.05), suggesting mRNA transcription efficiency is intact in tumor cells. Additionally, the gene integrity of SDHB was tested through radioactive labeled short tandem repeat PCR in tumor specimen. This analysis confirmed that there was a loss of heterozygosity in SDHB gene in tumor specimen, but both alleles remained intact in normal tissue. These result indicate that mutant mRNA was intact at the transcriptional level in the absence of wild type SDHB gene.

SDHB is an essential subunit of the mitochondrial complex II on the inner membrane of mitochondria. In order to evaluate SDHB enzyme activity, mitochondrial complex II activity in SDHB-associated tumor specimens was measured and compared to normal human adrenal medullary tissue (Figure 11E). Consistent with protein loss, we identified a parallel reduction in enzyme activity of complex II in SDHB nonsense and missense mutation associated tumors. The complex II activity in all specimens tested was consistent with the quantitative loss of SDHB protein product in these tumors. Nonsense mutation associated tumors showed a loss of 93.4% of normal enzymatic activity while missense mutation associated tumors showed a loss of 86.5% of normal enzymatic activity.

### Impaired protein stability causes quantitative loss of SDHB protein

The intact mRNA expression and quantitative loss of SDHB protein product in tumors suggested that posttranslational changes occur in SDHB mutant proteins that could have led to their premature quantitative loss. It was hypothesized that increased protein misfolding and degradation would underlie the net loss of functional SDHB subunit in these tumors. To test this hypothesis, the protein stability of wild type and mutant SDHB protein were compared. Hotspot missense mutations in SDHB genes were selected based on previous findings (Van Nederveen et al. (2006); Henderson et al. (2009); Pawlu et al. (2005))) and constructed into pCMV6-Entry mammalian expression vector. SDHB mutant vectors were transfected into HeLa cells, which were later treated with cycloheximide (CHX) to inhibit protein synthesis. SDHB protein residue was measured by Western blot (Figure 12A). We identified successful expression of both wild type and mutant SDHB protein at the beginning of the treatment. Wild type SDHB protein was stable after synthesis and was observed at > 80% after 4 hr CHX treatment. The protein half-life of the wild type SDHB was 1043 hr. A rapid and significant loss of SDHB proteins was observed across all mutations measured in the same assay. The protein residue was found reduced to < 45% among all mutants. One phase decay non-linear regression showed that A43P, R46Q, L65P and W200C SDHB mutant protein half-lives were reduced to 0.58, 0.74, 0.43 and 1.54 hr, respectively (Figure 12B).

This protein stability change was confirmed through a [³⁵S]-methionine mediated pulse chase assay. Wild type and ΔSDHB-R46Q vectors were delivered into HeLa cells and protein was pulse labeled with [³⁵S]-methionine. SDHB protein was precipitated and analyzed through autoradiography (Figure 12C). Consistent with effective protein translation, the pulse chase analysis revealed that both wild type and mutant SDHB were synthesized in their full length immediately after labeling and that no notable differences in expression levels were observed after initial translation. There was a marked reduction in [³⁵S] labeled SDHB protein in R46Q mutant over the course of 12 hrs, which is in agreement with the data from CHX treatment. Autoradiography and [³⁵S] scintillation demonstrated that there was minimal reduction in wild type SDHB expression 4 hr after labeling and >75% of labeled protein remained intact. After 12 hr pulse labeling, around 50% of wild type protein was degraded, indicating an efficient turnover of SDHB protein under normal conditions. On the contrary, R46Q mutant protein exhibited accelerated degradation. Radioactive labeled protein was found extensively lost within 6 hrs after chase (Figure 2D). Similar protein losses were observed in other tumorigenic missense mutants (A43P, C196Y and W200C, data not shown). To understand the degradation dynamic of mutant SDHB protein, protein half-life was calculated through liquid scintillation counting of radioactive-labeled SDHB protein (Figure 12D). Consistent with the findings above, wild type SDHB protein was found stable and the half-life was 11.23 hr, whereas a significant loss of protein stability was found in R46Q mutant. [³⁵S]-labeled SDHB protein was found < 50% within 3 hr after labeling and further reduced below 5% within 12 hr after labeling. Consistent with CHX treatment, half-life of mutant SDHB protein was reduced to 1.73 hr.

To confirm a rapid degradation in mutant SDHB protein, we investigated the ubiquitination status of the SDHB protein with hot spot missense mutations (Figure 2E). Wild type SDHB protein was found ubiquitinated, indicating a rapid turnover and steady degradation in physiologic condition. However, elevated ubiquitin binding by an average of 57.4% was found across all missense mutant tested, suggesting the mutant proteins undergo an ubiquitin-proteasome derived degradation pathway (Figure 2F).

### Mutant SDHB proteins maintain intrinsic functions

Missense mutations may alter three-dimensional protein structure leading to both protein misfolding and degradation as well as changes to its intrinsic biological function. Since the quantitative level of protein loss was in agreement with functional loss of complex II activity, it was hypothesized that missense mutations on SDHB would have minimal effects on its intrinsic targeting and transport into the mitochondria, complex formation, and biological function. To test these possibilities, mitochondria binding, insertion, and mitochondria complex II assembly for tumorigenic missense mutants were investigated.

SDHB mutant localization through immunofluorescent staining (Figure 13A). SDHB mitochondria localization was measured based on colocalization with mitotracker. Wild type SDHB was found to co-localize with mitotracker, suggesting a correct mitochondria cargo and assembly. Interestingly, mutant SDHB proteins were also found to co-localize with mitotracker, suggesting that mutants of SDHB are capable to be assembled into mitochondria. A small amount of SDHB mutant protein was found trapped in cytoplasm, which result in a "leaky" distribution outside mitochondria.

Tounderstand whether complex II assembly efficiency is affected by missense SDHB mutations, mitochondria binding and insertion assay was performed as previously described (Zhang et al. (1998); Zhang et al. (1992))). SDHB mutant protein was synthesized in a cell free system supplemented with [³⁵S] methionine. Radioactive SDHB proteins were then incubated with isolated HeLa mitochondria to investigate protein binding. Mitochondria insertion was further tested through proteinase K incubation. Wild type SDHB protein appeared both successful mitochondria binding and insertion. On the contrary, green fluorescent protein (GFP), a cytoplasmic protein, showed little mitochondria binding and undetectable insertion. Seven SDHB missense mutants were tested in the same assay, and found almost every mutant protein exhibit both successful binding and insertion (Figure 13B). Quantification on autoradiography and [³⁵S] scintillation demonstrated a slight increased mitochondria binding and minimal changes in insertion in mutant protein, which is in agreement with immunofluorescent staining data.

SDHB forms mitochondria complex II through assembly with SDHA, C and D subunits. SDHB is physically associated with SDHA subunit on the hydrophilic, catalytic end of the complex II (Yankovskaya et al. (2003)). Further evaluation of mitochondria complex II assembly through testing SDHA binding to mutant SDHB proteins wereperformed. Wild type and mutant SDHB proteins were immunoprecipitated and tested for SDHA binding through Western blot (Figure 13C). The binding of SDHA was detected on both wild type and mutant SDHB proteins. The binding efficiency of SDHA was similar between wild type and mutant proteins, indicating that mitochondria II complex assembly of SDHB in missense mutants was intact (Figure 13D).

### Increase functional SDHB subunit through proteostasis modulators

The missense SDHB proteins tested in these studies have not only remained intact but appear to be functional as well. Therefore, these same mutated SDHB proteins are likely quantitatively insufficient in PHEOs/PGLs due to rapid degradation by ubiquitin-proteasome degradation. A potential treatment strategy would be to target the mediators that lead to the rapid degradation of SDHB mutant proteins. This may increase the functional SDHB subunit in mitochondria, recovering adequate succinate metabolism to reverse the state of pseudohypoxia. Specifically, we applied small molecule compounds known to modulate protein stability and proteostasis, including histone deacetylase inhibitors (HDACi), celastrol, and quercetin, and investigated their impact on protein half-lives. We tested SDHB stability changes through a [³⁵S]-methionine pulse chase assay. R46Q and W200C mutants were transiently labeled with [³⁵S]-methionine. Cells were chased for 4 hrs under various treatments. Protein residues were measured through autoradiography and liquid scintillation (Figure 14A and 14B). Interestingly, ther was SDHB protein stabilization in both mutants under the treatment. In particular, the W200C mutant was found to be more responsive to the treatment, with increases approaching 5 fold according to the liquid scintillation.

To confirm the drug effect on mutant protein stability, we measured ΔSDHB-L65P protein half-life through CHX treatment (Figure 14C and 14D). Consistent with data shown above, L65P mutant protein has an extremely short half-life and the protein residue was reduced to <50% within 1 hr. HDACi LB-201 and SAHA (comparative example) slowed the degradation of mutant protein in the same assay. Celastrol exhibited a stronger protein stabilization effect such that more than 50% SDHB protein remained intact after a 2 hr CHX treatment. Half-life calculation showed LB-201, SAHA (comparative example) and celastrol (comparative example) elongated L65P protein half-lived into 0.67, 2.36, and 2.67 hr, respectively.

The effect of HDACi was confirmed through investigating the ubiquitination of mutant SDHB protein through immunoprecipitation (Figure 14E). We found LB-201, LB-205 and SAHA (comparative example) decreased SDHB mutant protein ubiquitination. Hsp90 binding, which targets misfolded proteins to the ubiquitin-proteasome degradation pathway, was also decreased in the same assay.

### Example 9. Pheochromocytoma and Paraganglioma

### Methods

Tumors cells from SDHB derived tumors were treated with SAHA (comparative example), LB201 or LB2 (LB205), and SDHB protein levels were analyzed.

The structure of SAHA is:

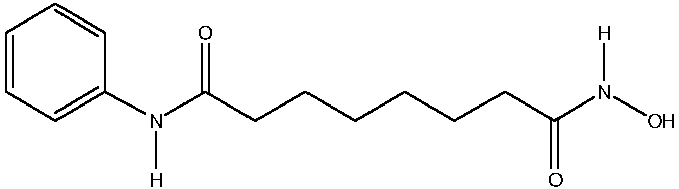

The structure of LB2 (LB205) is:

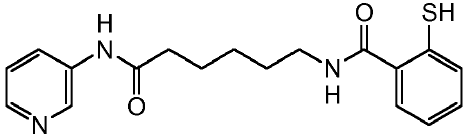

The Structure of LB201 is:

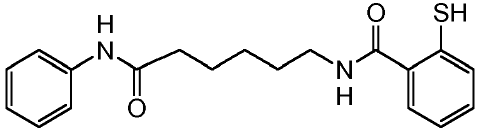

### Results

Tumors cells were treated with SAHA (comparative example), LB201 or LB205 and SDHB levels were quantified and compared. In the treated cells, SDHB levels were significantly higher than in untreated cells. Treatment with a histone deacetylase inhibitor, SAHA, LB201 or LB205, resulted in higher SDHB levels and decreased degradation of SDHB than in untreated cells. The half-life of SDHB was significantly increased as a result of treating the SDHB protein with these compounds.

### Example 10. Pheochromocytoma and Paraganglioma

### Methods

Tumors cells from SDHB derived tumors are treated with a combination of LB100, SAHA, LB201 or LB2 (LB205), and SDHB protein levels are analyzed.

The structure of SAHA is:

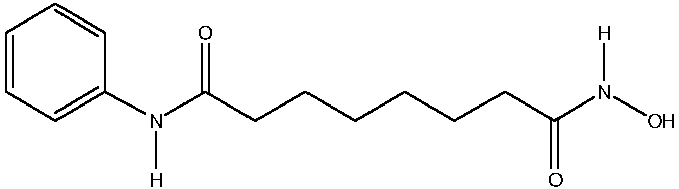

The structure of LB2 (LB205) is:

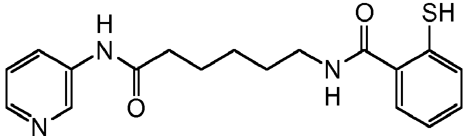

The Structure of LB201 is:

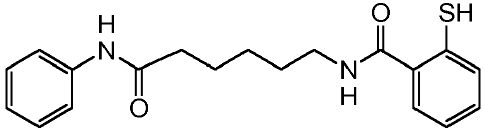

### Results

Tumors cells are treated with a combination of SAHA, LB201 or LB205 and SDHB levels are quantified and compared. In the treated cells, SDHB levels are significantly higher than in untreated cells. Treatment with a combination of SAHA, LB201 or LB205, results in higher SDHB levels and decreased degradation of SDHB than in untreated cells. The half-life of SDHB is significantly increased as a result of treating the SDHB protein with these compounds.

### Discussion

### Protein Misfunction

There a number of diseases associated with protein misfunction. Inappropriate folding of proteins may be due to mutations which destabilise the protein. The misfolded proteins are therefore rendered non-functional or sub-optimally functional and may lead to dysfunctional interactions with other proteins. The misfolded proteins may be recognized and eliminated by the cell's own machinery. Loss-of-function diseases are characterised by the absence of such proteins. Diseases caused by lack of a particular functioning protein include Gaucher's disease (misfolded beta-glucocerebrosidase), cystic fibrosis (misfolded CFTR protein) (not encompassed by the present invention), Marfan syndrome (misfolded fibrillin) (not encompassed by the present invention), Fabry disease (misfolded alpha galactosidase) (not encompassed by the present invention), von Hippel-Landau disease (misfolded VHL protein), retinitis pigmentosa 3 (misfolded rhodopsin) (not encompassed by the present invention), Pheochromocytomas (misfolded SDHB), paragangliomas (misfolded SDHB) and Hemophilia A & B (misfolded Factor VIII or Factor IX) (not encompassed by the present invention).

Misfoled proteins that escape cellular surveillance may form aggregates that lead to diseases not encompassed by the present invention such as Alzheimer's disease (Amyloid precursor protein), Huntington's disease (Huntington protein), Parkinson's disease (A-synuclein) or Transthyretin (TTR) Amyloid Polyneuropathy (TTR protein).

### Gaucher's Disease

Individuals afflicted with Gaucher's Disease have a genetic mutation in the gene coding for beta-glucocerebrosidase (GBA). Beta-glucocerebrosidase catalyzes the hydrolysis of glucocerebroside in the lysosomes, a key step in the degradation of complex lipids. However, well-characterized mutations within the gene coding for beta-glucocerebrosidase give rise to a protein that fails to localize properly to the lysosomes. Without beta-glucerebrosidase localizing to lysosomes, intracellular glucocerebroside levels increase, ultimately causing Gaucher's Disease in an individual.

Gaucher disease (GD) represents a spectrum of genetic mutations within the gene encoding for the lysosomal enzyme glucocerebrosidase (GBA). These mutations often lead to unstable and misfolded proteins that are poorly recognized by the unfolded protein response system and rapidly degraded through the ubiquitin-proteasome pathway. Modulating this response with histone deacetylase inhibitors (HDACi) have been shown to improve protein stability in other disease settings. In order to identify the mechanisms involved in the regulation of GBA and determine the effects of HDACi on protein stability, we investigated common hotspot mutations for non-neuronopathic (N370S) and neuronopathic (L444P) GD in cultured fibroblasts derived from patients and HeLa cells transfected with these mutations. Protein half-life of mutant GBA decreased corresponding to a decrease in protein levels and enzymatic activity. GBA was found to bind to Hsp70 and Hsp90, members of the chaperonin family where Hsp70 directed the protein to TCP1 for proper folding and Hsp90 directed the protein to the ubiquitin-proteasome pathway. After HDAC inhibition using a known HDACi (SAHA) and a novel small molecule HDACi (LB-205), GBA levels increased rescuing enzymatic activity in mutant cells. This increase in protein quantity can be attributed to increases in protein half-life, which corresponds primarily with a decrease in degradation rather than an increase in chaperoned folding. HDACi reduces binding to Hsp90 and prevents subsequent ubiquitination and proteasomal degradation without affecting binding to Hsp70 or TCP1. These findings provide insight into the pathogenesis of GD and demonstrate the therapeutic potential of HDAC inhibitors in the treatment of GD and other human misfolding disorders.
The discovery that novel phosphatase inhibitors and HDAC inhibitors increase the amount of protein whose deficiency underlies the human disease Gaucher's Disease, offers the potential for a new therapeutic approach to this disease. The primary approach to the treatment of Gaucher's Disease at present is to administer lifelong preparations of the protein these patients are lacking, which is highly expensive. In addition, most if not all, of such protein treatments are ineffective for treatment of disease in the brain because the proteins do not gain entry into the brain. The compounds studied, both the phosphatase inhibitors and the HDAC inhibitors, were shown previously to enter the brain (Pipalia et al., 2011; Munkacsi et al., 2011). Furthermore, protein phosphatase inhibitors have a global effect upon dephosphorylation of histones (Nowak et al., 2003). Addition of HDAC inhibitors or phosphatase inhibitors to cells harvested from an individual afflicted with Gaucher's Disease promotes a longer half-life of the mutant beta-glucocerebrosidase. Under these conditions, beta-glucocerebrosidase functions in binding and catabolizing glucocerebroside.

Gaucher disease (GD), the most prevalent hereditary metabolic disorder is transmitted in an autosomal recessive manner. This lysosomal storage disorder (LSD) results from mutations in the glucocerebrosidase gene (*GBA*) leading to accumulation of glucocerebroside in lysosomes of affected tissues (Brady et al. (1965), Brady et al. (1966)). GD is clinically classified into three types. Type I GD is non-neuronopathic, characterized by hepatosplenomegaly, cytopenias, and bone disease. Both types II and III are neuronopathic, with either acute (type II) or chronic (type III) progression of central nervous system (CNS) degeneration (Beutler and Grabowski (2001)). While any of the approximately 200 mutations identified in *GBA* may lead to the disease, these mutations do not fully account for the phenotypic variation among patients with the same genotype. Even siblings with the same mutation often present with discordant phenotypes of GD (Lachmann et al. (2004); Amato et al. (2004)), suggesting a more complex mechanism of disease surrounding this single gene mutation.

An explanation for this inconsistent genotype-phenotype correlation is differences in sensitivity of mutant glucocerebrosidase (GBA) to degradation by mediators of the protein quality control system (Lu et al. (2010)). Proteins undergo significant post-translational modification in the endoplasmic reticulum (ER). Nascent peptides form complexes with several chaperone proteins that facilitate proper folding and targeting. Misfolded proteins bind to other chaperones that direct them towards the ubiquitin-preoteasome pathway for degradation. Missense mutations in GBA destabilize the protein, rendering it vulnerable to retention and degradation in the ER (Ron and Horowitz (2005); Schmitz et al. (2005); Mu et al. (2008)). This is consistent with our previous findings that loss of cellular GBA catalytic activity result from reduced localization to the lysosome and proteasomal degradation of the mutant enzyme, rather than a decrease in its intrinsic function (Lu et al. (2010)). Thus, targeting mediators of protein homeostasis, or proteostasis, may prevent GBA degradation and restore function to affected cells.

Histone deacetylase inhibitors (HDACi) are a class of proteostasis regulators (Powers et al. (2009)) that may increase quantitative levels of enzymatically active GBA. HDAC inhibition have been demonstrated to be effective in correcting the phenotype of other diseases of aberrant protein folding, including Neimann-Pick C disease (Pipalia et al. (2011); Munkacsi et al. (2011)), cystic fibrosis (Hutt et al. (2010)), and type II diabetes mellitus (Ozcan et al. (2006)). Histone deacetylases regulate cellular function by post-translational modification of histones, transcriptional factors, and chaperones, including Hsp90 (Scroggins et al. (2007)). By altering acetylation of these proteins, HDACi can modulate gene expression of proteins in the heat shock response (HSR), alter the sensitivity of the unfolded protein response, and decrease ubiquitination and proteasomal degradation to restore function to misfolded proteins. Here, we investigate the effect of a known HDACi, suberoylanilide hydroxamic acid (SAHA) (not encompassed by the present invention), and a newly developed small-molecule HDACi, LB-205, on the stability of mutant GBA with two common mutations, N370S and L444P, in for Type I GD and Type II and Type III GD.

We report on a novel approach for the treatment of GD using HDACi to increase enzymatic activity of mutant GBA in affected tissue. After translation at the rough endoplasmic reticulum, nascent GBA is cleaved and glycosylated at four asparagine residues for translocation through the ER membrane (Erickson et al. (1985)). At the Golgi apparatus, GBA undergoes further modifications in its high mannose sugar moieties, and the mature protein is trafficked to the lysosomes to carry out its function as a lysosomal-membrane glycoprotein (Takasaki et al. (1984)). In contrast, mutated GBA proteins are eliminated by ER-associated degradation (ERAD) before they can reach the lysosome. Generally, proteins that are unable to fold property are identified by ER chaperones and funneled back to the cytosol for ubiquitin-dependent proteasomal degradation. Although several mutations produce the various phenotypes of GD, many reports have indicated that improper folding and subsequent degradation of mutant GBA by the ubiquitin-proteasome pathway leads to the cellular accumulation of glucocerebroside (Offman et al. (2010)). In a previous report, we found that the catalytic activity of GBA was not impaired by common mutations found in GD types I, II, and III, but that enzyme activity was correlated with the effective concentration of the protein in the cell (Lu et al. (2010)). Therefore, using proteostasis regulators such as HDACi to preventing degradation of GBA is a rational therapeutic option to rescue GBA function.

Current treatment for GD utilizes enzyme replacement therapy of recombinant GBA. Although effective for slowing the accumulation of glucocerebrosides in the viscera, enzyme replacement cannot treat neuronopathic forms of GD, as recombinant GBA does not cross the blood-brain barrier efficiently (Sawkar, Schmitz, Zimmer, Reczek, Edmunds, Balch and Kelly (2006); Sawkar, D'Haeze and Kelly (2006)). Recent insight into the misfolding of GBA has led to the development of pharmacological chaperones like isofagomine, which are designed to aid in the proper folding and delivery of GBA to the lysosome. Although these agents increase lysosomal delivery of several mutant variants of GBA (Khanna et al. (2010); Steet et al. (2006)), they are active site inhibitors that must be washed out of the lysosome before restoring enzyme activity (Lieberman et al. (2007); Yu et al. (2007)). Other compounds can also modulate proteostasis and may increase the quantity of active GBA without the need for a washout period. While celastrol has also been shown to rescue mutant GBA from degradation (Mu et al. (2008)), it is cytotoxic at concentrations higher than 1 µM, indicating the need for less toxic equivalents. HDACi have been shown to increase proper folding and trafficking in several other protein misfolding disorders, such as cystic fibrosis and Neimann-Pick C disease, and this strategy could be applied to GD as a means to improve endogenous GBA stability and increase delivery of active enzyme to the lysosome.

The effects of two broad-spectrum HDACi, LB-205 and SAHA (not encompassed by the present invention), were investigated to determine whether HDACi could modulate the quality control and degradation of mutant GBA. SAHA (vorinostat) is the first HDACi approved for advanced cutaneous T-cell lymphoma (Marks and Breslow (2007)) and has been found to inhibit class I HDACs 1, 2, 3, and 8, and class II HDACs, 6, 10, and 11 (27). LB-205 has a similar HDAC inhibitory function as SAHA (Figure 4A) and a longer biological half-life than SAHA *in vivo* (Figure 4B). Western blot analysis of patient-derived GD fibroblasts treated with LB-205 and SAHA revealed a significant increase in GBA quantity in both N370S and L444P fibroblasts. Similar increases in protein stability and enzymatic activity were observed in HeLa cells transfected with N370S and L444P mutants after treatment with LB-205 and SAHA. Although the relative increase in GBA quantity was less than what was observed in patient-derived type I and type III GD fibroblasts, the effect of HDACi on increasing total GBA remained. Due to the high transfection efficiency, high expression of mutant GBA is increased in these cells resulting in elevated absolute protein yield in transfected cells compared to patient-derived fibroblasts harboring the same mutations. In addition, treatment with HDACi increased the relative proportion of L444P mutant GBA protein more than N370S similar to the trend observed in patient-derived fibroblasts. Other known proteostasis regulators, including celastrol and quercetin (Nagai et al. (1995); Westerheide et al. (2004); and Westerheide and Morimoto (2005)), yielded similar increases in GBA quantity as treatment with HDACi, providing further support that HDACi act through the protein quality control system.

Although the exact mechanism of how HDACi facilitate the observed increase in enzymatically active protein is unclear, we propose that at least two parallel processes shift the cellular machinery towards increased protein expression and decreased protein degradation. The first is a direct action of HDACi on the hyperacetylation and inactivation of Hsp90, a chaperone protein implicated in the degradation of misfolded GBA. A separate, indirect action of HDACi involves the saturation of the ubiquitin-proteasome degradation pathway through the up-regulation of gene expression that non-specifically decreases the rate of GBA degradation.

HDACi could regulate proteostasis machinery through several possible mechanisms involving chaperonins and the ubiquitin-proteasome degradation pathway. Protein ubiquitination and binding to three well-accepted mediators of protein folding and maturation, Hsp90, Hsp70, and TCP1 (Khanna et al. (2010); McClellan et al. (2005)), was investigated to elucidate which of these mechanisms played a predominant role in the proteostasis of GBA. Both N370S and L444P mutants demonstrated increased ubiquitination, increased binding to Hsp90, and decreased binding to Hsp70 and TCP1. These are consistent with previous findings in von Hippel-Lindau tumor suppressor protein (McClellan et al. (2005)) and merlin (Yang et al. (2011)) where two distinct pathways mediate folding and quality control. Hsp70 and TRiC are required for correct folding of nascent protein while defective protein is transferred to the Hsp90 complex for degradation. Treatment with HDAC decreased ubiquitination in L444P mutants compared to the control, suggesting that HDAC inhibition curbed the rate of degradation by modulating the Hsp70-Hsp90 system. These findings are consistent with previous studies that have shown HDAC6 inhibition leading to hyperacetylation of Hsp90, the disassociation of Hsp90 from its co-chaperone, p23, and loss of chaperone activity (Kovacs et al. (2005)). By reducing the rate of ubiquitination and degradation and maintaining the rate of transcription, translation, and folding of GBA, the half-life of synthesized GBA increases along with its steady-state concentration in the lysosome and restores sufficient enzymatic activity to potentially rescue cells from glucocerebroside toxicity.

Another mechanism that may act in concert with the direct modulation of the Hsp70-90 pathway is a non-specific saturation of the protein quality control system, which may decrease selectivity and allow misfolded proteins to evade proteasomal degradation. HDACi alter the acetylation of histones, modulating the transcription of multiple genes that act alone or in concert to promote proper folding of GBA and other proteins (Hutt et al. (2010)). This mechanism is consistent with studies on HDAC inhibitor function in multiple myeloma and other human solid and hematologic cancers. SAHA works synergistically with proteasome inhibitors to promote protein buildup and aggregation, inducing cell death in multiple myeloma cells with uniquely high protein synthetic load (Kikuchi et al. (2010)). Although cells affected by GD are unlike cancer cells in their rate of protein synthesis, a similar principle may apply due to the general increase in gene expression after treatment with HDACi. By increasing the synthetic load and decreasing the available number of chaperones, HDACi may limit the effective binding of newly synthesized GBA to Hsp90 and prevent further ubiquitination and degradation.

Our findings suggest that HDACi may be valuable as a therapeutic option for neuronopathic and non-neuronopathic GD by decreasing the rate of degradation of mutant GBA. HDACi increased enzyme activity of N370S and L444P GD fibroblasts nearly two-fold, which may be sufficient to ameliorate the symptoms of Gaucher disease (Yu et al. (2007)). These findings also add to the general hypothesis that misfolded proteins that lose conformational stability but otherwise retain intrinsic function can be rescued from degradation by HDAC inhibition.

### von Hippel-Lindau Disease

To demonstrate that the use of HDAC inhibitors, particularly LB-201, LB-205 or SAHA (not encompassed by the present invention) stabilize defective proteins responsible for serious human diseases in addition to the mutant glucocerebrosidase of Gaucher's disease, it was demonstrated that defective proteins resulting from loss-of-function mutations in the VHL gene, which underlie the pathogenesis of von Hippel-Lindau syndrome, are also stabilized by exposure to LB-205 or SAHA. Von Hippel-Lindau disease is an autosomal dominant inherited illness caused by mutations in the VHL gene. These mutations predispose affected individuals to the occurrence of several types of tumors including hemangioblastomas, pheochromocytomas, and kidney carcinomas, although other sites may also give rise tumors (Maher et al. (2011)).
While Knudson's two-hit model of tumorigenesis describes the genetic mechanisms of inherited neoplasia syndromes, the resultant specific pathogenic mechanisms underlying functional loss of gene-specific translated proteins are not known. Despite variable mutations in tumor suppressor genes, including VHL, loss of protein function in familial neoplasia syndromes, results in dysregulation of cellular growth control pathways and results in uncontrolled proliferation and tumorigenesis. Here, we investigated the pathways that mediate mutant VHL protein quality control and degradation and then identified potential modifiers of pathways that could serve as a model for therapeutic manipulation in VHL. These potential the mechanisms of protein function loss in VHL may possibly be generalized to other inherited tumor suppressor neoplasia disorders.

Initially, VHL expression levels in tumors (hemangioblastomas, HB, and clear cell renal cell carcinomas, ccRCC) from VHL patients were studied and it was found that there was a fundamental loss in the quantity of VHL protein across all samples. These findings are consistent with results from previous studies examining the qualitative expression pattern of VHL in tumors from HB and RCC specimen (REF). While these data indicated that reduced expression could underlie tumor formation, several mechanisms could underlie this quantitative loss of mutant VHL protein.

To determine the mechanism underlying the quantitative decrease in expression and function of mutant VHL, we investigated the possible mechanisms of quantitative protein loss, including altered gene transcription, abnormal protein translation and stability changes in protein products. Quantitative real-time PCR analysis of HB and RCC harboring missense VHL mutation demonstrated that VHL mRNA expression levels similar to control tissue. However the function of VHL protein is absent since the expression of hypoxia related genes were up regulated in the same cases. Evidence of successful protein translation was demonstrated by pulse chase analysis that revealed full-length protein translation of mutant and wild type VHL. Further, based on pulse chase analysis demonstrating a reduction in mutant protein half life compared to wild type control, it appears that diminished VHL expression in VHL-associated tumor occurs as a result of increased degradation.

Next, it was investigated whether mutant VHL functional capacity is maintained by inserting plasmid vectors containing common VHL missense mutations into VHL deficient cell lines 786-0 and UMRC6. These cell lines permitted measurement of VHL expression and function attributable to mutated or wild type genes that were empirically introduced. Specifically, steady state expressed HIF1α protein was diminished with reintroduction of wild type and mutant VHL genes. These investigations were performed based on reports that VHL function includes E3 ligase directing HIF1α proteasomal degradation. Our findings showed VHL mutants exhibited similar affinity to HIF1α and HIF-derived peptides, indicatively that the catalytic activity of VHL is similar in mutant proteins. Additionally, recovery of VHL function was shown with luciferase assay that was demonstrated by a reduction in the transcription activity of hypoxia response element (HRE) indicating a reduction in the HIF signaling. This is further confirmed by decreased mRNA expression of hypoxia related gene in 786-0 cells with VHL mutant reintroduction. Taken together, these results show that missense mutant VHL functional capacity is competent and that intrinsic protein function does not seem to underlie the pathogenesis of VHL associated tumor formation.

The finding that amino acid substitutions in VHL results in quantitative reductions in protein expression, rather than intrinsic functional changes, suggest that VHL missense mutations result in altered protein stability and fate. To confirm this possibility, we used the pulse chase assay to demonstrate that mutant protein half life is significantly reduced in comparison to wild type protein. Calculated degradation kinetics of mutated VHL was found to be 2 to 4 times decreased compared to normal, an effect that may be even greater considering the effects of accumulation of mature VHL products. These findings suggest that the increased degradation rate of mutant VHL underlies net loss of protein function in VHL and implicate a protein quality control pathway through which such mutant VHL is detected and degraded within cells. Indeed, VHL protein with certain missense mutation may have higher probability in misfolding and is highly susceptible for co-translational ubiquitination and pre-mature degradation. Acceleration of these degradative pathways with missense VHL mutants may occur as a result of protein misfolding, a concept supported by previous work examining the crystal structure of VHL. We observed similar misfolding reaction by decreased Hsp70 and TCP1, and increased Hsp90 binding to mutant VHL protein.

To explore the concept that increased ubiquitin-mediated proteosomal degradation of mutant VHL underlies pathogenesis, we utilized compounds known to modulate protein degradation and quality control pathways in an attempt to illicit an increase in VHL expression. Using proteostasis regulators SAHA and LB205, we were able to show significant increases in VHL half life with manipulation of such pathways.

The findings described above have significant implications for the understanding of the pathogenesis of VHL, which may be generalizable to other familial neoplasia syndromes. Net loss of functional protein in such syndromes is due to quantitative decreases in protein expression due to accelerated involvement of protein degradation machinery within the cell, rather than an intrinsic functional loss of mutant protein. These results have implications for both diagnostics and therapeutics in such disorders. Current molecular diagnostics in this area are aimed at detecting gene mutations and loss of heterozygosity in tumor suppressor genes, which largely relies on the comparison of single-nucleotide polymorphisms on different alleles using a relatively large quantity of tumor sample as well as patient-matched normal control tissue. Our results suggest that a tumor suppressor protein quantification assay might be of great utility in the diagnosis of genetic disorders.

The therapeutic implications of the findings in this study are significant. It was demonstrated that missense mutations in VHL retain functional capacity when reintroduced in cells lacking analogous VHL expression. Additionally, it was shown that manipulation of protein quality control pathways using proteostasis regulators results in increased expression of mutant tumor suppressor protein. These results provide a potential therapeutic strategy for the pharmacologic treatment of VHL associated diseases. While further exploration is needed to identify the specific molecular mediators involved in these pathways, we provide evidence that augments the functional aspect of the two-hit hypothesis and suggest a model of intervention that may be applicable to a variety of inherited conditions.

The functional capacity of mutant VHL protein was studied by inserting 2 common mutated VHL proteins into VHL deficient cell lines. Because one reported activity of VHL protein is E3 ligase, which affects HIF1α degradation, the effect of each of the mutant proteins on HlF1a stability was measured. The VHL mutants had affinities to HIF1α and HIF-derived peptides indicating that both mutant proteins had catalytic function similar to VHL.

The half-lives of the mutant proteins were significantly shorter than wild-type VHL by a factor of 2 to 4. The addition to cultures of cells bearing mutant VHL proteins were associated with a significant increase in the stability of the mutant VHL molecules. As was showed for missense mutations in the protein associated with Gaucher's disease, the HDAC inhibitors tested again stabilized mutant VHL proteins, with the net result of increasing the amount of VHL activity in treated cells. Because HDAC inhibitors may be able to be administered to affected individuals with little or acceptable toxicity, continuously or intermittently, these results suggest a potential strategy for the pharmacologic treatment of VHL associated illnesses. Whether early intervention in the life of an affected child will lessen her expected life time burden of disease is not certain but is a possibility that should be explored.
It was also demonstrated that quantitative reduction of mutant protein in VHL-associated hemangioblastoma and clear cell renal cell carcinomas whereas mRNA expression remained at physiologic level. VHL mutant proteins were highly unstable and likely to be degraded contemporarily with protein translation. However, mutant VHL protein products exhibit considerable function as an E3 ligase to degrade hypoxia inducible factor. We demonstrated that the pre-mature degradation of VHL protein is due to protein misfolding and imbalance of chaperonin binding. Modulating this pathway with histone deacetylase inhibitors (HDACis) was found to improve VHL protein stability. Missense VHL protein has the ability to function at wild type VHL protein under circumstances that increase the longevity of the protein. These findings provide insight into the pathogenesis of VHL associated tumors and indicate a novel therapeutic potential of HDAC inhibitors for the treatment of VHL disease and other human protein misfolding disorders.

Additionally, it was demonstrated that tumorigenic mutations in VHL gene cause significant changes of VHL protein. Loss of VHL protein stability is related to the loss of VHL protein and potentially related to the pathogenesis of the syndrome. The VHL protein was rapidly degraded immediately after translation. Chaperonine and cochaperonines, such as Hsp70, Hsp90 and TCP1, were found related to the rapid degradation for mutant VHL. Interestingly, the intrinsic functions of tumor derived VHL mutants were found to be normal. Several proteostasis regulators appear to be helpful in elongating protein half life, which might provide therapeutic insight in to this type of VHL disease.

### Pheochromocytomas and Paragangliomas

Pheochromocytomas (PHEO) and paragangliomas (PGL) are tumors that can be potentially life threatening and for which no treatment other than surgery for localized masses are available. Genetic alterations in genes coding for succinate dehydrogenase (SDH) are believed to be important in the development of the most aggressive PHEO/PHL. SDH is an important component of mitochondrial function. Inherited mutations in the gene for subunit B of SDH (SDHB) are the basis of an autosomal dominant type of PHEO/PGL. PHEO/PGL have missense mutations in the SDHB gene.

Addition of HDACi LB-201, LB-205, and SAHA (not encompassed by the present invention) decreased the loss of mutant SDHB in cultures of cells containing the specific mutation L65P. Pharmacologic modulation with the HDACi resulting in an increase in functional SDHB to reduce the negative sequellae stemming from defective mitochondrial function due to insufficient SDHB may be a useful therapeutic approach to the treatment of PHEO and PGL.

Pheochromocytomas (PHEOs) and paragangliomas (PGLs) are rare but life-threatening catecholamine-producing neuroendocrine tumors. Mutations of succinate dehydrogenase subunit B (SDHB) play a crucial role in the pathogenesis of the most aggressive and metastatic PHEOs/PGLs. Although a variety of missense mutations in the coding sequence of the SDHB gene have been found in PHEOs/PGLs, the mechanism of the loss of functional SDHB is unknown. Each mutation may give rise to changes in gene expression, mRNA stability or processing, protein translation or stability, mitochondrial localization and importing, or intrinsic protein function. SDHB mRNA and protein expression from patient derived PHEOs/PGLs demonstrated intact mRNA expression, but significantly reduced protein expression. Investigations of common SDHB missense mutations in transfected cell lines demonstrated that the loss of SDHB function was due to a reduction in mutant protein half-life and its increased degradation, whereas protein subcellular localization and intrinsic functions remained unchanged. Proteostasis regulators increased mutant SDHB protein expression suggesting the involvement of protein quality control machinery in the degradation of mutant SDHB proteins. These findings provide the first direct mechanism of functional loss resulting from SDHB gene mutation. Reducing degradation of SDHB mutant protein through post-translational modification opens a novel therapeutic paradigm for SDHB-related tumors.

Classically, the mechanism for the genetic pathogenesis of solid inherited neoplastic syndromes has fallen under the generic umbrella of the Knudson two-hit paradigm (Knudson et al. (1971)). However, in many cases of cancer, the precise mechanism of how missense mutations contribute to the loss of the functional gene specific protein and lead to tumor formation remains unclear. PHEOs/PGLs can often be attributed to well-known defects in the VHL tumor suppressor pathway and stabilization of HIF-1α coupled with changes in expression of many genes that lead to unopposed proliferation and tumor development (Ivan et al. (2001); Maxwell et al. (1999)). However, PHEOs/PGLs due to SDHB mutations are less well understood. Various missense mutations in the SDHB gene have been described in these tumors, but little is understood about how these mutations lead to the loss of SDHB function as well as the fate of the resulting mutant proteins. Many defects along the pathway from gene to protein could play a role in the functional loss of mutated SDHB protein in these tumors. Reduced transcription, impaired mRNA stability, decreased translation, increased protein degradation, impaired mitochondrial localization and transport, and loss of intrinsic function all prevent proper formation and function of mitochondria complex II and inhibits the conversion of succinate to fumarate. However, in our investigations of these various potential mechanisms, we demonstrate that accelerated protein degradation gives rise to the quantitative loss of mutant SDHB protein and results in the functional insufficiency of protein function (Figure 12). Moreover, the use of proteostasis regulators can be used to prevent the degradation of SDHB mutant proteins and potentially reduce the proliferative signals of pseudohypoxia that lead to tumorigenesis.

A recent study observed an absence of SDHB protein in PHEOs/PGLs harboring the SDHB missense mutation (Astuti et al. (2001); van Nederveen et al. (2009)). We confirmed this and demonstrated a significant decrease in SDHB protein by both immunohistochemistry and by Western blot of cells isolated from biopsy specimens compared to SDHB-related tumors with VHL- and MEN2-related PHEOs/PGLs (Figure 1A, B and C). Together, our findings support that this quantitative absence of proteins is the hallmark feature of these tumors and central to the functional mechanism of SDHB mutations in its related PHEOs/PGLs. However, these tumor specimens only demonstrate slight changes in the yield of corresponding extracted mRNA that is insufficient to account for the amount of protein losses observed. Likewise, mitochondrial complex II activity in SDHB-related tumor samples was reduced in a manner parallel with the quantitative loss of SDHB protein product, suggesting that the pathogenesis of PHEOs/PGLs in SDHB mutations are due to changes at the protein level and not changes at the RNA level (Figure 11D).

Several processes play a role in protein synthesis, degradation, and function and these processes are not mutually exclusive in reducing enzymatic activity to the levels observed in SDHB-related tumors. To elucidate which mechanisms are responsible for the findings from SDHB tumor samples, protein synthesis, protein stability, targeting to the mitochondria, import into the mitochondria, and intrinsic enzyme activity were all examined. Based on previous studies related to neurofibromatosis type 2 and Gaucher's disease (Yang et al. (2011); Lu et al. (2010)), quantitative loss of SDHB is likely due to a defect in protein stability and an increase in ubiquitin-mediated proteasomal degradation. Post-translational modifications are essential mechanisms that cells utilize to sort out pre-mature target proteins for degradation as part of the endogenous protein quality control system (Bukau et al. (2006); McClellan et al. (2005)). Mutations of single amino acid substitutions lead to post-translational ubiquitination and pre-mature degradation of merlin and glucocerebrosidase, suggesting that the protein quality control system is responsible for regulating mutant proteins by accelerating degradation (37,48). Accelerated degradation for SDHB mutant protein can occur as a result of protein misfolding during maturation, a mechanism extensively studied in VHL mutations (Feldman et al. (2003); McClellan et al. (2005); Melville et al. (2003)). A similar misfolding reaction was observed by changes in Hsp90 binding to mutant SDHB protein, which has been demonstrated to be essential for the establishment of early degradation complex (Dickey et al. (2007)). Further investigation will be necessary to understand the dimensional changes caused by missense mutations and explain the targeting of SDHB to the ubiquitin-proteasome degradation pathway.

Missense mutations may affect not only to protein instability, but also to changes in intrinsic protein targeting, transport, and function in its target organelle. Although not demonstrated for every potential variant of SDHB mutations, the hotspot SDHB mutations we analyzed maintain the proper conformation for transport into the mitochondria and form an active mitochondria complex II supported by immunofluorescence and biochemical assays (Figure 11E and 13). Furthermore, SDHB appears to maintain proper function once imported into the mitochondria as these mutant SDHB proteins maintain their ability to associate with SDHA, another subunit in mitochondria complex II. The physical interaction between mutated SDHB proteins with SDHA confirms that although these mutants may have increased susceptibility for degradation, they still retain their intrinsic capacity to target and insert into proper mitochondrial compartment and form complex II associations (Figure 13C and 13D). Therefore, the loss of mitochondrial complex II enzymatic activity occurs more likely due to a quantitative loss of the mutated SDHB protein instead of an intrinsic loss of enzymatic activity.

The demonstration that PHEOs/PGLs arise from the background of a quantitative loss of SDHB rather than a qualitative defect in its localization and complex formation may explain some aspects of its tumorigenic risk. Interestingly, Ricketts et al. describes an increased risk for the development of PHEOs/PGLs in patients with missense mutations of SDHB compared to patients with nonsense mutations of SDHB (Ricketts et al. (2010)). This finding seems counterintuitive given the proposed mechanism of pseudohypoxia that leads to tumorigenesis in patients with SDHB mutations. We found that patients with nonsense mutations had a greater reduction in SDHB protein expression in their tumors than patients with missense mutations (Figure 11E). These patients should also have less functional activity of mitochondrial complex II, greater accumulation of succinate in their cytoplasm, and increased stabilization of HIF-α. However, since loss of heterozygosity appears to be a necessary precursor to tumorigenesis in SDHB-related PHEOs/PGLs and a quantitative loss of protein due to degradation occurs rather than a change in intrinsic function in missense mutations of SDHB, the increased risk of tumorigenesis in missense mutations is consistent with other solid tumor neoplasia syndromes such as VHL (Chen et al. (1995); Chen, Kishida, Yao et al. (1995)). Complete loss of both copies of the gene is more likely to cause severe dysfunction of the target cell that is developmentally lethal and not conducive to tumor formation. However, partial quantitative loss of a missense mutation followed by a deletion of the second allele leads to an insufficiency of protein function that results in an imbalance of cell growth towards proliferation that underlies tumorigenesis.

By understanding the underlying mechanism that leads to tumorigenesis, this study also provides significant clinical implications for both diagnosis and future treatment options of tumors associated with SDHB mutations. Current diagnosis of tumors with SDHB mutations largely relies on the detection of loss of heterozygosity (Gimenez-Roqueplo et al. (2004); Benn et al. (2003)), which applies comparisons of single nucleotide polymorphisms of different alleles. This requires a large amount of tumor sample in order to obtain consistent results, leading to significant variability in predictive value. However, this study suggests an alternative diagnostic approach via direct quantification of the SDHB protein or measurements of complex II activity from relative small amount of sample. Because the pathogenesis of these tumors occurs due to changes in protein quantity, a technique that relies on protein content and not on genetic material could provide more consistent diagnosis and prognosis of these aggressive and lethal tumors.

This understanding also provides a mechanism of reversing much of the risk associated with SDHB mutations. In this study, we demonstrate the potential of proteostasis regulators such as HDACi to increase the quantity of functional SDHB (Figure 14). The manipulation of the protein quality control system via HDACi and other proteostasis regulators results in increased stability of the protein, increasing the total amount of SDHB protein in the mitochondria. This may lead to the formation of enough functional mitochondrial complex II to rescue cellular enzymatic activity in order to scavenge succinate, reduce the formation of ROS, and destabilize HIF-α. In this way, HDACi may limit, if not prevent, the exact perturbations that lead to the pathogenesis of PHEOs/PGLs. The use of such specific molecular mediators targeting the ubiquitin-proteasome degradation pathway may provide an effective treatment paradigm for SDHB-related neoplasias and other similar human diseases of accelerated protein degradation.

### Summary

Although both direct and indirect inhibition of protein degradation are likely involved in the rescue of mutant GBA clarifying the mechanism by which HDACi mediate proteostasis has significant implications for many other diseases caused by misfolded proteins, including other lysosomal storage diseases (Mu et al. (2008); Powers et al. (2009)), neurofibromatosis type 2 (Yang et al. (2011)), cystic fibrosis (Hutt et al. (2010)), and type II diabetes mellitus (Ozcan et al. (2006)). By targeting the pathways involved in proteostasis, regulators such as HDACi or protein phosphatase inhibitors may be able to universally restore normal protein function in these common diseases.

Protein phosphatase inhibitors (LB100) or HDACi (SAHA, LB-201 and LB-205) are used for the treatment of any condition characterized by protein misfunction that results from a genetic defect. The treatment is potentially effective for a multitude of diseases because it reduces the rate of degradation of an essential protein. The general approach to these diseases in the past has been developing a treatment that attempts to increase the amount of the deficient protein either 1) directly synthesize the protein and inject it into the patient or 2) duplicate the gene without the destabilizing mutation and establish that element in the patient (gene therapy). These "replacement" approaches are disease specific and much more complicated whereas the present invention is not disease specific as it affects a general cellular process affecting the stability (half-life) of a multitude of mutationally defective proteins and should be useful for many diseases.

### References

Amar, L. et al. (2005) J Clin Oncol 23, 8812-8818.
Amato D et al. (2004)Gaucher disease: variability in phenotype among siblings. J Inherit Metab Dis 27(5):659-669.
Astuti, D. et al.(2011) Endocr Relat Cancer 18, 73-83.
Astuti, D., et al. (2001) Lancet 357, 1181-1182.
Baysal, B. E. et al. (2000) Science 287, 848-851.
Baysal, B. E. (2003) Trends Endocrinol Metab 14, 453-459.
Benn, D. E. et al. (2003) Oncogene 22, 1358-1364.
Berger, A. H. et al. (2011) Nature 476, 163-169.
Bergmann JE & Grabowski GA (1989) Posttranslational processing of human lysosomal acid beta-glucosidase: a continuum of defects in Gaucher disease type 1 and type 2 fibroblasts. Am J Hum Genet 44 (5) :741-750. Beutler E & Grabowski G (2001) The Metabolic & Molecular Bases of Inherited Disease (McGraw-Hill, New York) 8 Ed.
Brady RO, Kanfer JN, Bradley RM, & Shapiro D (1966) Demonstration of a deficiency of glucocerebroside-cleaving enzyme in Gaucher's disease. J Clin Invest 45(7):1112-1115.
Brady RO, Kanfer JN, & Shapiro D(1965) Metabolism of Glucocerebrosides. Ii. Evidence of an Enzymatic Deficiency in Gaucher's Disease. Biochem Biophys Res Commun 18:221-225.
Bukau, B. et al. (2006) Cell 125, 443-451.
Chen, F., Kishida, T., Duh, F. M., Renbaum, P., Orcutt, M. L., Schmidt, L., and Zbar, B. (1995) Cancer Res 55, 4804-4807.
Chen, F. et al. (1995) Hum Mutat 5, 66-75.
Dahia, P. L. et al. (2005) PLoS Genet 1, 72-80.
de Fost M (2003) "Gaucher disease: from fundamental research to effective therapeutic interventions". Netherlands Journal of Medicine 61(1)3-8.
DeLellis, R. A. et al. (2004) Tumors of Endocrine Organs, Lyon: IARC Press.
Dickey, C. A. et al. (2007) J Clin Invest 117, 648-658.
DiMartino, J. (2004) US 2004/0204339 A1.
Eisenhofer, G. et al. (2004) Endocr Relat Cancer 11, 423-436.
Eng, C. (1999) J Clin Oncol 17, 380-393.
Eng, C. et al. (2003) Nat Rev Cancer 3, 193-202.
Erickson AH, Ginns EI, & Barranger JA (1985) Biosynthesis of the lysosomal enzyme glucocerebrosidase. J Biol Chem260(26):14319-14324.
Favier, J., et al. (2010) Best Pract Res Clin Endocrinol Metab 24, 957-968.
Feldman, D. E., et al. (2003) Mol Cell 12, 1213-1224.
Fliedner, S. M., et al. (2010) Cell Tissue Res 340, 607-612.
Franchini, M. et al. (2012) Orphanet Journal of Rare Diseases 7, 24.
Furuta, M., et al. (2004) Oncogene 23, 6806-6814.
Gao, P., et al. (2007) Cancer Cell 12, 230-238.
Gerald, D., et al. (2004) Cell 118, 781-794.
Gill, A. J. et al. (2011) N Engl J Med, 364, 885-886.
Gimenez-Roqueplo, A. P. et al. (2003) Cancer Res 63, 5615-5621.
Gottlieb, E., and Tomlinson, I. P. (2005) Nat Rev Cancer 5, 857-866.
Gregersen, N. et al. (2006) annu. Rev. Genomics Hum. Genet. (2006) 7, 103-124.
Gutmann, D. H. et al. (1997) JAMA 278, 51-57.
Guzy, R. D. et al. (2008) Mol Cell Biol 28, 718-731.
Hensen, E. F. et al. (2011) Fam Cancer 10, 355-363.
Henderson, A. et al. (2009) Fam Cancer 8, 257-260.
Hentsch, B. et al. (2011) US 2011/0092447 A1.
Huang, H. et al. (2008) Cancer 113, 2020-2028.
Hutt DM, et al. (2010) Reduced histone deacetylase 7 activity restores function to misfolded CFTR in cystic fibrosis. Nat Chem Biol 6(1):25-33.
Ivan, M. et al. (2001) Science 292, 464-468
Kaelin, W. G. (2007) Annu Rev Pathol 2, 145-173.
Kamitani, T. et al. (1997) J Biol Chem 272, 28557-28562.
Khanna R, et al. (2010) The pharmacological chaperone isofagomine increases the activity of the Gaucher disease L444P mutant form of β-glucosidase. FEBS Journal 277(7):1618-1638.
Kikuchi J, et al. (2010) Histone deacetylases are critical targets of bortezomib-induced cytotoxicity in multiple myeloma. Blood 116(3):406-417.
Knudson, A. G., Jr. (1971) Proc Natl Acad Sci USA 68, 820-823.
Kovacs JJ, et al. (2005) HDAC6 Regulates Hsp90 Acetylation and Chaperone-Dependent Activation of Glucocorticoid Receptor. Molecular Cell 18(5):601-607.
Lachmann RH, Grant IR, Halsall D, & Cox TM (2004) Twin pairs showing discordance of phenotype in adult Gaucher's disease. QJM 97(4):199-204.
Laurila, K. et al. (2009) BMC Genomics, 10, 122.
Lee, S. et al. (2005) Cancer Cell 8, 155-167.
Lenders, J. W. et al. (2005) Lancet 366, 665-675.
Li, J., et al. (2012) Vol. 18, No. 5, 783-791.
Lieberman RL, et al. (2007) Structure of acid beta-glucosidase with pharmacological chaperone provides insight into Gaucher disease. Nat Chem Biol 3(2):101-107.
Lu J, et al. (2010) Decreased glucocerebrosidase activity in Gaucher disease parallels quantitative enzyme loss due to abnormal interaction with TCP1 and c-Cbl. Proc Natl Acad Sci USA 107(50):21665-21670.
Lu, J. et al. (2010) Proc Natl Acad Sci USA 107, 21665-21670.
Marks PA & Breslow R (2007) Dimethyl sulfoxide to vorinostat: development of this histone deacetylase inhibitor as an anticancer drug. Nat Biotechnol 25(1):84-90.
Marks PA & Xu WS (2009) Histone deacetylase inhibitors: Potential in cancer therapy. J Cell Biochem 107(4):600-608.
Maxwell, P. H. et al. (1999) Nature 399, 271-275.
McClellan AJ, Scott MD, & Frydman J (2005) Folding and Quality Control of the VHL Tumor Suppressor Proceed through Distinct Chaperone Pathways. Cell 121(5):739-748.
McClellan, A. J. et al. (2005) Nat Cell Biol 7, 736-741.
Melville, M. W. et al. (2003) Mol Cell Biol 23, 3141-3151. Mu TW, et al. (2008) Chemical and biological approaches synergize to ameliorate protein-folding diseases. Cell 134(5):769-781.
Munkacsi AB et al. (2011) "An 'exacerbate-reverse' strategy in yeast identifies histone-deacetylase inhibition as a correction for cholesterol and sphingolipid transport defects in human Niemann-Pick type C disease". J Biol Chem 286: 23842-23851.
Nagai N, Nakai A, & Nagata K (1995) Quercetin suppresses heat shock response by down regulation of HSF1. Biochem Biophys Res Commun 208(3):1099-1105.
Neumann, H. P. et al. (2002) N Engl J Med 346, 1459-1466.
Neumann, H. P. et al. (2004) JAMA 292, 943-951.
Nowak SJ et al. (2003) "Protein phosphatase 2A activity affects histone H3 phosphorylation and transcription in Drosophila melanogaster". Mol Cell Bio 23(17)6129-6138.
Offman MN, Krol M, Silman I, Sussman JL, & Futerman AH (2010) Molecular basis of reduced glucosylceramidase activity in the most common Gaucher disease mutant, N370S. J Biol Chem 285(53):42105-42114.
Ozcan U, et al. (2006) Chemical Chaperones Reduce ER Stress and Restore Glucose Homeostasis in a Mouse Model of Type 2 Diabetes. Science 313(5790):1137-1140.
Pawlu, C. et al. (2005) Fam Cancer 4, 49-54.
Pey, A. L. et al. (2007) The American Journal of Human genetics, 81, 1006-1024.
Pipalia NH, et al. (2011) Histone deacetylase inhibitor treatment dramatically reduces cholesterol accumulation in Niemann-Pick type C1 mutant human fibroblasts. Proc Natl Acad Sci USA 108: 5620-5625.
Powers ET, et al. (2009) Biological and chemical approaches to diseases of proteostasis deficiency. Annu Rev Biochem 78:959-991.
Ricketts, C. et al. (2008) J Natl Cancer Inst 100, 1260-1262.
Ricketts, C. J. et al. (2010) Hum Mutat 31, 41-51. Ron I & Horowitz M (2005) ER retention and degradation as the molecular basis underlying Gaucher disease heterogeneity. Human Molecular Genetics 14(16):2387-2398.
Sancho-Pelluz, J. et al. (2010) Cell Death and Disease, 1, e24.
Sawkar AR, D'Haeze W, & Kelly JW (2006) Therapeutic strategies to ameliorate lysosomal storage disorders--a focus on Gaucher disease. Cell Mol Life Sci 63(10):1179-1192.
Sawkar AR, et al. (2006) Chemical chaperones and permissive temperatures alter localization of Gaucher disease associated glucocerebrosidase variants. ACS Chem Biol 1(4):235-251.
Schmitz M, et al. (2005) Impaired trafficking of mutants of lysosomal glucocerebrosidase in Gaucher's disease. The International Journal of Biochemistry & Cell Biology 37(11) :2310-2320.
Scroggins BT, et al. (2007) An Acetylation Site in the Middle Domain of Hsp90 Regulates Chaperone Function. Molecular cell 25(1) :151-159.
Sek Tong Ong et al. Nature Chemical Biology 6, 424-432.
Selak, M. A. et al. (2005) Cancer Cell 7, 77-85.
Sifers et al. (2010) Nature Chemical Biology 6, 400-401.
Slane, B. G. et al. (2006) Cancer Res 66, 7615-7620.
Steet RA, et al. (2006) The iminosugar isofagomine increases the activity of N370S mutant acid beta-glucosidase in Gaucher fibroblasts by several mechanisms. Proc Natl Acad Sci U S A 103 (37) :13813-13818.
Takasaki S, et al. (1984) Structure of the N-asparagine-linked oligosaccharide units of human placental beta-glucocerebrosidase. J Biol Chem 259(16):10112-10117.
Tormos, K. V., and Chandel, N. S. (2010) J Cell Mol Med 14, 795-804.
Trotman, L. C. et al. (2003) PLoS Biol 1, E59.
Thusberg, J. "Molecular Effectrs of Missense Mutations" (2010) Acta Universitatis Tamperensis, No. 1528, Tampere University Press, Distributed by Coronet Books..
Van Nederveen, F. H. et al. 2006) Ann N Y Acad Sci 1073, 177-182.
Van Nederveen, F. H. et al. (2009) Lancet Oncol 10, 764-771. Maher et al. European Journal of Human genetics (2011) 19, 617-623.
Westerheide SD, et al. (2004) Celastrols as Inducers of the Heat Shock Response and Cytoprotection. J BiolChem 279(53) :56053-56060.
Westerheide SD & Morimoto RI (2005) Heat Shock Response Modulators as Therapeutic Tools for Diseases of Protein Conformation. J Biol Chem 280(39):33097-33100.
Wiest, O. et al. (2011) US 2011/0071109 A1.
Wilkie, S. E. et al. (2008) Molecular Vision, 14, 683-690.
Yaguchi J. et al. (2004) Mol. Cell. Biol. 24, 15, 6569.
Yang C, et al. (2011) Missense mutations in the NF2 gene result in the quantitative loss of merlin protein and minimally affect protein intrinsic function. Proc Natl Acad Sci USA 108(12) :4980-4985.
Yang, C. et al. (2011) Proc Natl Acad Sci USA 108, 4980-4985.
Yankovskaya, V. et al. (2003) Science 299, 700-704.
Yu Z, Sawkar AR, Whalen LJ, Wong CH, & Kelly JW (2007) Isofagomine- and 2,5-anhydro-2,5-imino-D-glucitol-based glucocerebrosidase pharmacological chaperones for Gaucher disease intervention. J Med Chem 50(1) :94-100.
Zhuang, Z. et al. (1995) Am J Pathol 146, 620-625.
Zhuang, Z., Hogan, M., and McCauley, R. (1988) FEBS Lett 238, 185-190.
Zhuang, Z. P. et al. (1992) J Biol Chem 267, 591-596.

## Claims

1. A histone deacetylase inhibitor having the structure: 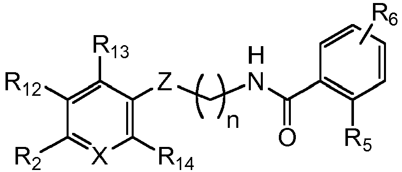 wherein
n is 1-10;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
Z is 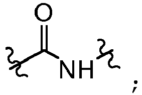
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, SO₂R₁₅, NO₂, trifluoromethyl, methoxy, or CO-R₁₅, wherein R₁₅ is alkyl, alkenyl, alkynyl, C₃-C₈ cycloalkyl, or aryl, or
a salt of the compound, for use in the treatment of a mammalian subject afflicted with a disease **characterized by** a loss of protein function caused by a genetic abnormality associated with the disease,
wherein the disease is Gaucher's disease, von Hippel-Lindau disease, pheochromocytoma or paraganglioma.

2. The inhibitor for use according to claim 1, wherein the disease is pheochromocytoma or paraganglioma.

3. The inhibitor for use according to claim 1, wherein the disease is von Hippel-Lindau disease.

4. The inhibitor for use according to claim 1, wherein the disease is Gaucher's disease.

5. The inhibitor for use according to claim 1, wherein the histone deacetylase inhibitor has the structure 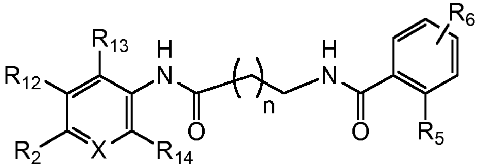 wherein
n is 1-9;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, SO₂R₁₅, NO₂, trifluoromethyl, methoxy, or CO-R₁₅, wherein R₁₅ is alkyl, alkenyl, alkynyl, C₃-C₈ cycloalkyl, or aryl.

6. The inhibitor for use according to claim 1, wherein the histone deacetylase inhibitor has the structure 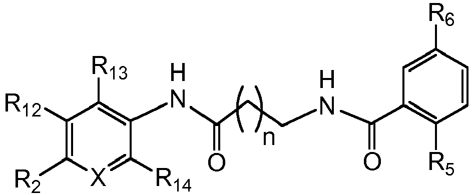 wherein
n is 1-9;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently H, C₁-C₆ alkyl, or C₃-C₈ cycloalkyl;
R₅ is OH or SH; and
R₆, R₁₂, R₁₃, and R₁₄ are each independently H, OH, SH, F, Cl, trifluoromethyl, methoxy, or CO-R₁₅, wherein R₁₅ is alkyl, alkenyl, alkynyl, C₃-C₈ cycloalkyl, or aryl.

7. The inhibitor for use according to claim 1, wherein the histone deacetylase inhibitor has the structure 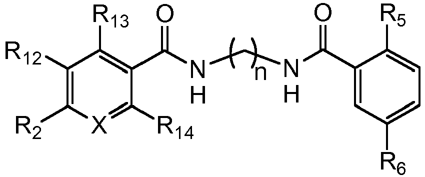 wherein
n is 1-8;
X is C-R₁₁ or N, wherein R₁₁ is H, OH, SH, F, Cl, SO₂R₇, NO₂, trifluoromethyl, methoxy, or CO-R₇, wherein R₇ is alkyl, alkenyl, alkynyl, C₃-C₈ cycloalkyl, or aryl;
R₂ is H or NR₃R₄, wherein R₃ and R₄ are each independently C₁-C₆ alkyl or C₃-C₈ cycloalkyl; and
R₅ is OH or SH; and
R₆, R₁₂, R₁₃, and R₁₄ are each independently H, OH, SH, F, Cl, SO₂R₁₅, NO₂, trifluoromethyl, methoxy, or CO-R₁₅, wherein R₁₅ is alkyl, alkenyl, alkynyl, C₃-C₈ cycloalkyl, or aryl, or
a salt of the compound.

8. The inhibitor for use according to claim 1 having the structure 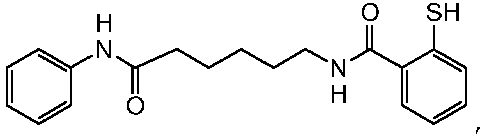 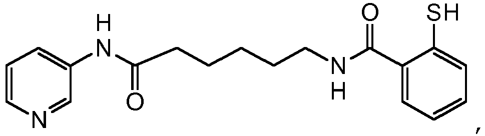 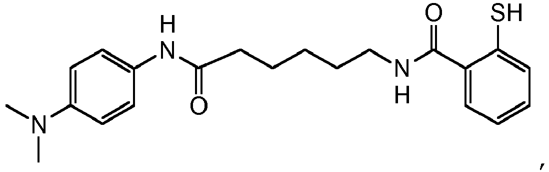 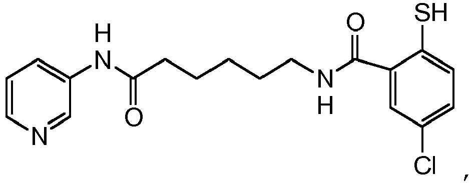 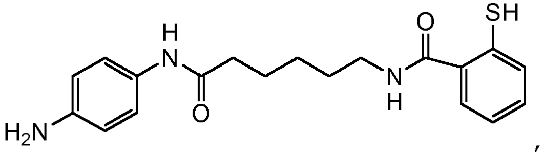 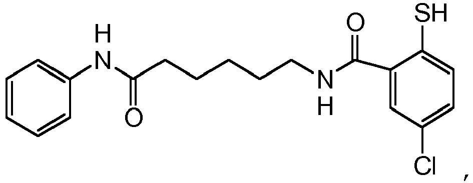 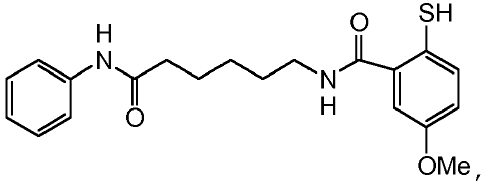 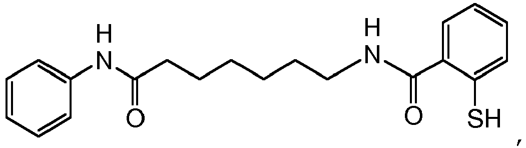 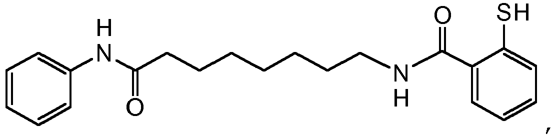 or 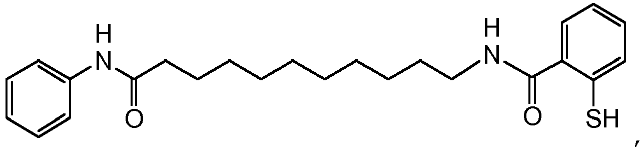 or a salt of the compound.

9. The inhibitor for use according to claim 1 having the structure 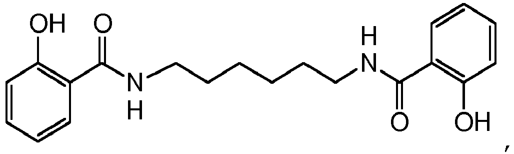 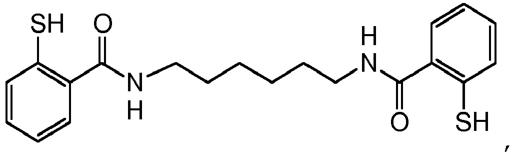 or 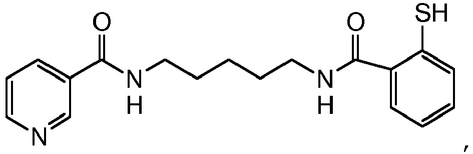 or a salt of the compound.

10. A histone deacetylase inhibitor having the structure: 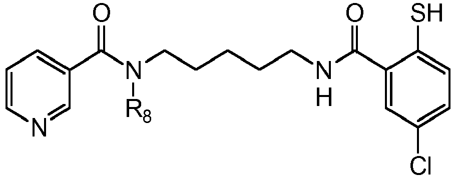 wherein R₈ = alkyl or aryl, or a salt of the compound, for use in the treatment of a mammalian subject afflicted with a disease **characterized by** a loss of protein function caused by a genetic abnormality associated with the disease,
wherein the disease is Gaucher's disease, von Hippel-Lindau disease, pheochromocytoma or paraganglioma.

11. The inhibitor for use according to claim 10, wherein the disease is pheochromocytoma or paraganglioma.

12. The inhibitor for use according to claim 10, wherein the disease is von Hippel-Lindau disease.

13. The inhibitor for use according to claim 10, wherein the disease is Gaucher's disease.

14. The inhibitor for use according to any one of claims 1-13, further comprising a protein phosphatase 2A inhibitor.

## Patentansprüche

1. Ein Histondeacetylase-Inhibitor mit der Struktur: 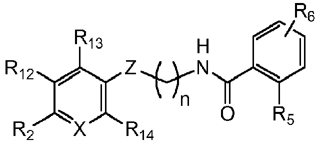 wobei
n gleich 1-10 ist;
X gleich C-R₁₁ oder N ist, wobei R₁₁ gleich H, OH, SH, F, Cl, SO₂R₇, NO₂, Trifluormethyl, Methoxy oder CO-R₇ ist, wobei R₇ für Alkyl, Alkenyl, Alkinyl, C₃-C₈- Cycloalkyl oder Aryl steht;
Z gleich 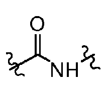 ist;
R₂ gleich H oder NR₃R₄ ist, wobei R₃ und R₄ jeweils unabhängig H, C₁-C₆-Alkyl oder C₃-C₈-Cycloalkyl sind;
R₅ gleich OH oder SH ist; und
R₆, R₁₂, R₁₃ und R₁₄ jeweils unabhängig H, OH, SH, F, Cl, SO₂R₁₅, NO₂, Trifluormethyl, Methoxy oder CO-R₁₅ sind, wobei R₁₅ für Alkyl, Alkenyl, Alkinyl, C₃-C₈-Cycloalkyl oder Aryl steht oder
ein Salz der Verbindung, zur Verwendung bei der Behandlung eines Säugetiers, das an einer Krankheit **gekennzeichnet durch** einen Verlust von Proteinfunktion, verursacht durch eine genetische Abnormalität, die mit der Krankheit in Verbindung steht, leidet, wobei die Krankheit Morbus Gaucher, von-Hippel-Lindau-Syndrom, Phäochromozytom oder Paragangliom ist.

2. Der Inhibitor zur Verwendung gemäß Anspruch 1, wobei die Krankheit Phäochromozytom oder Paragangliom ist.

3. Der Inhibitor zur Verwendung gemäß Anspruch 1, wobei die Krankheit von-Hippel-Lindau-Syndrom ist.

4. Der Inhibitor zur Verwendung gemäß Anspruch 1, wobei die Krankheit Morbus Gaucher ist.

5. Der Inhibitor zur Verwendung gemäß Anspruch 1, wobei der Histondeacetylase-Inhibitor die Struktur 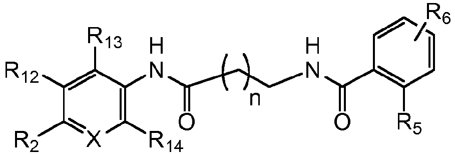 aufweist, wobei
n gleich 1-9 ist;
X gleich C-R₁₁ oder N ist, wobei R₁₁ gleich H, OH, SH, F, Cl, SO₂R₇, NO₂, Trifluormethyl, Methoxy oder CO-R₇ ist, wobei R₇ für Alkyl, Alkenyl, Alkinyl, C₃-C₈-Cycloalkyl oder Aryl steht;
R₂ gleich H oder NR₃R₄ ist, wobei R₃ und R₄ jeweils unabhängig H, C₁-C₆-Alkyl oder C₃-C₈-Cycloalkly sind;
R₅ gleich OH oder SH ist; und
R₆, R₁₂, R₁₃ und R₁₄ jeweils unabhängig H, OH, SH, F, Cl, SO₂R₁₅, NO₂, Trifluormethyl, Methoxy oder CO-R₁₅ sind, wobei R₁₅ für Alkyl, Alkenyl, Alkinyl, C₃-C₈-Cycloalkyl oder Aryl steht.

6. Der Inhibitor zur Verwendung gemäß Anspruch 1, wobei der Histondeacetylase-Inhibitor die Struktur 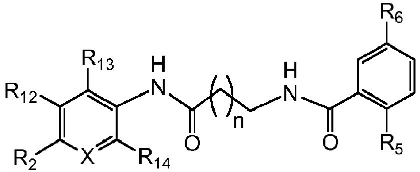 aufweist, wobei
n gleich 1-9 ist;
X gleich C-R₁₁ oder N ist, wobei R₁₁ gleich H, OH, SH, F, Cl, SO₂R₇, NO₂, Trifluormethyl, Methoxy oder CO-R₇ ist, wobei R₇ für Alkyl, Alkenyl, Alkinyl, C₃-C₈-Cycloalkyl oder Aryl steht;
R₂ gleich H oder NR₃R₄ ist, wobei R₃ und R₄ jeweils unabhängig H, C₁-C₆-Alkyl oder C₃-C₈-Cycloalkyl sind;
R₅ gleich OH oder SH ist; und
R₆, R₁₂, R₁₃ und R₁₄ jeweils unabhängig H, OH, SH, F, Cl, Trifluormethyl, Methoxy oder CO-R₁₅ sind, wobei R₁₅ für Alkyl, Alkenyl, Alkinyl, C₃-C₈-Cycloalkyl oder Aryl steht.

7. Der Inhibitor zur Verwendung gemäß Anspruch 1, wobei der Histondeacetylase-Inhibitor die Struktur 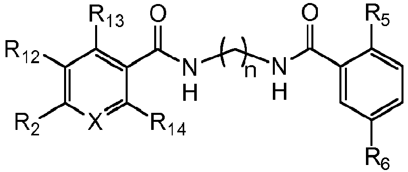 aufweist, wobei
n gleich 1-8 ist;
X gleich C-R₁₁ oder N ist, wobei R₁₁ gleich H, OH, SH, F, Cl, SO₂R₇, NO₂, Trifluormethyl, Methoxy oder CO-R₇ ist, wobei R₇ für Alkyl, Alkenyl, Alkinyl, C₃-C₈-Cycloalkyl oder Aryl steht;
R₂ gleich H oder NR₃R₄ ist, wobei R₃ und R₄ jeweils unabhängig C₁-C₆-Alkyl oder C₃-C₈-Cycloalkyl sind; und
R₅ gleich OH oder SH ist; und
R₆, R₁₂, R₁₃ und R₁₄ jeweils unabhängig H, OH, SH, F, Cl, SO₂R₁₅, NO₂, Trifluormethyl, Methoxy oder CO-R₁₅ sind, wobei R₁₅ für Alkyl, Alkenyl, Alkinyl, C₃-C₈-Cycloalkyl oder Aryl steht oder
ein Salz der Verbindung.

8. Der Inhibitor zur Verwendung gemäß Anspruch 1 mit der Struktur 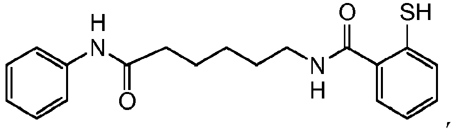 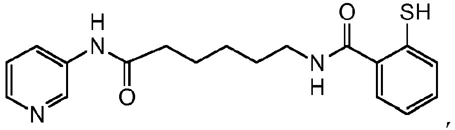 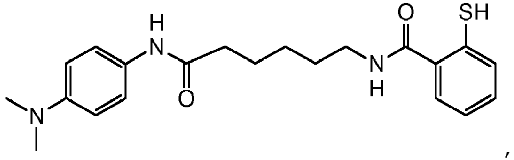 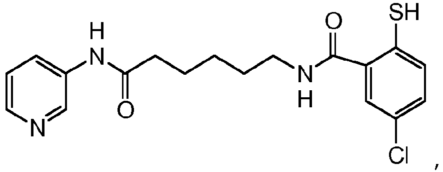 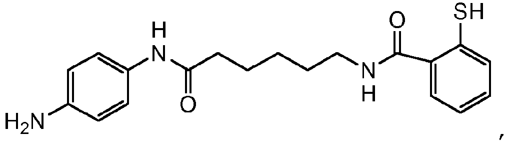 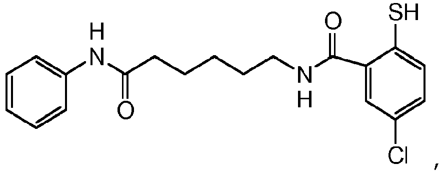 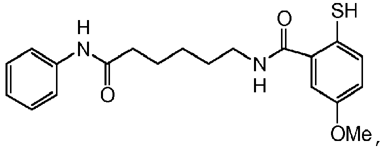 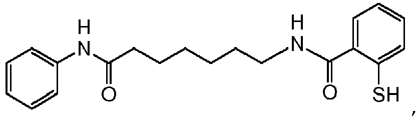 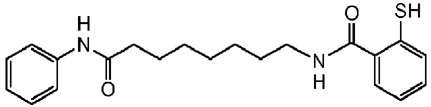 oder 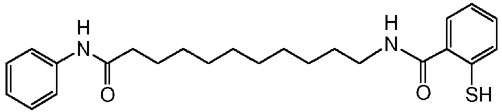 oder ein Salz der Verbindung.

9. Der Inhibitor zur Verwendung gemäß Anspruch 1 mit der Struktur 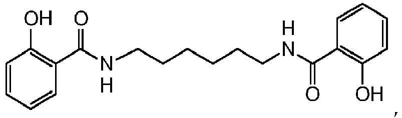 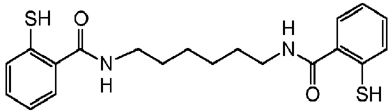 oder 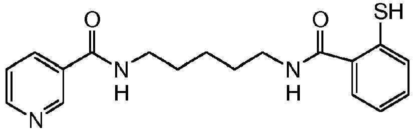 oder ein Salz der Verbindung.

10. Ein Histondeacetylase-Inhibitor mit der Struktur: 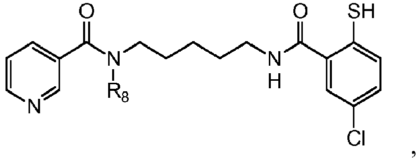
wobei R₈ = Alkyl oder Aryl oder
ein Salz der Verbindung, zur Verwendung bei der Behandlung eines Säugetiers, das an einer Krankheit **gekennzeichnet durch** einen Verlust von Proteinfunktion, verursacht durch eine genetische Abnormalität, die mit der Krankheit in Verbindung steht, leidet,
wobei die Krankheit Morbus Gaucher, von-Hippel-Lindau-Syndrom, Phäochromozytom oder Paragangliom ist.

11. Der Inhibitor zur Verwendung gemäß Anspruch 10, wobei die Krankheit Phäochromozytom oder Paragangliom ist.

12. Der Inhibitor zur Verwendung gemäß Anspruch 10, wobei die Krankheit von-Hippel-Lindau-Syndrom ist.

13. Der Inhibitor zur Verwendung gemäß Anspruch 10, wobei die Krankheit Morbus Gaucher ist.

14. Der Inhibitor zur Verwendung gemäß einem der Ansprüche 1-13, ferner umfassend einen Proteinphosphatase-2A-Inhibitor.

## Revendications

1. Inhibiteur d'histone désacétylase ayant la structure : 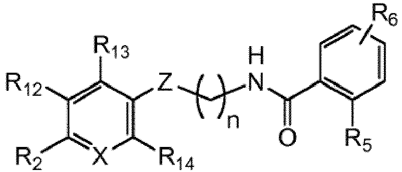 dans laquelle
n vaut 1-10 ;
X est C-R₁₁ ou N, R₁₁ étant H, OH, SH, F, Cl et SO₂R₇, NO₂, trifluorométhyle, méthoxy, ou CO-R₇, R₇ étant alkyle, alcényle, alcynyle, cycloalkyle en C₃-C₈, ou aryle ;
Z est 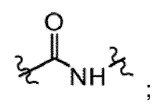
R₂ est H ou NR₃R₄, R₃ et R₄ étant chacun indépendamment H, alkyle en C₁-C₆, ou cycloalkyle en C₃-C₈;
R₅ est OH ou SH ; et
R₆, R₁₂, R₁₃, et R₁₄ sont chacun indépendamment H, OH, SH, F, Cl, SO₂R₁₅, NO₂, trifluorométhyle, méthoxy, ou CO-R₁₅, R₁₅ étant alkyle, alcényle, alcynyle, cycloalkyle en C₃-C₈, ou aryle, ou
un sel du composé, pour une utilisation dans le traitement d'un sujet mammifère atteint d'une maladie **caractérisée par** une perte de fonction protéique provoquée par une anomalie génétique associée à la maladie,
la maladie étant la maladie de Gaucher, la maladie de von Hippel-Lindau, un phéochromocytome ou un paragangliome.

2. Inhibiteur pour une utilisation selon la revendication 1, la maladie étant un phéochromocytome ou un paragangliome.

3. Inhibiteur pour une utilisation selon la revendication 1, la maladie étant la maladie de von Hippel-Lindau.

4. Inhibiteur pour une utilisation selon la revendication 1, la maladie étant la maladie de Gaucher.

5. Inhibiteur pour une utilisation selon la revendication 1, l'inhibiteur d'histone désacétylase ayant la structure 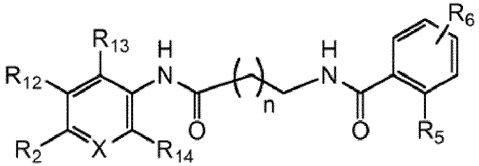 dans laquelle
n vaut 1-9 ;
X est C-R₁₁ ou N, R₁₁ étant H, OH, SH, F, Cl, SO₂R₇, NO_{2,} trifluorométhyle, méthoxy, ou CO-R₇, R₇ étant alkyle, alcényle, alcynyle, cycloalkyle en C₃-C₈, ou aryle ;
R₂ est H ou NR₃R₄, R₃ et R₄ étant chacun indépendamment H, alkyle en C₁-C₆, ou cycloalkyle en C₃-C₈;
R₅ est OH ou SH ; et
R₆, R₁₂, R₁₃, et R₁₄ sont chacun indépendamment H, OH, SH, F, Cl, SO₂R₁₅, NO₂, trifluorométhyle, méthoxy, ou CO-R₁₅, R₁₅ étant alkyle, alcényle, alcynyle, cycloalkyle en C₃-C₈, ou aryle.

6. Inhibiteur pour une utilisation selon la revendication 1, l'inhibiteur d'histone désacétylase ayant la structure 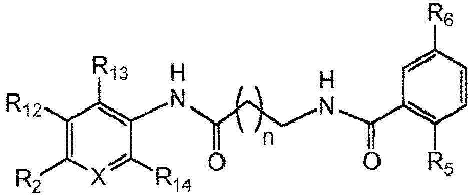 dans laquelle
n vaut 1-9 ;
X est C-R₁₁ ou N, R₁₁ étant H, OH, SH, F, Cl, SO₂R₇, NO₂, trifluorométhyle, méthoxy, ou CO-R₇, R₇ étant alkyle, alcényle, alcynyle, cycloalkyle en C₃-C₈, ou aryle ;
R₂ est H ou NR₃R₄, R₃ et R₄ étant chacun indépendamment H, alkyle en C₁-C₆, ou cycloalkyle en C₃-C₈;
R₅ est OH ou SH ; et
R₆, R₁₂, R₁₃, et R₁₄ sont chacun indépendamment H, OH, SH, F, Cl, trifluorométhyle, méthoxy, ou CO-R₁₅, R₁₅ étant alkyle, alcényle, alcynyle, cycloalkyle en C₃-C₈, ou aryle.

7. Inhibiteur pour une utilisation selon la revendication 1, l'inhibiteur d'histone désacétylase ayant la structure 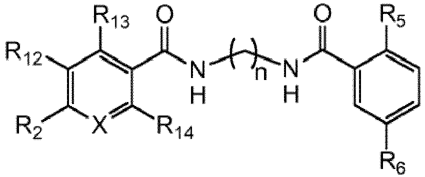 dans laquelle
n vaut 1-8 ;
X est C-R₁₁ ou N, R₁₁ étant H, OH, SH, F, Cl, SO₂R₇, NO₂, trifluorométhyle, méthoxy, ou CO-R₇, R₇ étant alkyle, alcényle, alcynyle, cycloalkyle en C₃-C₈, ou aryle ;
R₂ est H ou NR₃R₄, R₃ et R₄ étant chacun indépendamment alkyle en C₁-C₆ ou cycloalkyle en C₃-C₈ ; et
R₅ est OH ou SH ; et
R₆, R₁₂, R₁₃, et R₁₄ sont chacun indépendamment H, OH, SH, F, Cl, SO₂R₁₅, NO₂, trifluorométhyle, méthoxy, ou CO-R₁₅, R₁₅ étant alkyle, alcényle, alcynyle, cycloalkyle en C₃-C₈, ou aryle, ou
un sel du composé.

8. Inhibiteur pour une utilisation selon la revendication 1 ayant la structure 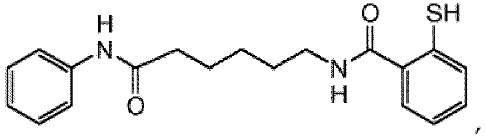 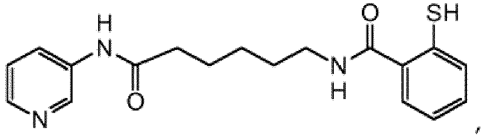 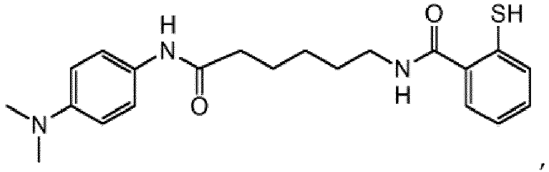 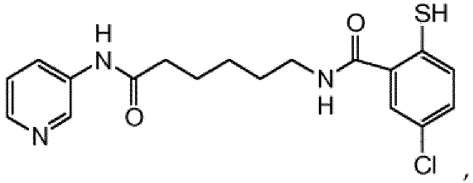 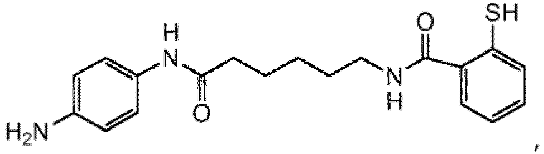 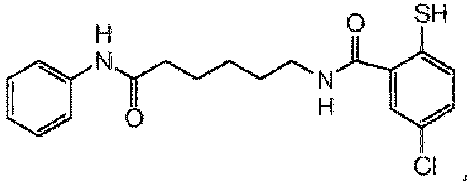 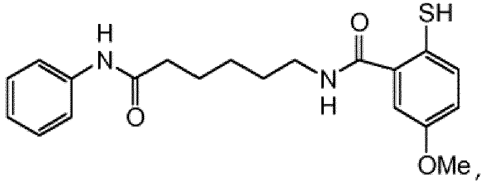 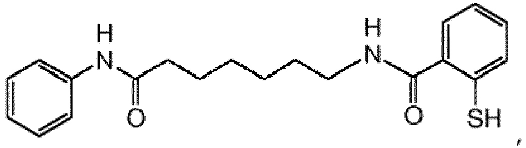 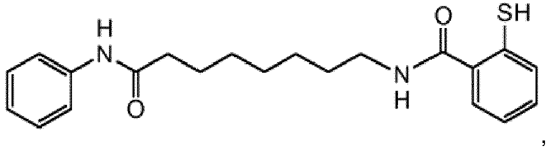 ou 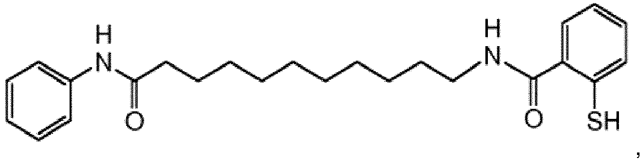 ou un sel du composé.

9. Inhibiteur pour une utilisation selon la revendication 1 ayant la structure 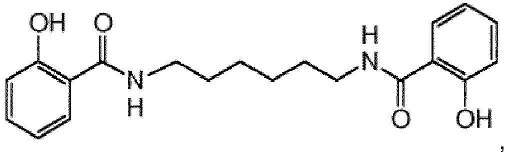 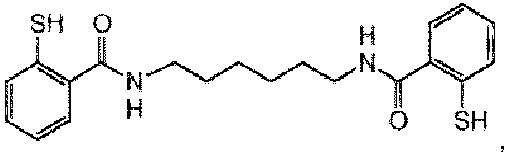 ou 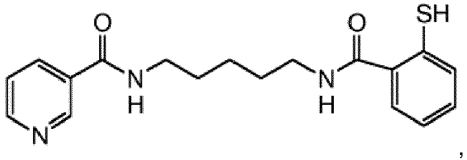 ou un sel du composé.

10. Inhibiteur d'histone désacétylase ayant la structure : 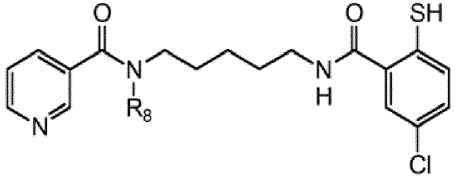 dans laquelle R₈ = alkyle ou aryle, ou
un sel du composé, pour une utilisation dans le traitement d'un sujet mammifère atteint d'une maladie **caractérisée par** une perte de fonction protéique provoquée par une anomalie génétique associée à la maladie,
la maladie étant la maladie de Gaucher, la maladie de von Hippel-Lindau, un phéochromocytome ou un paragangliome.

11. Inhibiteur pour une utilisation selon la revendication 10, la maladie étant un phéochromocytome ou un paragangliome.

12. Inhibiteur pour une utilisation selon la revendication 10, la maladie étant la maladie de von Hippel-Lindau.

13. Inhibiteur pour une utilisation selon la revendication 10, la maladie étant la maladie de Gaucher.

14. Inhibiteur pour une utilisation selon l'une quelconque des revendications 1-13, comprenant en outre un inhibiteur de protéine phosphatase 2A.
